# EUROPEAN PATENT APPLICATION

(11) **EP 1 862 464 A1**
(43) Date of publication of application: **05.12.2007**
(21) Application number: 06729621.0
(22) Date of filing: 22.03.2006
(51) Int. Cl.: C07D 417/12

(54) **NOVEL CYCLIC AMINOPHENYLALKANOIC ACID DERIVATIVE**

(30) Priority: 23.03.2005 JP 2005083024
(71) Applicant: Kyorin Pharmaceutical Co., Ltd., Tokyo 101-8311 (JP)
(72) Inventor: NOMURA, Masahiro, Tochigi, 3290101 (JP); TAKANO, Yasuo, Saitama 347-0063 (JP); YUMOTO, Kazuhiro, Tochigi, 3290101 (JP); OKADA, Kyoko, Tochigi, 3290101 (JP); SHINOZAKI, Takehiro, Tochigi, 3290101 (JP); ISOGAI, Shigeki, Tokyo 175-0084 (JP)
(74) Representative: Vossius & Partner
(86) International application number: PCT/JP2006/305654
(87) International publication number: WO 2006/101108

(57) **Abstract**

The present invention provides cyclic aminophenylalkanoic acid derivatives that act as agonists for human peroxisome proliferator-activated receptors (PPARs), in particular human PPARα isoform, and are effective in the treatment of abnormal lipid metabolism, diabetes and other disorders. The present invention also provides addition salts of such cyclic aminophenylalkanoic acid derivatives and pharmaceutical compositions containing these compounds.

Specifically, the present invention provides cyclic aminophenylalkanoic acid derivatives represented by the following general formula (1) : 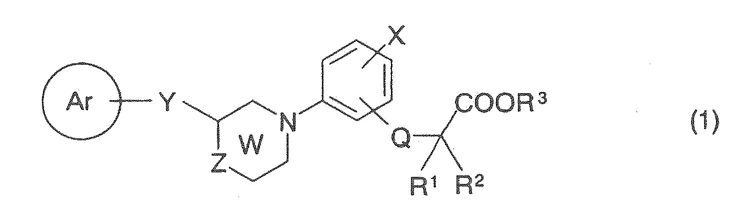 or pharmaceutically acceptable salts thereof.

## Description

### TECHNICAL FIELD

The present invention relates to cyclic aminophenylalkanoic acid derivatives that act as agonists of human peroxisome proliferator-activated receptors (PPARs), in particular human PPARα isoform, and are effective in the treatment of abnormal lipid metabolism, diabetes and other disorders. The present invention also relates to addition salts of such cyclic aminophenylalkanoic acid derivatives and pharmaceutical composition containing these compounds.

### BACKGROUND ART

Peroxisome proliferator-activated receptors (PPARs) are a group of ligand-dependent transcription factors that, like steroid receptors, retinoid receptors and thyroid receptors, belong to the nuclear receptor superfamily. Three isoforms of PPARs (α, γ and δ (or β)) have been identified in different animal species (Non-Patent Document 1). PPARα is found in liver and kidney where active fatty acid catabolism occurs (Non-Patent Document 2). PPARα positively or negatively regulates the expression of genes involved in fatty acid metabolisms and intracellular transportation of fatty acids (such as acyl-CoA synthase, fatty acid-binding protein and lipoprotein lipase) and apolipoprotein (AI, AII, CIII) genes involved in cholesterol and neutral lipid metabolisms. PPARγ is expressed at high levels in adipocytes and is involved in the differentiation of adipocytes (Non-Patent Document 3). PPARδ is widely expressed in various tissues, but predominantly in nerve cells. PPARδ has recently been reported to be involved in fatty acid oxidation (Non-Patent Documents 4 and 5) though its physiological functions mostly remain unclear. As described, each isoform of PPARs has a specific role in a particular organ or tissue.

It has been reported that PPARα knock-out mice develop hypertriglyceridemia as they grow old and become obese due mainly to an increase in the number of white adipocytes (Non-Patent Document 6). The observation strongly suggests that the activation of PPARα is correlated with the decrease in the blood lipid levels (cholesterols and neutral lipids).

Statins and fibrates are major drugs currently used to treat hyperlipidemia. The drugs have their own drawbacks: Statins are less effective in decreasing free fatty acid and triglyceride levels; fibrates are less effective in decreasing cholesterol levels. Fibrates are associated with side effects such as gastrointestinal disorders, anthema, headache, hepatic failure, renal failure and biliary calculus supposedly caused by the broad pharmacological effects of the drugs. Thus, there is a significant need for side effect-free therapeutic agents for hyperlipidemia are needed.

Considering the present state of conventional treatments for hyperlipidemia and the roles of PPARα, a transcription factor, in the modulation of lipid metabolisms and its associations with hyperlipidemia, compounds that serve as a ligand that directly binds and strongly activates PPARα, in particular human PPARα, may be used as drugs that effectively decrease the blood lipid levels (both cholesterols and neutral lipids) through a unique mechanism.

Apart from LTB₄, a metabolite of arachidonic acid, eicosanoids of a group of hydroxyeicosatetraenoic acids (HETEs), in particular 8-HETE and 8-HEPE, that are produced by oxidation of arachidonic acid by cytochrome p450 have been reported as endogenous ligands for PPARα (Non-Patent Document 7). However, these endogenous unsaturated fatty acid derivatives are metabolically and chemically unstable and are therefore unsuitable for use in pharmaceutical products.

Certain compounds have been reported to act as PPARα agonists. Shown in Table 1 are some examples of such compounds (compounds (A) through (J)). Unlike the compounds of the present invention, none of these compounds includes a phenylalkanoic acid structure substituted with an alicyclic amino group, a characteristic feature the present invention.

**(Table 1)**

| Patent Document No. | Formula | Patent Document No. | Formula |
|---|---|---|---|
| 1 | 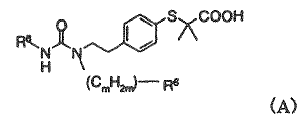 | 6 | 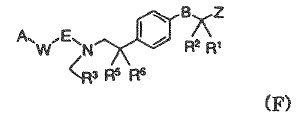 |
| 2 | 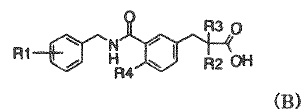 | 7 | 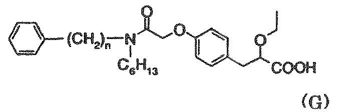 |
| 3 | 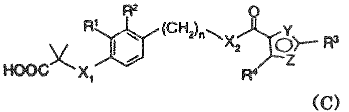 | 8 | 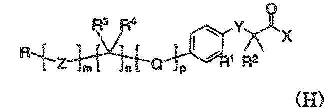 |
| 4 | 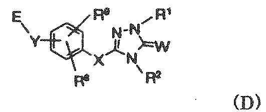 | 9 | 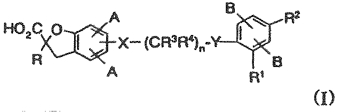 |
| 5 | 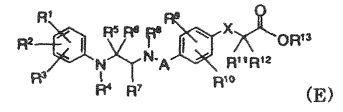 | 10 | 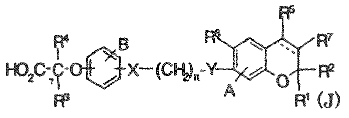 |

Patent Document 11 describes compounds represented by the following general formula (K) that are structurally similar to the compound of the present invention and act as PPARα agonists:

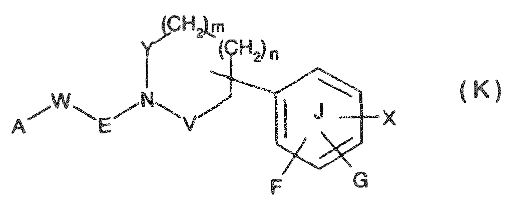

[wherein Y and V represent a methylene group or a carbonyl group; F and G represent a hydrogen atom or a halogen atom; X represents Z or -B-C(R¹R²)-Z; B represents an oxygen atom or a sulfur atom; Z represents -C(O)OH or a -C(O)O-(C₁-C₆) alkyl group; R¹ represents a hydrogen atom or a (C₁-C₆) alkyl group; R² represents a hydrogen atom or a (C₃-C₆) cycloalkyl group; E represents a carbonyl group, a sulfonyl group or a methylene group; W represents a bond, a carbonyl group or -N(H)-; and A represents a mono-N- or di-N,N-(C1-C6) alkylamino group, a (C2-C6) alkanoylamino group or a partially or fully saturated or unsaturated 3- to 8-membered ring that may contain 1 to 4 oxygen atoms, sulfur atoms or nitrogen atoms] (only some of the substituents are described). These compounds share a common feature that the ring J is linked to the alicyclic amino group containing Y and V at a position other than the nitrogen atom. This feature structurally distinguishes these compounds from the compound of the present invention, in which phenylalkanoic acid is linked to the nitrogen atom of the alicyclic amino group.

Patent Document 12 describes compounds represented by the following general formula (L) that are structurally similar to the compound of the present invention and act as PPARγ agonists:

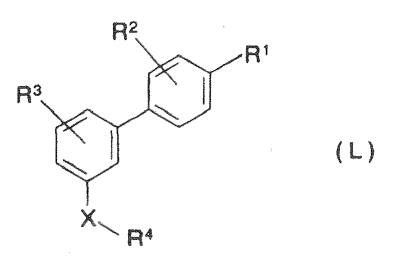

[wherein R¹ represents the following general formula (L-a):

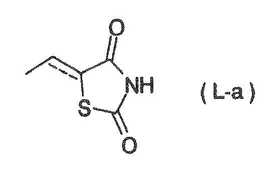

or the following general formula (L-b):

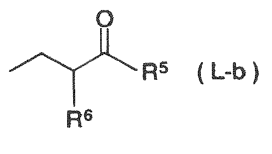

(wherein R⁵ represents a hydroxyl group or a C₁-C₉ alkoxy group, and R⁶ represents a C₁-C₆ alkyl group); R² and R³ represent a hydrogen atom or a alkyl group; X represents -CH₂-NR⁸CO- or -N(R⁸)-COCH₂-; R⁴ represents a phenyl group or a benzyl group; and R⁸ represents a hydrogen atom or a C₁-C₆ alkyl group] (only some of the substituents are described). These compounds are biphenylalkanoic acid derivatives and are structurally different from the compound of the present invention.

Patent Document 13 describes compounds represented by the following general formula (M) that are structurally similar to the compound of the present invention and act as PPAR agonists:

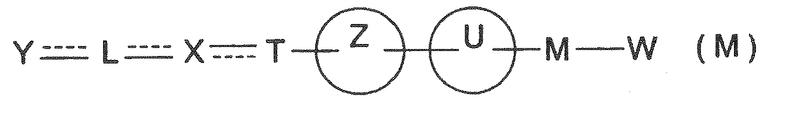

[wherein L represents a single bond or a substituted or unsubstituted C₁-C₆ alkylene group; M represents a single bond or a substituted or unsubstituted C₁-C₆ alkylene group; T represents a single bond; W represents a carboxyl group;

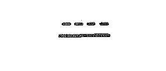

represents a single or double bond; X represents a single bond or an oxygen atom; Y represents a substituted or unsubstituted 5- to 14-membered aromatic ring that may contain at least one heteroatom or a C₃-C₇ alicyclic hydrocarbon group; and rings Z and U are each independently a saturated or unsaturated 5- to 14-membered aromatic ring that may have 1 to 4 substituents and at least one heteroatom] (only some of the substituents are described). Patent Document 14 describes compounds represented by the following general formula (N) that act as PPAR receptor ligands:

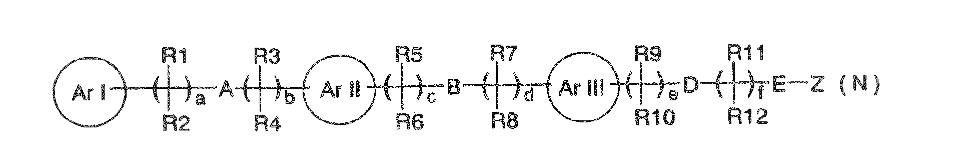

[wherein rings ArI, ArII and ArIII each independently represent an aryl or a heteroaryl; A represents an oxygen atom, a sulfur atom or a single bond; B represents an oxygen atom or a sulfur atom; D represents an oxygen atom, a sulfur atom or a single bond; E represents a single bond or an ethylene group; a, b, c and e represent an integer of 0 to 4; d represents an integer of 0 to 5; f represents an integer of 0 to 6; R¹, R³, R⁵, R⁷, R⁹ and R¹¹ each independently represent a hydrogen atom or a halogen atom; R², R⁴, R⁶, R⁸, R¹⁰ and R¹² each independently represent - (CH₂)_{q}-X; q represents an integer of 0 to 3; X represents a hydrogen atom or a halogen atom; Z represents R²¹0₂C- or R²¹CO-; and R²¹ represents a hydrogen atom] (only some of the substituents are described). Neither the ring Z nor the ring ArII of the compounds described in Patent Documents 13 and 14 contains an alicyclic amino group, an essential feature of the compound of the present invention. This structurally distinguishes these compounds from the compound of the present invention.

Other compounds structurally similar to the compound of the present invention and acting as PPAR agonists are also known. Patent Document 15 describes compounds represented by the following general formula (O):

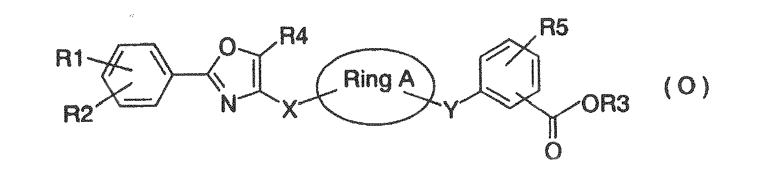

[wherein ring A represents a C₃-C₈ cycloalkyl that may contain an oxygen atom or a C₃-C₈ cycloalkenyl that may contain an oxygen atom; R¹, R², R⁴ and R⁵ represent a hydrogen atom, a fluorine atom, a chlorine atom, a bromine atom, a hydroxyl group, a nitro group, a trifluoromethyl group, a trifluoromethoxy group, a C₁-C₆ alkyl group or a C₁-C₆ alkoxy group; R³ represents a hydrogen atom or a C₁-C₆ alkyl group; and X and Y represent a C₁-C₆ alkylene group that may be substituted with an oxygen atom]. Patent Document 16 describes compounds represented by the following general formula (P):

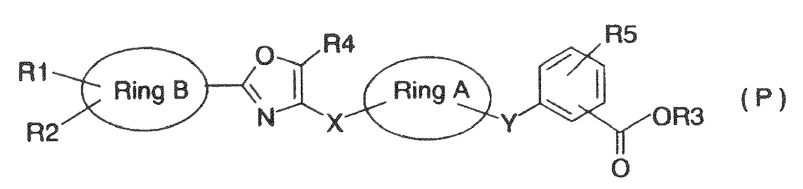

[wherein ring A represents a C₃-C₈ cycloalkyl that may contain an oxygen atom or a C₃-C₈ cycloalkenyl that may contain an oxygen atom; ring B represents a phenyl group or a 5- to 12-membered heteroaromatic ring containing N, O or S; R¹ represents SCF₃ or OCF₂-CF₃; R² represents a hydrogen atom or CF₃; R⁴ represents a phenyl group; R⁵ represents a hydrogen atom, a fluorine atom, a chlorine atom, a bromine atom, a hydroxyl group, a nitro group, a trifluoromethyl group, a trifluoromethoxy group, a C₁-C₆ alkyl group or a C₁-C₆ alkoxy group; R³ represents a hydrogen atom or a C₁-C₆ alkyl group; and X and Y represent a C₁-C₆ alkylene group that may be substituted with an oxygen atom] (only some of the substituents are described). Patent Document 17 describes compounds represented by the following general formula (Q):

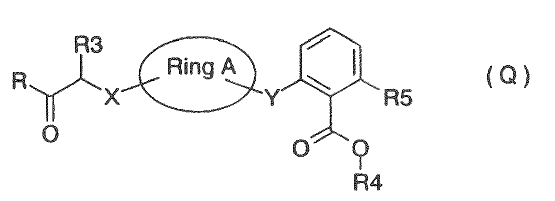

[wherein ring A represents a C₃-C₈ cycloalkyl that may contain an oxygen atom or a C₃-C₈ cycloalkenyl that may contain an oxygen atom; R represents NR¹R² or OR¹ R¹ and R² represent a hydrogen atom or a C₁-C₆ alkyl group; R³ represents a C₃-C₆ cycloalkyl group or a C₁-C₁₀ alkyl group; X and Y represent a C₁-C₆ alkylene group that may be substituted with an oxygen atom; R⁴ represents a hydrogen atom or a C₁-C₄ alkyl group; and R⁵ represents a C₁-C₄ alkyl group] (only some of the substituents are described). Patent Document 18 describes compounds represented by the following general formula (R):

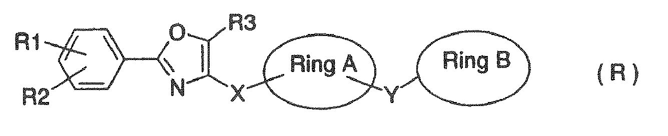

[wherein ring A represents a C₃-C_{B} cycloalkyl that may contain an oxygen atom or a C₃-C₈ cycloalkenyl that may contain an oxygen atom; R¹ and R² represent a hydrogen atom or a fluorine atom; R³ represents a hydrogen atom or CF₃; X represents a C₁-C₆ alkylene group that may be substituted with an oxygen atom; Y represents an oxygen atom or a C₁-C₆ alkylene group that may be substituted with CO; ring B represents a phenyl group that may substituted with NO₂ or a chlorine atom; and R₄ represents a C₁-C₆ alkyl group, a phenyl group or a benzyl group] (only some of the substituents are described). The compounds described in Patent Documents 15 through 18 share a common feature that the ring A does not contain an aliphatic amino group or a phenylalkanoic acid structure, each an essential structure of the compound of the present invention. In addition, the ring A is linked to a phenyl group or the ring B via a linker Y. These features structurally distinguish the compounds of Patent Document 15 through 18 from the compound of the present invention.

Patent Document 19 through 23 describe compounds represented by the following general formula (S) that are structurally similar to the compound of the present invention and act to decrease the triglyceride or cholesterol levels:

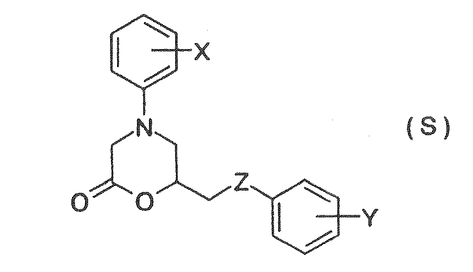

[wherein Z represents an oxygen atom or an sulfur atom; X represents a hydrogen atom, a halogen atom, a lower alkyl group, a carboxyl group or a lower alkoxycarbonyl group; Y represents a hydrogen atom, a halogen atom or a lower alkyl group with the proviso that Z is a sulfur atom, or Y represents a hydrogen atom, a halogen atom, a lower alkyl group or a lower alkoxy group with the proviso that Z is an oxygen atom] (only some of the substituents are described). Unlike the alicyclic amino group-containing compound of the present invention, these compounds are morpholinone derivatives. Furthermore, the substituent X does not include alkanoic acid structure, another structural difference from the compound of the present invention. In addition, nothing is mentioned about the PPARα agonist activity in any of Patent Document 19 through 23.

Patent Document 24 describes compounds represented by the following general formula (T) that are structurally similar to the compound of the present invention and act as a p38MAP kinase inhibitor:

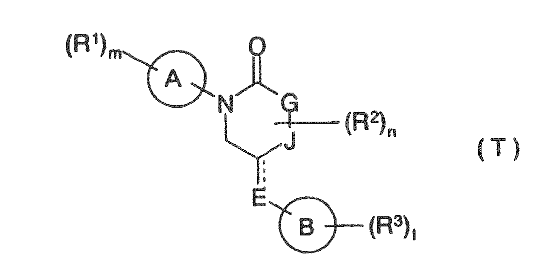

[wherein ring A represents a C₅-C₁₀ monocyclic or bicyclic hydrocarbon ring; R¹ represents a C₁-C₈ alkyl group or C₂-C₈ alkenyl group substituted with COOR¹¹; R² represents a C₁-C₈ alkyl group; G and J each independently represent a carbon atom or a nitrogen atom; E represents a C₁-C₈ alkylene group or a C₂-C₈ alkenylene group; ring B represents a C₅-C₁₀ monocyclic or bicyclic hydrocarbon ring; R³ represents a C₁-C₈ alkyl group or a C₂-C₈ alkenyl group; m represents an integer of 0 to 5; n represents an integer of 0 to 7; 1 represents an integer of 0 to 12; R¹¹ represents a hydrogen atom; and

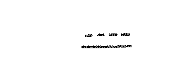

represents a single or double bond] (only some of the substituents are described). Unlike the alicyclic amino group-containing compound of the present invention, these compounds are cyclic amide derivatives. Furthermore, the phenylalkanoic acid derivatives described in the Example section of the specification are each a para-substituted derivative and are structurally different from the compound of the present invention. In addition, nothing is mentioned about the PPARα agonist activity in Patent Document 24.

Patent Document 25 describes compounds represented by the following general formula (U) that are structurally similar to the compound of the present invention and act as β₃ adrenaline receptor agonists:

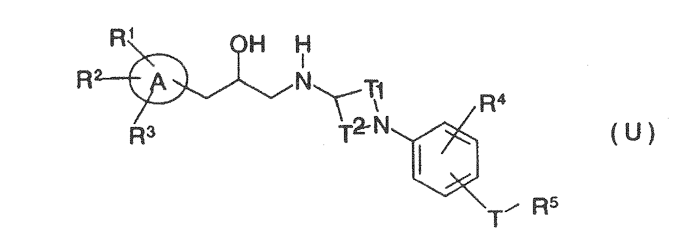

[wherein ring A represents an aromatic ring or hetero ring; X represents -OCH₂-, -SCH₂- or a bond; T¹ represents (CH₂)ₘ; T² represents (CH₂)ₙ; T represents a bond or a C₁-C₆ alkyl group that may be substituted with R¹¹; R¹, R² and R³ each independently represent a hydrogen atom or a C₁-C₆ alkyl group; R⁴ represents a hydrogen atom or a C₁-C₆ alkyl group; R⁵ represents COOR⁶ or the following general formula (U-a) :

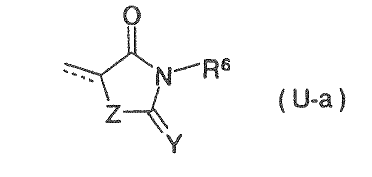

(wherein Y and Z each independently represent NR⁷, O or S; R⁶ represents a hydrogen atom or a C₁-C₆ alkyl group that may be substituted with R¹¹, R¹² or R¹³; and a dashed line represents a single or double bond) ; m represents an integer of 1 to 3; n represents an integer of 1 to 3; R⁶ represents a hydrogen atom or a C₁-C₆ alkyl group; and R¹¹, R¹² and R¹³ each independently represent a C₁-C₆ alkyl group or a halogen atom] (only some of the substituents are described). These compounds structurally differ from the compound of the present invention in that they all have a characteristic aminoethanol structure. Also, the phenylalkanoic acid derivatives, which are described in the Example section of the specification and are substituted with a cyclic amino group, are each substituted at the para-position of phenylalkanoic acid, and are structurally different from the compound of the present invention. In addition, nothing is mentioned about the PPARα agonist activity in Patent Document 25.

Patent Document 26 describes compounds represented by the general formula (V) that are structurally similar to the compound of the present invention and act as calcium receptor antagonists:

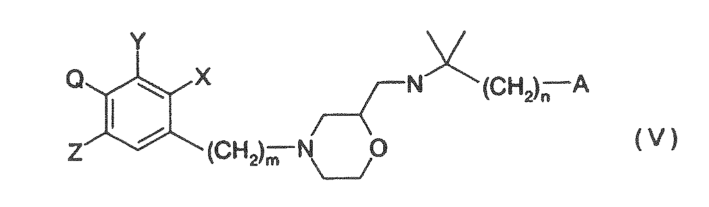

[wherein m represents an integer of 0 to 2; n represents an integer of 1 to 3; X represents a cyano group or a nitro group; Y represents a chlorine atom or a fluorine atom; Q and Z each independently represent a hydrogen atom, R₁, SO₂R₁' or C(O)OR₁"; A represents a hydroxyl group, a phenyl group or a naphthyl group that may substituted with a halogen atom (s); R₁, R₁' and R₁" each independently represent a hydrogen atom or a C₁-C₄ alkyl group] (only some of the substituents are described). Patent Document 26 mentions nothing about the PPARα agonist activity of these compounds, nor does it describe any phenylalkanoic acid derivatives substituted with a cyclic amino group.

Compounds are also known that are structurally similar to the compound of the present invention and act as factor Xa inhibitors. For example, Patent Document 27 describes compounds represented by the following general formula (W):

R⁰-Q-X-Q'-W-U-V-G-M (W)

[wherein R⁰ represents an aryl group that may be substituted with R²; Q and Q' represent a bond or a carbonyl group; X represents a bond or a 3- to 7-membered heteroaryl group; W represents a 5- to 14-membered aryl group that may be substituted with R¹, or a 4- to 15-membered ring that may be substituted with R¹ and may contain one to four oxygen atoms, nitrogen atoms or sulfur atoms; U and G represent a bond, - (CH₂)ₘ or - (CH₂)ₘ-O- (CH₂)ₘ-; V represents a 6-to 14-membered aryl group or a bond; M represents a C₆-C₁₄ alkyl group that may be substituted with R¹⁴, or a hydrogen atom; R¹ represents a halogen atom or a nitro group; R² represents a halogen atom or a nitro group; and R¹⁴ represents a halogen atom, OH or COOH] (only some of the substituents are described). Patent Document 28 describes compounds represented by the following general formula (X) :

A-Y-D-E-G-J-Z-L (X)

[wherein A represents a phenyl group that may be substituted with 0 to 2 R¹, a C₁-C₆ alkyl group or a C₃-C₈ cycloalkyl group; Y represents a bond or -C (=O) -; D represents a bond or a phenyl group substituted with 0 to 2 R^{1a}; E represents -N(R⁵)-C(=O) - or -C(=O)-N(R⁵)-; G represents a bond or -CR⁷R⁸-; J represents the following general formula (X-a):

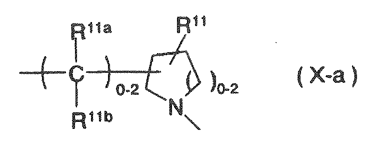

(wherein R¹¹, R^{11a} and R^{11b} each independently represent a hydrogen atom or a hydroxyl group); Z represents a phenyl group substituted with 0 to 2 R^{1b} or a naphthyl group substituted with 0 to 2 R^{1b}; L represents a hydrogen atom, a cyano group, C(=O)NR¹²R¹³ or C(=NR¹²)NR¹²R¹³; R¹ represents a halogen atom or a C₁-C₄ alkyl group; R^{1a} represents a halogen atom or a C₁-C₄ alkyl group; R^{1b} represents a halogen atom or -OCH₂-COOR^{2b}; R^{2b} represents a hydrogen atom or a C₁-C₄ alkyl group; and R¹² and R¹³ each independently represent a hydrogen atom or a C₁-C₄ alkyl group] (only some of the substituents are described). Patent Document 29 describes compounds represented by the following general formula (Y):

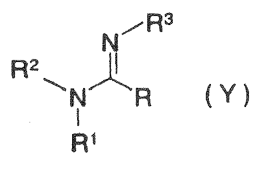

[wherein R¹, R² and R³ each independently represent a hydrogen atom or a hydroxyl group; and R represents the following general formula (S-a) :

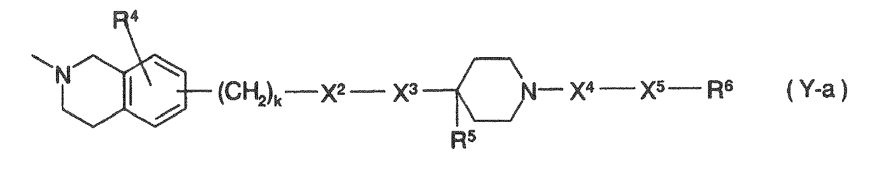

(wherein R⁴ represents a hydrogen atom or a hydroxyl group; R⁵ represents a hydrogen atom or a cyano group; R⁶ represents a hydrogen atom, a lower alkyl group or a carboxyl group; X² represents an oxygen atom or a sulfur atom; X³ represents -(CH₂)ₘ-; X⁴ represents -CO-, -C(=NH)- or

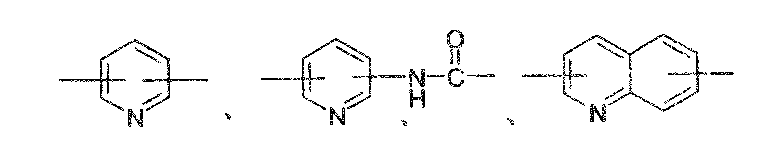

X⁵ represents a C₁-C₆ alkylene; R⁶ represents a carboxy group or a lower alkoxycarbonyl group; k represents 0 or 1; and m represents an integer of 0 to 3) (only some of the substituents are described). Nothing is mentioned about the PPARα agonist activity of the described compounds in Patent Document 27 or 28, nor is anything described in the Example sections of the respective specifications about phenylalkanoic acid derivatives substituted with a cyclic amino group. The compounds described in Patent Document 29 are pyridylalkanoic acids or quinolylalkanoic acids and differ from phenylalkanoic acids of the present invention. The only alicyclic amino group described in the specification is 1,4-substituted piperidyl group, which is structurally different from the compound of the present invention. Patent Document 29 mentions nothing about the PPARα agonist activity, either.

Patent Document 30 describes compounds represented by the following general formula (z) that are structurally similar to the compound of the present invention and act as integrin inhibitors:

U-V-A-(Alk)ⱼ-(CO-NH)ₕ-(Alk)_{g}-B (Z)

[wherein g, h and I each independently represent 0 or 1; Alk represents an alkylene group; U represents an amidino group, a guanidino group or -(G-Alk)ₖ-C(Q)-N(R)R₁ (wherein G represents a single bond or an oxygen atom, Q represents an oxygen atom or a sulfur atom, R represents a hydrogen atom or an alkyl group, R₁ represents an alkyl group or an aryl group, and k represents 0 or 1); V represents the following general formula (Z-a):

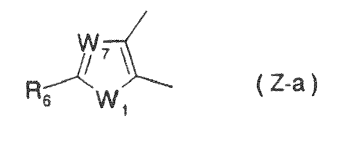

or the following general formula (Z-aa):

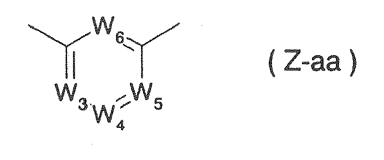

(wherein W₁ represents an oxygen atom or a sulfur atom, W₃, W₄, W₅ and W₆ represent N or C-R₄, W₇ represents a nitrogen atom, R₄ represents a hydrogen atom or a halogen atom, and R₆ represents a hydrogen atom or a halogen atom) ; A represents the following general formula (Z-b) :

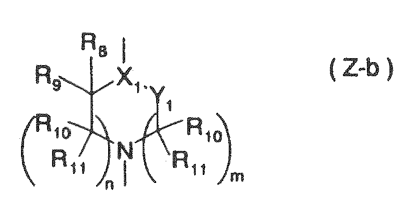

or the following general formula (Z-bb):

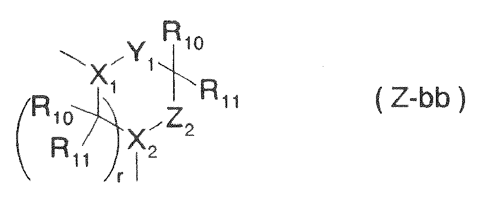

(wherein X₁ represents a nitrogen atom or C-H; X₂ represents C-H; Y₁ represents -C(O)- or -C(S)-; Z₂ represents an oxygen atom or a sulfur atom; n and m each independently represent 0, 1 or 2 with the proviso that n+m = 1, 2, 3 or 4; r is 1 or 2; R₈, R₉, R₁₀ and R₁₁ each independently represent a hydrogen atom or an alkyl group; B represents the following general formula (Z-c):

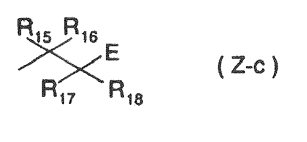

(wherein R₁₅ represents a hydrogen atom or an alkyl group; R₁₇ represents a hydrogen atom, an alkyl group or an aryl group; R₁₆ and R₁₈ are each independently represent a hydrogen atom or an alkyl group; and E represents a carboxyl group or an amide group)] (only some of the substituents are described). These compounds are cyclic amide derivatives and therefore differ from alicyclic amino group which is one of characteristics of the compound of the present invention. The functional group to serve as the substituent U includes amidino group and guanidino group, but not alkanoic acids, a structural feature essential to the present invention. The compounds described in Patent Document 30 are therefore structurally distinguished from the compound of the present invention. The Example section of the specification mentions phenylalkanoic acid derivative intermediates. These compounds include phenylalkanoic acid structures substituted with a cyclic amide group and thus structurally differ from the compound of the present invention. Patent Document 30 describes nothing about the PPARα agonist activity of these compounds.

Patent Document 31 describes compounds represented by the following general formula (ZA) that are structurally similar to the compound of the present invention and act as integrin αᵥβ₃ antagonists:

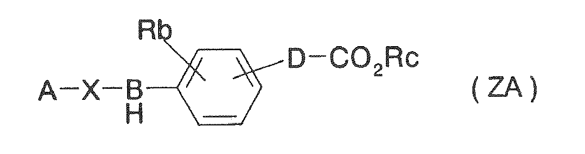

[wherein A represents a hydrogen atom or a saturated or unsaturated 5- to 7-membered heterocyclic group containing at least one nitrogen atom; X represents -NH-, -CH₂- or a bond; B represents a C₁₋₆ alkylene group that may contain an unsaturated bond, pyrrolidine or piperidine; D represents -CONR⁴CHR⁵CHR⁶- or -CHR⁵CHR⁶-; Rb represents a hydrogen atom or a halogen atom; Rc represents a hydrogen atom or a C₁₋₆ alkyl group; R⁴ represents a hydrogen atom or a C₁₋₆ alkyl group; and R⁵ and R⁶ each independently represent a hydrogen atom] (only some of the substituents are described). The specification mentions nothing about the PPARα agonist activity of these compounds. The Example section of the specification does not describe anything about phenylalkanoic acids substituted with a cyclic amino group.

Compounds are also known that are structurally similar to the compound of the present invention and act as integrin αᵥβ₃ antagonists. For example, Patent Document 32 describes compounds represented by the following general formula (AA):

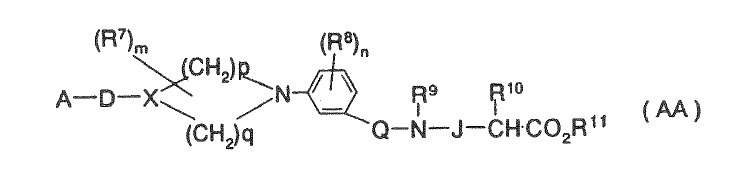

[wherein A represents a saturated or unsaturated 5- or 7-membered heterocyclic group containing 2 nitrogen atoms; D represents a bond or -NR⁴- (where R⁴ represents a hydrogen atom or a C₁₋₆ alkyl group) ; X represents CH or N; R⁷ represents a C₁₋₆ alkyl group or a C₁₋₆ alkoxy group; R⁸ represents a C₁₋₆ alkyl group or a C₁₋₆ alkoxy group; Q represents C=O; R⁹ represents a hydrogen atom or a C₁₋₆ alkyl group; J represents a bond or a C₁₋₃ alkylene chain; R¹⁰ represents a hydrogen atom or a hydroxyl group; R¹¹ represents a hydrogen atom or an aralkyl group; m represents an integer of 0 to 5; n represents an integer of 0 to 4; p represents an integer of 0 to 3; and q represents an integer of 0 to 3] (only some of the substituents are described). Patent Document 33 describes compounds represented by the following general formula (AB):

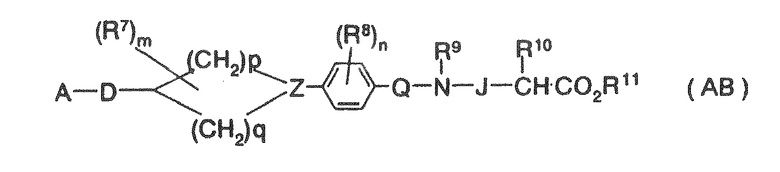

[wherein A represents a saturated or unsaturated 5- to 7-membered heterocyclic group containing at least one nitrogen atom; D represents -NR⁴- (where R⁴ represents a hydrogen atom or an C₁₋₆ alkyl group) or -O-; Z represents CH or N; R⁷ represents a C₁₋₆ alkyl group or a C₁₋₆ alkoxy group; R⁸ represents a C₁₋₆ alkyl group or a C₁₋₆ alkoxy group; Q represents C=O; R⁹ represents a hydrogen atom or a C₁₋₆ alkyl group; J represents a bond or a C₁₋₃ alkylene chain; R¹⁰ represents a hydrogen atom or a hydroxyl group; R¹¹ represents a hydrogen atom or a C₁₋₆ alkyl group that may be substituted with 1 or 2 phenyl groups; m represents an integer of 0 to 5; n represents an integer of 0 to 4; p represents 3 or 4; and q represents an integer of 0 to 3] (only some of the substituents are described). These compounds share a common feature that the phenyl group is linked to the carboxyl group via a nitrogen-containing linker. This feature structurally distinguishes these compounds from the compound of the present invention, which comprises a phenylalkanoic acid substituted with a cyclic amino group. Nothing is mentioned about the PPARα agonist activity either in Patent Document 32 or No. 33.

Patent Document 34 describes compounds represented by the following general formula (AC) that are structurally similar to the compound of the present invention and act as integrin αᵥβ₃ antagonists:

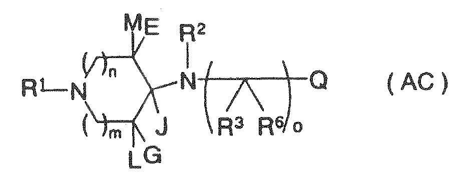

[wherein Q represents the following general formula (AC-a):

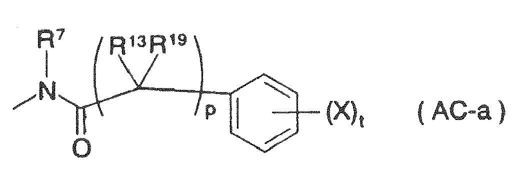

(wherein R⁷ represents a hydrogen atom or a C₁₋₈ alkyl group; R¹³ represents a hydrogen atom or a C₁₋₈ alkyl group; R¹⁹ represents a hydrogen atom or a C₁₋₈ alkyl group; X represents a halogen atom or a cyano group; p represents an integer of 0 to 4; and t represents an integer of 0 to 5); E, G, L and M each independently represent a hydrogen atom or a C₁₋₈ alkyl group; J represents a hydrogen atom or a C₁₋₈ alkyl group; R¹ represents a halogen atom or a phenyl group that may be substituted with (CH₂)₀₋₄CO₂R¹⁶; R² represents a hydrogen atom or a C₁₋₈ alkyl group; R³ and R⁶ each independently represent a hydrogen atom or a C₁₋₈ alkyl group; R¹⁶ represents a hydrogen atom or a C₁₋₈ alkyl group; m, n and p each independently represent an integer of 0 to 4; and o represents an integer of 2 to 5] (only some of the substituents are described). These compounds share a common feature that the aminoalkylamino group is linked to the cyclic amino group, which structurally distinguishes them from the compound of the present invention. In addition, the Example section of the specification does not describe phenylalkanoic acid derivatives. Moreover, nothing is mentioned about the PPARα agonist activity.

Patent Document 35 describes compounds represented by the following general formula (AD) that are structurally similar to the compound of the present invention and act as PPAR receptor ligands:

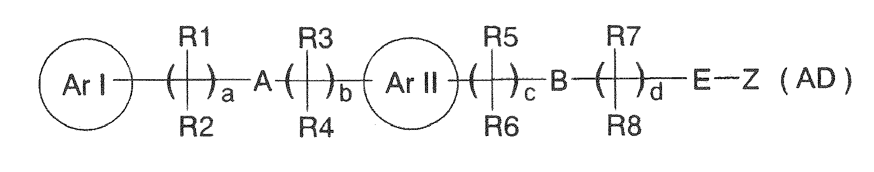

[wherein the rings ArI and ArII each independently represent an aryl group or a heteroaryl group; A represents an oxygen atom, a sulfur atom or the following general formula (AD-a):

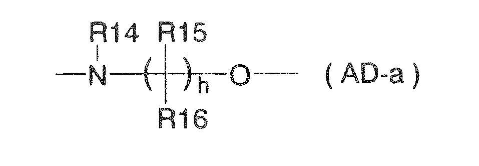

(wherein h represents an integer of 1 to 4; and R¹⁴, R¹⁵ and R¹⁶ represent a hydrogen atom or an alkyl group, or R¹⁴ and R¹⁵, together with a nitrogen atom, form a 5- or 6-membered heteroring); B represents an oxygen atom or a sulfur atom; E represents a single bond or an ethylene group; a and d represent an integer of 0 to 6; b and c represent an integer of 0 to 4; R¹, R³, R⁵ and R⁷ each independently represent a hydrogen atom or a halogen atom; R², R⁴, R⁶, R⁸ and R¹² each independently represent -(CH₂)_{q}-X; q represents an integer of 0 to 3; X represents a hydrogen atom or a halogen atom; Z represents R²¹0₂C- or R²¹CO-; and R²¹ represents a hydrogen atom] (only some of the substituents are described). These compounds share a common feature that the cyclic amino group represented by the general formula (AF-a) is linked to the ring ArII via a linker. This feature structurally distinguishes these compounds from the compound of the present invention. Moreover, the Example section of the specification describes nothing about compounds having a cyclic amino group.

Compounds are also known that are structurally similar to the compound of the present invention and act to reduce the lipid levels. For example, Patent Document 36 describes compounds represented by the following general formula (AE):

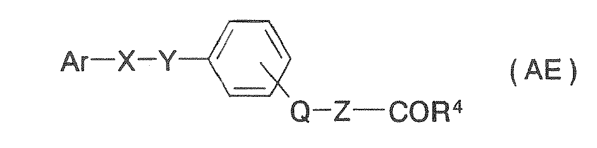

[wherein Ar represents a naphthyl group or a pyridyl group; X represents -CO- or -SO₂-; Y represents the following:

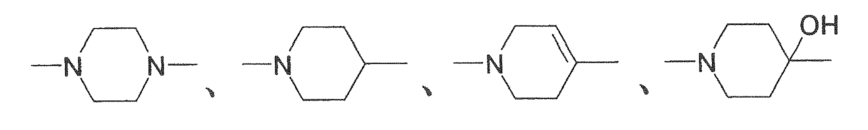

; Q represents -O- or a single bond; Z represents 1 to 3 alkylene groups or -CR⁵R⁶-(wherein R⁵ and R⁶ represent an alkyl group); and R⁴ represents a hydroxyl group or -NH(CH₂)ₘCOOH (wherein m represents an integer of 1 to 3) (only some of the substituents are described). Patent Document 37 describes compounds represented by the following general formula (AF):

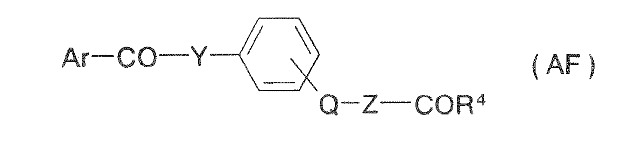

[wherein Ar represents a naphthyl group or a pyridyl group; Y represents the following:

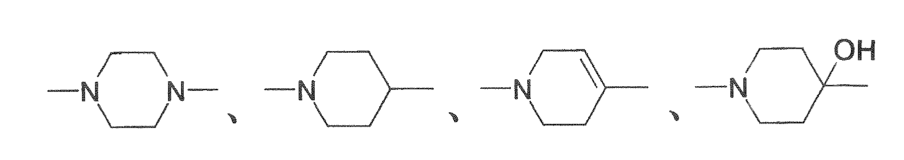

; Q represents -O- or a single bond; Z represents 1 to 3 alkylene groups or -CR⁵R⁶- (wherein R⁵ and R⁶ represent an alkyl group); and R⁴ represents a hydroxyl group or -NH(CH₂)ₘCOOH (wherein m represents an integer of 1 to 3)] (only some of the substituents are described). These compounds share a common feature that the substituent Ar-X or Ar-CO- is linked to the nitrogen atom at position 4 of the ring Y with respective to the phenyl group. This feature structurally distinguish these compounds from the compound of the present invention. Furthermore, nothing is mentioned about the PPAR agonist activity in Patent Documents 36 or 37.
Patent Document 1 WO00/23407 pamphlet
Patent Document 2 WO00/75103 pamphlet
Patent Document 3 WO01/40207 pamphlet
Patent Document 4 WO02/38553 pamphlet
Patent Document 5 WO02/28821 pamphlet
Patent Document 6 WO02/064549 pamphlet
Patent Document 7 WO03/051821 pamphlet
Patent Document 8 WO03/059875 pamphlet
Patent Document 9 WO2004/010936 pamphlet
Patent Document 10 WO2004/010992 pamphlet
Patent Document 11 WO2004/048334 pamphlet
Patent Document 12 WO03/055867 pamphlet
Patent Document 13 WO02/098840 pamphlet
Patent Document 14 WO00/64876 pamphlet
Patent Document 15 WO03/020269 pamphlet
Patent Document 16 WO2004/075815 pamphlet
Patent Document 17 WO2004/076402 pamphlet
Patent Document 18 WO2004/076447 pamphlet
Patent Document 19 Japanese Patent Laid-Open Publication No. Sho 52-83676
Patent Document 20 Japanese Patent Laid-Open publication No. Sho 51-149234
Patent Document 21 Japanese Patent Laid-Open Publication No. Sho 51-149235
Patent Document 22 Japanese Patent Laid-Open Publication No. Sho 51-146478
Patent Document 23 Japanese Patent Laid-Open Publication No. Sho 51-146479
Patent Document 24 WO03/043988 pamphlet
Patent Document 25 WO02/06232 pamphlet
Patent Document 26 WO00/09132 pamphlet
Patent Document 27 WO02/051831 pamphlet
Patent Document 28 WO00/71515 pamphlet
Patent Document 29 Japanese Patent Laid-Open Publication No. 2000-136190
Patent Document 30 WO01/44230 pamphlet
Patent Document 31 WO01/054726 pamphlet
Patent Document 32 WO01/027090 pamphlet
Patent Document 33 WO01/027082 pamphlet
Patent Document 34 WO98/57638 pamphlet
Patent Document 35 WO00/64888 pamphlet
Patent Document 36 WO93/12086 pamphlet
Patent Document 37 EP0607536 pamphlet
Non-Patent Document 1 Proc. Natl. Acad. Sci., 1992, 89, 4653
Non-Patent Document 2 Endocrinology, 1995, 137, 354
Non-Patent Document 3 J. Lipid. Res., 1996, 37, 907
Non-Patent Document 4 Nat. Med., 1998, 4, 1046.
Non-Patent Document 5 Cell, 2003, 113, 159.
Non-Patent Document 6 J. Biol. Chem., 1998, 273, 29577
Non-Patent Document 7 Proc. Natl. Acad. Sci., 1997, 94, 312

### DISCLOSURE OF THE INVENTION

### PROBLEMS TO BE SOLVED BY THE INVENTION

Accordingly, it is an object of the present invention to provide a compound that is structurally different from any of the above-described publicly known compounds, has a strong PPARα agonist activity and exhibits its strong effects *in vivo.*

### MEANS FOR SOLVING THE PROBLEMS

In our attempt to create effective, safe and structurally novel compounds that serve as therapeutic agents for hyperlipidemia with prolonged effect, the present inventors took advantage of the specific role of human PPARα in lipid metabolism and conducted extensive studies. Our studies have revealed that novel cyclic aminophenylalkanoic acid derivatives and their addition salts can effectively activate transcription of human PPARα and exhibit a marked ability to decrease the lipid levels *in vivo.*

Specifically, the present invention concerns (1) through (20) below.
(1) A cyclic aminophenylalkanoic acid derivative represented by the following general formula (1):

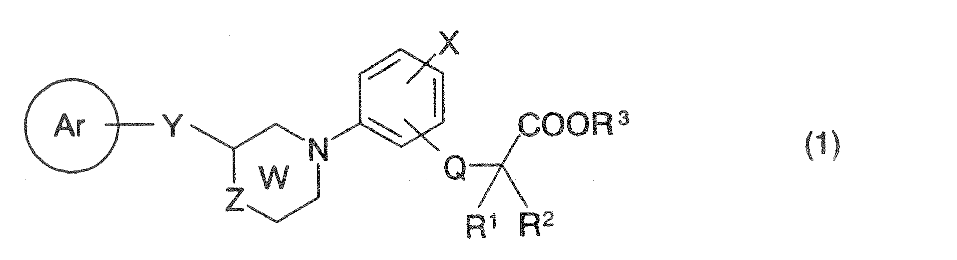

[wherein the ring Ar represents a substituted or unsubstituted aryl group or a substituted or unsubstituted 5- or 6-membered aromatic heterocyclic ring group and a fused ring group thereof;
Y represents C₁-C₄ alkylene, C₂-C₄ alkenylene, C₂-C₄ alkynylene, or the following general formula (2):

-T-A-U - (2)

(wherein T represents a single bond, C₁-C₄ alkylene, C₂-C₄ alkenylene or C₂-C₄ alkynylene;
U represents a single bond, C₁-C₄ alkylene or C₂-C₄ alkenylene; and
A represents a carbonyl group, an oxygen atom, a sulfur atom, -NR⁴- (wherein R⁴ represents a hydrogen atom, a lower alkyl group that may be substituted with a halogen atom(s), a substituted or unsubstituted aralkyl group, a substituted or unsubstituted aryl group or a substituted or unsubstituted 5- or 6-membered aromatic heterocyclic ring group and a fused ring thereof), the following general formula (3):

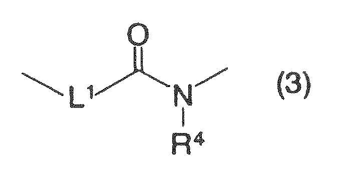

(wherein L¹ represents a single bond, an oxygen atom or -NR⁴- with R⁴ being the same as defined above), or the following general formula (4):

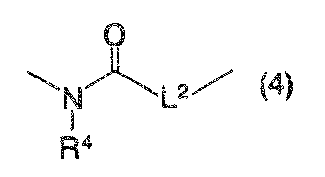

(wherein L² represents a single bond or an oxygen atom; R⁴ is as defined above));
the ring W represents a saturated heterocyclic ring containing a nitrogen atom N;
Z represents an oxygen atom, a sulfur atom or - (CH₂)ₙ- (wherein n represents an integer of 0,1 or 2);
X represents a hydrogen atom, a halogen atom, a lower alkyl group that may be substituted with a halogen atom (s), a lower alkoxy group that may be substituted with a halogen atom(s), a hydroxyl group, a nitro group, a cyano group, a substituted or unsubstituted amino group, a substituted or unsubstituted aryl group, a substituted or unsubstituted 5- or 6-membered aromatic heterocyclic group and a fused ring group thereof, a substituted or unsubstituted aralkyl group, a substituted or unsubstituted aryloxy group, orasubstituted or unsubstituted aralkyloxy group;
Q represents a single bond, methylene, an oxygen atom or a sulfur atom;
R¹ and R² each independently represent a hydrogen atom or a lower alkyl group that may be substituted with a halogen atom(s); and
R³ represents a hydrogen atom or a lower alkyl group, wherein -QCR¹R²COOR³ positioned ortho or meta to the ring W], and a pharmaceutically acceptable salt thereof.

(2) The cyclic aminophenylalkanoic acid derivative according to (1) above, and a pharmaceutically acceptable salt thereof, wherein in the general formula (1), Y represents the following general formula (2a) :

-T¹-A¹-U¹- (2a)

(wherein T¹ represents a single bond, C₁-C₄ alkylene or C₂-C₄ alkenylene; U¹ represents a single bond or C₁-C₄ alkylene; and
A¹ represents an oxygen atom, a sulfur atom, the following general formula (3):

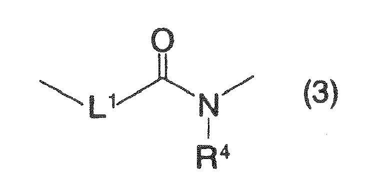

(wherein L¹ represents a single bond, an oxygen atom or -NR⁴- with R⁴ being the same as defined above), or the following general formula (4) :

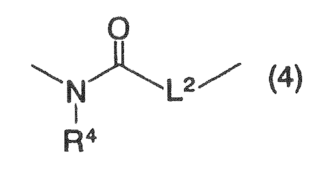

(wherein L² represents a single bond or an oxygen atom and R⁴ is as defined above)).

(3) The cyclic aminophenylalkanoic acid derivative according to (1) above, and a pharmaceutically acceptable salt thereof, wherein in the general formula (1), Y represents the following general formula (2b):

-T¹-A²-U¹- (2b)

(wherein T¹ represents a single bond, C₁-C₄ alkylene or C₂-C₄ alkenylene; U¹ represents a single bond or C₁-C₄ alkylene; and
A² represents an oxygen atom, a sulfur atom, the following general formula (3a):

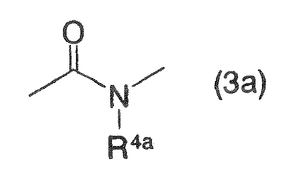

(wherein R^{4a} represents a hydrogen atom, an alkyl group that may be substituted with a halogen atom(s), or a substituted or unsubstituted aralkyl group), or the following general formula (4a) :

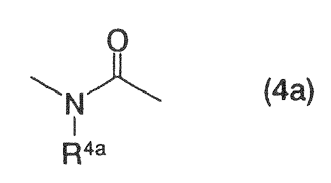

(wherein R^{4a} is as defined above)).

(4) The cyclic aminophenylalkanoic acid derivative according to (1) above, and a pharmaceutically acceptable salt thereof, wherein in the general formula (1), Y represents the following general formula (2c) :

-T¹-A³-U²- (2c)

(wherein T¹ represents a single bond, C₁-C₄ alkylene or C₂-C₄ alkenylene; U² represents a single bond or methylene; and
A³ represents the following general formula (3a):

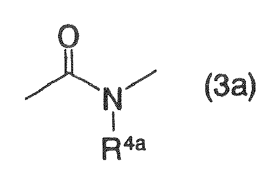

(wherein R^{4a} represents a hydrogen atom, an alkyl group that may be substituted with a halogen atom(s), or a substituted or unsubstituted aralkyl group), or the following general formula (4a) :

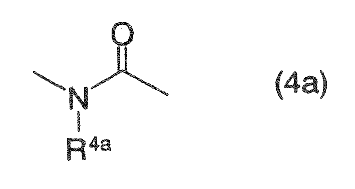

(wherein R^{4a} is as defined above)).

(5) The cyclic aminophenylalkanoic acid derivative according to any of (1) through (4) above, and a pharmaceutically acceptable salt thereof, wherein in the general formula (1), Z represents an oxygen atom, a sulfur atom or methylene.

(6) The cyclic aminophenylalkanoic acid derivative according to any of (1) through (5) above, and a pharmaceutically acceptable salt thereof, wherein in the general formula (1), Z represents methylene.

(7) The cyclic aminophenylalkanoic acid derivative according to any of (1) through (6) above, and a pharmaceutically acceptable salt thereof, wherein in the general formula (1), X represents a hydrogen atom, a halogen atom, a lower alkyl group that may be substituted with a halogen atom(s), a lower alkoxy group that may be substituted with a halogen atom(s), a hydroxyl group, or a substituted or unsubstituted amino group.

(8) The cyclic aminophenylalkanoic acid derivative according to any of (1) through (7) above, and a pharmaceutically acceptable salt thereof, wherein in the general formula (1), the ring Ar represents a substituted or unsubstituted 5- or 6-membered aromatic heterocyclic ring group.

(9) The cyclic aminophenylalkanoic acid derivative according to any of (1) through (8) above, and a pharmaceutically acceptable salt thereof, wherein in the general formula (1), the ring Ar represents the following general formula (5):

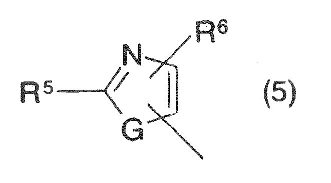

(wherein R⁵ represents a lower alkyl group that may be substituted with a halogen atom(s), a cyclic alkyl group, a lower alkoxy group that may be substituted with a halogen atom(s), a substituted or unsubstituted amino group, a 5- or 6- membered cyclic amino group, a substituted or unsubstituted aryl group, or a substituted or unsubstituted 5- or 6-membered aromatic heterocyclic ring group;
R⁶ represents a hydrogen atom or a lower alkyl group that may be substituted with a halogen atom(s) or a cycloalkyl group; and
G represents an oxygen atom or a sulfur atom).

(10) The cyclic aminophenylalkanoic acid derivative according to (1) above, and a pharmaceutically acceptable salt thereof, wherein the compound of the general formula (1) is
   2-[3-[[2-(4-chlorophenyl)-4-methylthiazol-5-yl]carbonylaminome thyl]piperidin-1-yl]phenylacetic acid,
   3-[3-[[2-(4-chlorophenyl)-4-methylthiazol-5-yl]carbonylaminome thyl]piperidin-1-yl]phenylacetic acid,
   3-[3-[[2-(4-chlorophenyl)-4-methylthiazol-5-yl]carbonylamino]p iperidin-1-yl]phenylacetic acid,
   2-[3-[3-[[2-(4-chlorophenyl)-4-methylthiazol-5-yl]carbonylamin o]piperidin-1-yl]phenoxy]-2-methylpropionic acid,
   (S)-2-[3-[[2-(4-chlorophenyl)-4-methylthiazol-5-yl]carbonylami nomethyl]piperidin-1-yl]phenylacetic acid,
   (R)-2-[3-[[2-(4-chlorophenyl)-4-methylthiazol-5-yl]carbonylami nomethyl]piperidin-1-yl]phenylacetic acid,
   (S)-3-[3-[[2-(4-chlorophenyl)-4-methylthiazol-5-yl]carbonylami nomethyllpiperidin-1-yllphenylacetic acid,
   (R)-3-[3-[[2-(4-chlorophenyl)-4-methylthiazol-5-yl]carbonylami nomethyl]piperidin-1-yl]phenylacetic acid,
   (S)-3-[3-[[2-(4-chlorophenyl)-4-methylthiazol-5-yl]carbonylami no]piperidin-1-yl]phenylacetic acid,
   (R)-3-[3-[[2-(4-chlorophenyl)-4-methylthiazol-5-yl]carbonylami no]piperidin-1-yl]phenylacetic acid,
   (S)-2-[3-[3-[[2-(4-chlorophenyl)-4-methylthiazol-5-yl]carbonyl aminolpiperidin-1-yllphenoxy]-2-methylpropionic acid, or
   (R)-2-[3-[3-[[2-(4-chlorophenyl)-4-methylthiazol-5-yl]carbonyl amino]piperidin-1-yl]phenoxy]-2-methylpropionic acid.

(11) A pharmaceutical product containing as an active ingredient at least one of the cyclic aminophenylalkanoic acid derivatives according to any of (1) to (10) above, and a pharmaceutically acceptable salt thereof.

(12) A PPARα agonist containing as an active ingredient at least one of the cyclic aminophenylalkanoic acid derivatives according to any of (1) to (10) above, and a pharmaceutically acceptable salt thereof.

(13) A PPARα/γ-dual agonist containing as an active ingredient at least one of the cyclic aminophenylalkanoic acid derivatives according to any of (1) to (10) above, and a pharmaceutically acceptable salt thereof.

(14) A PPARα/δ-dual agonist containing as an active ingredient at least one of the cyclic aminophenylalkanoic acid derivatives according to any of (1) to (10) above, and a pharmaceutically acceptable salt thereof.

(15) A PPARα/γ/δ-triple agonist containing as an active ingredient at least one of the cyclic aminophenylalkanoic acid derivatives according to any of (1) to (10) above, and a pharmaceutically acceptable salt thereof.

(16) A PPARα modulator containing as an active ingredient at least one of the cyclic aminophenylalkanoic acid derivatives according to any of (1) to (10) above, and a pharmaceutically acceptable salt thereof.

(17) A hypolipidemic agent containing as an active ingredient at least one of the cyclic aminophenylalkanoic acid derivatives according to any of (1) to (10) above, and a pharmaceutically acceptable salt thereof.

(18) A pharmaceutical composition for the prevention or treatment of arteriosclerosis containing as an active ingredient at least one of the cyclic aminophenylalkanoic acid derivatives according to any of (1) to (10) above, and a pharmaceutically acceptable salt thereof.

(19) A pharmaceutical composition for the prevention or treatment of diabetes containing as an active ingredient at least one of the cyclic aminophenylalkanoic acid derivatives according to any of (1) to (10) above, and a pharmaceutically acceptable salt thereof.

(20) A pharmaceutical composition for the prevention or treatment of obesity containing as an active ingredient at least one of the cyclic aminophenylalkanoic acid derivatives according to any of (1) to (10) above, and a pharmaceutically acceptable salt thereof.

### EFFECTS OF THE INVENTION

The novel cyclic aminophenylalkanoic acid derivatives of the present invention and addition salts thereof can effectively activate transcription of human PPARα and exhibit a marked ability to decrease the lipid levels *in vivo.*

The compounds of the present invention serve as an effective hypolipidemic agent that is particularly effective in lowering the lipid levels in liver and preventing the development of arteriosclerosis.

### BEST MODE FOR CARRYING OUT THE INVENTION

Terms regarding the compounds of the present invention represented by the general formula (1) will now be defined below.

As used herein, the term "halogen atom" refers to fluorine, chlorine, bromine or iodine.

As used herein, the term "lower alkyl group" refers to a straight-chained or branched alkyl group having 1 to 6 carbon atoms, such as methyl, ethyl, n-propyl and i-propyl.

As used herein, the term "cycloalkyl group" refers to those having 3 to 7 carbon atoms, such as cyclopropyl, cyclopentyl and cyclohexyl.

As used herein, the term "lower alkoxy group" refers to a straight-chained or branched alkoxy group having 1 to 5 carbon atoms, such as methoxy, ethoxy, n-propoxy and i-propoxy.

As used herein, the term "lower alkyl group that may be substituted with a halogen atom (s) " refers to a halogen-substituted lower alkyl group, such as chloromethyl group and trifluoromethyl group, as well as any of the above-described lower alkyl groups.

As used herein, the term "lower alkoxy group that may be substituted with a halogen atom(s) "refers to a halogen-substituted lower alkoxy group, such as trifluoromethoxy, as well as any of the above-described lower alkoxy groups.

As used herein, the term "aryl group" refers to an aromatic hydrocarbon group such as a phenyl group and a naphthyl group.

As used herein, the term "aryloxy group" includes a phenoxy group and a naphthoxy group.

As used herein, the term "aralkyl group" includes a benzyl group, a diphenylmethyl group, a triphenylmethyl group, a phenethyl group and a phenylpropyl group.

As used herein, the term "aralkyloxy group" includes a benzyloxy group and a phenethyloxy group.

The term "5- or 6-membered aromatic heterocyclic ring group" as in "5- or 6-membered aromatic heterocyclic ring group and a fused ring thereof" refers to a 5- or 6-membered aromatic ring group containing 1 to 3 nitrogen atoms, oxygen atoms or sulfur atoms. Examples include a furanyl group, a thienyl group, a pyrazolyl group, an imidazolyl group, an oxazolyl group, a thiazolyl group, an isoxazolyl group, an isothiazolyl group, a triazolyl group, an oxadiazolyl group, a thiadiazolyl group, a pyridyl group, a pyrimidyl group, a pyridazyl group and a pyratyl group. The term "fused ring thereof" refers to a benzene-fused ring of the above-described 5-or 6-membered aromatic heterocyclic ring group or a fused ring formed of any two of the 5- or 6-membered aromatic heterocyclic rings. Examples include an indolyl group, a benzoxazolyl group, a benzothiazolyl group, a benzofuranyl group, a benzothienyl group, a benzimidazolyl group, a quinolyl group, an isoquinolyl group, a quinazolyl group, a quinoxalyl group, an imidazopyridyl group, a pyrazolopyridyl group and an imidazopyrimidyl group. As used herein, the term "substituted or unsubstituted amino group" refers to an amino group that may be substituted with an acyl group, such as acetyl group, a lower alkylsulfonyl group that may be substituted with a halogen atom(s), such as a methanesulfonyl group and a trifluoromethanesulfonyl group, a substituted or unsubstituted arylsulfonyl group, such as a phenylsulfonyl group and a tolylsulfonyl group, a lower alkyl group that may be substituted with a halogen atom(s), a substituted or unsubstituted phenyl group, or a substituted or unsubstituted aralkyl group. As used herein, the term "5- or 6-membered cyclic amino group" includes a pyrrolidyl group, a piperidyl group, a piperadyl group, a morpholyl group and a thiomorpholyl group.

The term "substituent" as in "substituted or unsubstituted aryl group," "substituted or unsubstituted 5- or 6-membered heterocyclic ring and a fused ring thereof," "substituted or unsubstituted aralkyl group," " substituted or unsubstituted aralkyloxy group" and "substituted or unsubstituted aryloxy group" includes a halogen atom, a lower alkyl group that may be substituted with a halogen atom(s), a cycloalkyl group, a lower alkoxy group that may be substituted with a halogen atom(s), a lower alkylthio group, a lower alkoxycarbonyl group, a nitro group, a substituted or unsubstituted amino group, a 5- or 6-membered cyclic amino group, a cyano group, a carboxyl group, an aldehyde group, a substituted or unsubstituted aryl group, a substituted or unsubstituted aralkyl group, a substituted or unsubstituted aralkyloxy group, a substituted or unsubstituted aryloxy group, and a substituted or unsubstituted 5- or 6-membered aromatic heterocyclic ring and a fused ring thereof. As used herein, the term "lower alkylthio group" refers to a straight-chained or branched alkylthio group having 1 to 5 carbon atoms, such as a methylthio group, an ethylthio group and a propylthio group. As used herein, the term "lower alkoxycarbonyl group" refers to a straight-chained or branched alkoxycarbonyl group having 1 to 6 carbon atoms, such as a methoxycarbonyl group and an ethoxycarbonyl group. The term "substituent" as used herein refers to any of the above-described substituents.

When necessary, the compounds of the present invention represented by the general formula (1) may be provided in the form of pharmaceutically acceptable salts. Examples of such salts include salts formed with inorganic acids such as hydrochloric acid, hydrobromic acid or sulfuric acid, salts formed with organic acids such as acetic acid, fumaric acid, maleic acid, oxalic acid, citric acid, methanesulfonic acid and tosic acid, and salts formed with bases such as sodium salts, potassium salts and calcium salts.

The compounds of the present invention represented by the general formula (1) and pharmaceutically acceptable salts thereof may be provided in the form of intramolecular salts, anhydrides, hydrates or solvates.

The compounds of the present invention represented by the general formula (1) include optical isomers, geometric isomers, stereoisomers and tautomers depending on the asymmetric carbon atoms present. Such isomers and mixtures thereof are also encompassed by the scope of the invention.

The compounds of the present invention represented by the general formula (1) can be produced by Production Process 1 shown below, though they may also be produced by combinations of known techniques.

[Production Process 1]

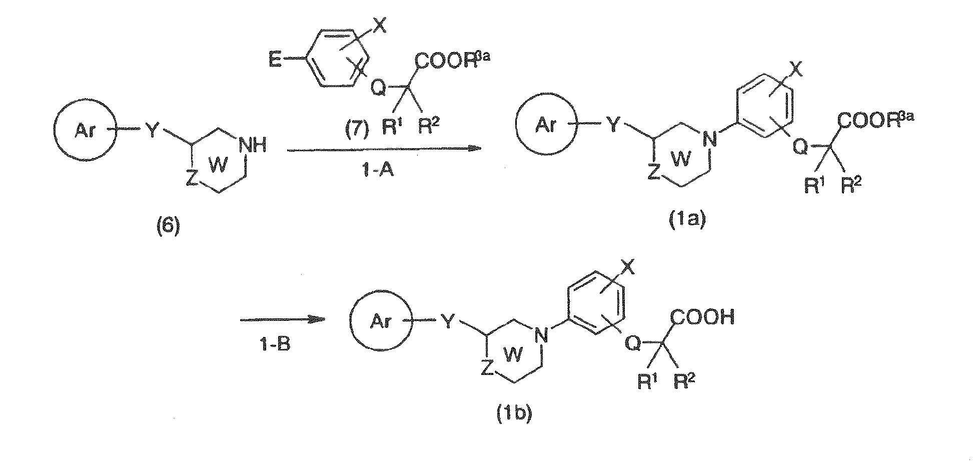

[wherein E represents a leaving group; R^{3a} represents a lower alkyl group; the Ar, R¹, R², Q, W, X, Y and Z are as defined above].
The leaving group E may be a halogen atom, a sulfonyloxy group, such as a trifluoromethanesulfonyloxy group and a p-tolylsulfonyloxy group, a trialkylstanyl group, such as a trimethylstanyl group, or (HO)₂B-.

When the leaving group E in the general formula (7) is a halogen atom or a sulfonyloxy group, the conversion from the general formula (6) or (7) into the general formula (1a) (Step 1-A) is carried out at room temperature to 120 °C for 12 to 48 hours by using a palladium catalyst, such as palladium (II) acetate, tris(dibenzilideneacetone)dipalladium(0) and [1,1'-bis(diphenylphosphino)ferrocene]dichloropalladium(II), or a nickel catalyst, such as bis(1,5-cyclooctadiene)nickel(0). The reaction is carried out in a suitable solvent, such as toluene, 1,4-dioxane, t-butylalcohol, N,N-dimethylformamide and tetrahydrofuran or a mixture thereof, and in the presence of a ligand, such as 2,2'-bis(diphenylphosphino)-1,1'-binaphthyl, 1,1'-bis(diphenylphosphino)ferrocene, and a base, such as sodium carbonate, potassium carbonate, cesium carbonate, sodium tert-butoxide, potassium tert-butoxide, potassium phosphate tribasic, triethylamine and pyridine.

When the leaving group E in the general formula (7) is a halogen atom, Step 1-A is carried at room temperature to 160°C for 1 to 70 hours in a suitable solvent, such as toluene, N,N-dimethylformamide, N,N-dimethylacetamide, dimethylsulfoxide, isopropanol, 1,2-dimethoxyethane and 1,4-dioxane, and in the presence of a base, such as potassium carbonate, sodium carbonate, cesium carbonate, cesium acetate and potassium phosphate tribasic. When necessary, the reaction may involve a copper (I) salt, such as copper (I) iodide and copper (I) bromide, a ligand, such as proline, N-methylglycine, ethyleneglycol and ethylenediamine, and a phase transfer catalyst, such as tetrabutylammonium iodide.

When the leaving group E in the general formula (7) is a trialkylstanyl group or (HO)₂B-, the reaction is carried out at 0 to 60°C for 6 to 70 hours in a suitable solvent, such as dichloromethane, 1,4-dioxane, N-methylpyrrolidone, tetrahydrofuran, N,N-dimethylformamide and N,N-dimethylacetamide, in the presence of copper (II) acetate and a base, such as triethylamine, pyridine, 2,6-lutidine, tetrabutylammonium fluoride. When necessary, molecular sieves and a suitable reaction aid, such as a co-oxidant (such as pyridine N-oxide, 1,1,6,6-tetramethylpiperidinyloxy radical and myristic acid) may be used.

The conversion from the general formula (1a) into the general formula (1b) (Step 1-B) is carried out by hydrolyzing the general formula (1a) at 0 to 100°C for 1 to 48 hours. Specifically, the reaction is carried out in the absence or in the presence of a suitable solvent, such as water, acetic acid, methanol, ethanol, tetrahydrofuran, 1,4-dioxane and mixtures thereof, and involves an acid, such as hydrochloric acid, sulfuric acid and nitric acid, or a base, such as lithium hydroxide, sodium hydroxide, potassium hydroxide, sodium carbonate and potassium carbonate.

Of the compounds represented by the general formula (1), those shown by the general formula (1c) or (1d) may be synthesized by Production Process 2 shown below.

[Production Process 2]

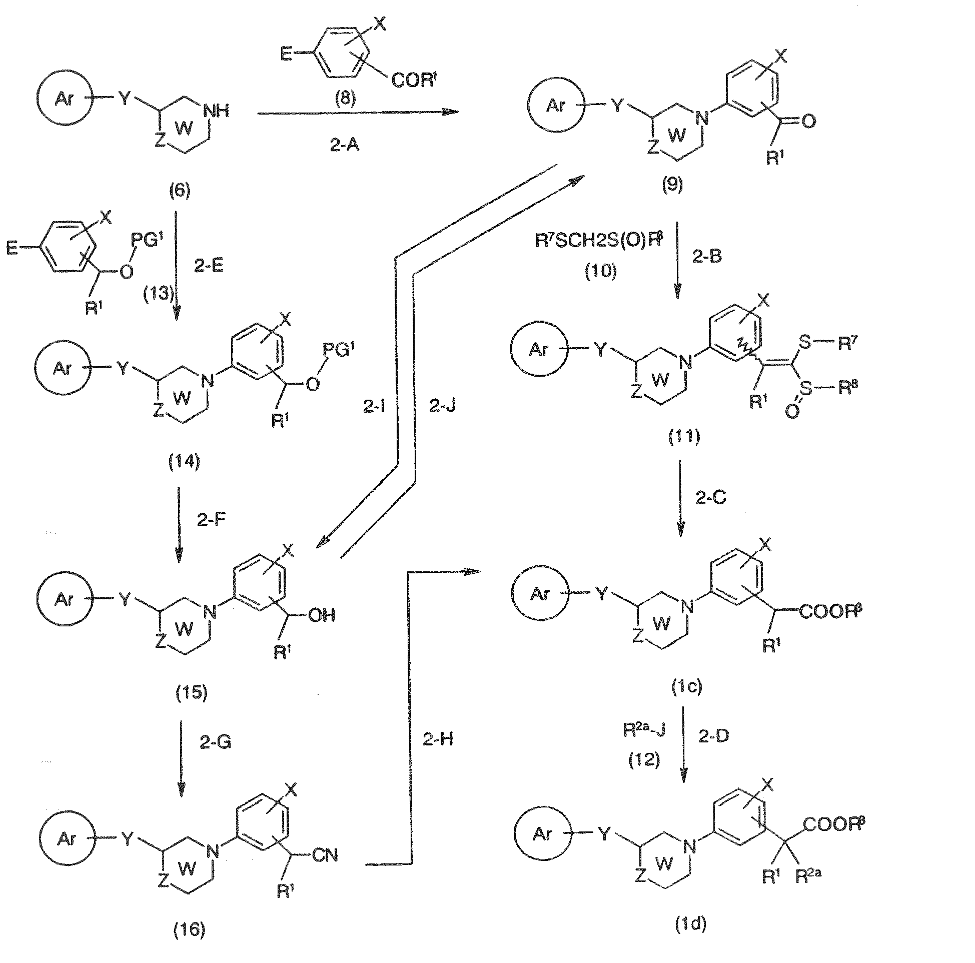

[wherein R^{2a} represents a lower alkyl group that may be substituted with a halogen atom(s); R⁷ and R⁸ are each independently a lower alkyl group; J represents a leaving group; PG¹ represents a protective group or a hydrogen atom; the wavy line represents an E-form or Z-form; and the ring Ar, R¹, R³, W, X, Y, Z and E are as defined above].

The leaving group J may be a halogen atom, or a lower alkylsulfonyloxy group that may be substituted with a halogen atom(s), such as a methane sulfonyloxy group and a trifluoromethanesulfonyloxy group, or an arylsulfonyloxy group that may be substituted with a lower alkyl group, such as a phenylsulfonyloxy group and p-tolylsulfonyloxy group. The protective group PG¹ may be an acyl group, such as an acetyl group, a lower alkoxycarbonyl group such as tert-butoxycarbonyl group, a substituted or unsubstitued aryloxycarbonyl group, such as a benzyloxycarbonyl group, a substituted or unsubstituted aralkyl group, such as a benzyl group andap-methoxybenzyl group, orasilylgroup, such as a trimethylsilyl group and a tert-butyldimethylsilyl group.

The conversion of the general formula (6) and the general formula (8) into the general formula (9) (Step 2-A) may be carried out as in Step 1-A.

The conversion of the general formula (9) and the general formula (10) into the general formula (11) (Step 2-B), as well as the conversion of the general formula (11) into the general formula (1c) (Step 2-C), may be carried out by a known process (See, for example, Journal of Synthetic Organic Chemistry, Japan, 37(1979) 903-913).

For example, the conversion of the general formula (9) and the general formula (10) into the general formula (11) (Step 2-B) can be carried out at room temperature to 100°C for 1 to 24 hours in a suitable solvent, such as methanol, ethanol, isopropyl alcohol, tetrahydrofuran, 1,4-dioxane and mixtures thereof, in the presence of a base, such as benzyltrimethylammonium hydroxide, sodium hydroxide and potassium hydroxide.

Alternatively, the general formula (10) may be reacted with the general formula (9) at -78°C to room temperature in a suitable solvent, such as tetrahydrofuran and 1,4-dioxane, in the presence of a base, such as butyl lithium and lithium diisopropylamide. Without purification, the product is reacted with an acid anhydride, such as acetic anhydride, or a acid halide, such as acetyl chloride, at -78°C to room temperature for 3 to 10 hours. The reaction is further carried out at 0 to 100°C for 3 to 30 hours in a suitable solvent, such as methanol, ethanol, tetrahydrofuran, 1,4-dioxane and mixtures thereof, in the presence of a base, such as sodium hydroxide, sodium methoxide and potassium tert-butoxide.

The conversion of the general formula (11) into the general formula (1c) (Step 2-C) may be carried out at 0 to 100°C for 0.5 to 12 hours in the absence or in the presence of a suitable solvent, such as water, methanol, ethanol, tetrahydrofuran, 1,4-dioxane and mixtures thereof, and in the presence of an acid, such as acetic acid, hydrochloric acid and sulfuric acid.

The conversion of the general formula (1c) and the general formula (12) into the general formula (1d) (Step 2-D) can be carried out by reacting the general formula (1c) with the general formula (12) at -78°C to 120°C for 0.5 to 12 hours in a suitable solvent, such as tetrahydrofuran, 1,4-dioxane, N-methylpyrrolidone, N,N-dimethylformamide, N,N-dimethylacetamide, hexamethylphosphoric acid triamide and mixtures thereof, in the presence of a base, such as sodium hydride, sodium amide, butyl lithium and lithium diisopropylamide.

The conversion of the general formula (6) and the general formula (13) into the general formula (14) (Step 2-E) may be carried out as in Step 1-A.

The conversion of the compound of the general formula (14), in which PG¹ represents a protective group, into the general formula (15) (Step 2-F) can be carried out by a common deprotection process (for example, see, Protecting Groups in Organic Synthesis (John Wily and Sons (1999)). Among known deprotection processes are those involving an acid, a base, ultraviolet rays, hydrazine, tetrabutylammonium fluoride and trimethylsilyl iodide, and reduction.

The conversion of the general formula (15) into the general formula (16) (Step 2-G) can be carried out as follows: First, the general formula (15) is reacted with a halogenating agent, such as thionyl chloride, phosphorus oxychloride and thionyl bromide, at -20 to 80°C for 0.5 to 6 hours in the absence or in the presence of a suitable solvent, such as dichloromethane, chloroform, tetrahydrofuran, benzene and mixtures thereof. When necessary, a base, such as triethylamine and pyridine, may be used. Alternatively, the general formula (15) is reacted with a suitable sulfonylating agent, such as methanesulfonyl chloride and trifluoromethanesulfonic anhydride, at -20 to 60°C for 0.5 to 3 hours in a suitable solvent, such as dichloromethane, tetrahydrofuran, N,N-dimethylformamide and mixtures thereof, and in the presence of a base, such as triethylamine and pyridine. Subsequently, the reaction is carried out at 0 to 100°C for 1 to 12 hours in a suitable solvent, such as dimethylsulfoxide, N-methylpyrrolidone, N,N-dimethylformamide, N,N-dimethylacetamide and acetonitrile, or in a two-phase system such as water/benzene, in the presence of a cyanide, such as sodium cyanide and potassium cyanide. When necessary, a catalyst, such as 18-crownether-6, or a phase transfer catalyst, such as tetrabutylammonium chloride, may be used.

Alternatively, the general formula (15) may be reacted with a cyanide, such as sodium cyanide and potassium cyanide, at room temperature to reflux temperature for 1 to 12 hours in a suitable solvent, such as dimethylsulfoxide, in the presence of triphenylphosphine or carbon tetrachloride.

The conversion of the general formula (16) into the general formula (1c) (Step 2-H) can be carried by hydrolyzing the general formula (16). Specifically, the reaction is carried out at 0 to 200°C for 1 to 48 hours in the absence or in the presence of a suitable solvent, such as water, acetic acid, methanol, ethanol, ethylene glycol, tetrahydrofuran, 1,4-dioxane and mixtures thereof, and an acid, such as hydrochloric acid, sulfuric acid and nitric acid, or a base, such as lithium hydroxide, sodium hydroxide, potassium hydroxide, sodium carbonate and potassium carbonate.

The conversion of the general formula (9) into the general formula (15) (Step 2-I) can be carried out at 0 to 100°C for 0.5 to 12 hours in a suitable solvent, such as methanol, ethanol, tetrahydrofuran, 1,4-dioxane, diethyl ether and mixtures thereof, in the presence of a reducing agent, such as sodium borohydride, lithium borohydride, sodium cyanoborohydride and lithium aluminum hydride.

The conversion of the general formula (15) into the general formula (9) (Step 2-J) can be carried out at 0 to 60°C for 0.5 to 24 hours in a suitable solvent, such as benzene, toluene, chloroform, dichloromethane and tetrahydrofuran, in the presence of an oxidizing agent, such as manganese dioxide.

Of the compounds represented by the general formula (1), those shown by the general formula (1e) or (1f) may be synthesized by Production Process 3 shown below.

[Production Process 3]

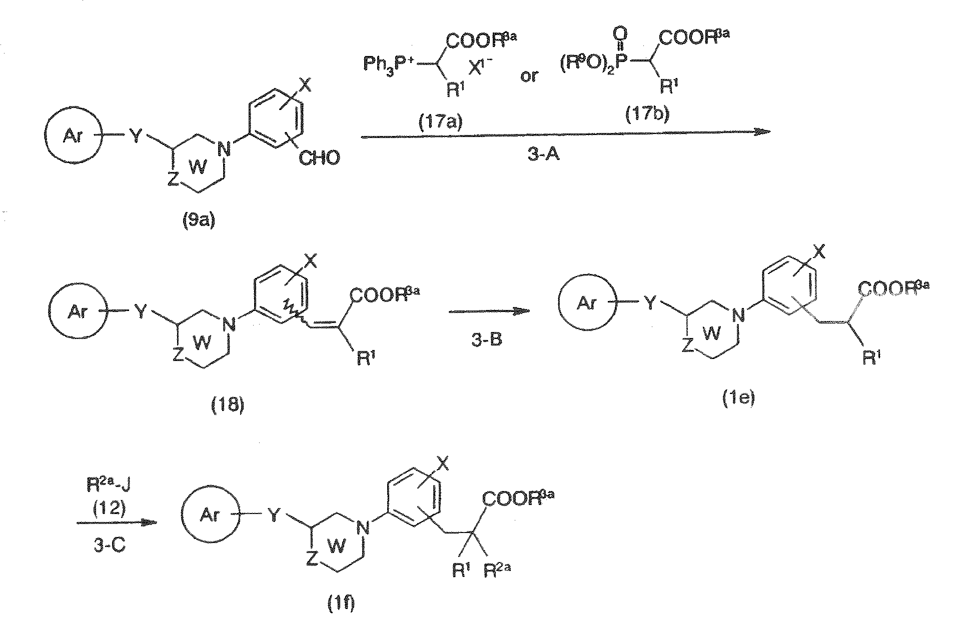

[wherein X¹ represents a halogen atom; R⁹ represents a lower alkyl group; and the ring Ar, R¹, R^{2a}, R^{3a}, W, X, Y, Z and the wavy line are as defined above].

The conversion of the general formula (9a) into the general formula (18) (Step 3-A) can be carried out by the Wittig reaction using the general formula (17a) or by the Horner-Wadsworth-Emmons reaction using the general formula (17b). For example, the general formula (17a) or (17b) is reacted with the general formula (9a) at -78 to 120°C for 1 to 12 hours in a suitable solvent, such as benzene, toluene, tetrahydrofuran, diethyl ether, 1,4-dioxane, dimethylsulfoxide or mixtures thereof, in the presence of a base, such as sodium hydride, lithium diisopropylamide, lithium bis(trimethylsilyl)amide, potassium tert-butoxide and sodium hydroxide.

The conversion of the general formula (18) into the general formula (1e) (Step 3-B) can be carried out by reducing the general formula (18) at 0 to 80°C for 0.5 to 12 hours in a suitable solvent, such as methanol, ethanol, ethyl acetate, tetrahydrofuran, N,N-dimethylformamide and mixtures thereof, in the presence of a metal catalyst, such as palladium-active carbon, palladium-active carbon/ethylene diamine complex, platinum/active carbon, platinum oxide and rhodium/alumina, in a hydrogen atmosphere under atmospheric pressure to 0.5 MPa.

The conversion of the general formula (1e) into the general formula (1f) (Step 3-C) can be carried out as in Step 2-D.

Of the compounds represented by the general formula (1), those shown by the general formula (1g) may be synthesized by Production Process 4 shown below.

[Production Process 4]

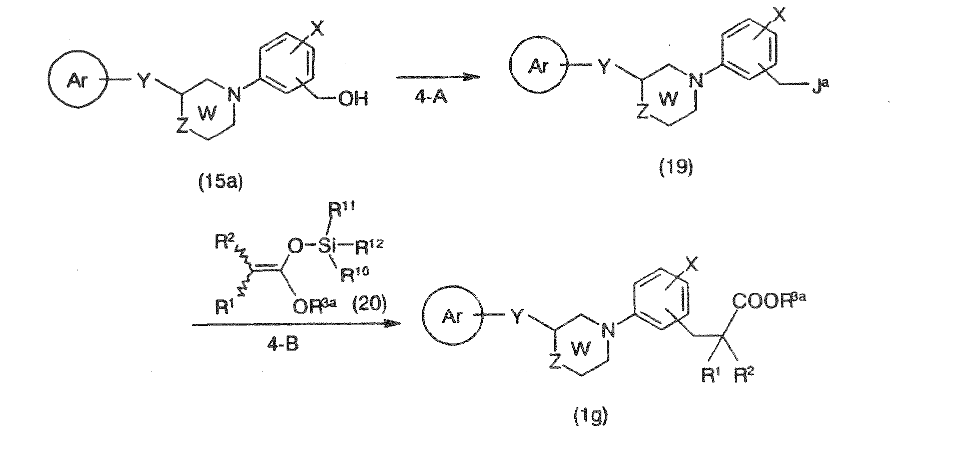

[wherein J^{a} represents a leaving group; R¹⁰, R¹¹ and R¹² are each independently an aryl group or a lower alkyl group; and the ring Ar, R¹, R², R^{3a}, W, X, Y, Z and the wavy line are as defined above].

The leaving group J^{a} may be a halogen atom or an acyloxy group, such as an acetoxy group and a benzoyl group.

When the leaving group J^{a} of the general formula (19) represents a halogen atom, the conversion of the general formula (15a) into the general formula (19) (Step 4-A) can be carried out at -20 to 80°C for 0.5 to 6 hours in the absence or in the presence of a suitable solvent, such as dichloromethane, chloroform, tetrahydrofuran, benzene and mixtures thereof, and in the presence of a halogenating agent, such as thionyl chloride, phosphorus oxychloride and thionyl bromide. When the leaving group J^{a} of the general formula (19) represents an acyloxy group, the reaction can be carried out at -20 to 60°C for 0.5 to 6 hours in a suitable solvent, such as ethyl acetate, dichloromethane, tetrahydrofuran, diethyl ether, 1,4-dioxane, N,N-dimethylformamide and mixtures thereof, in the presence of a base, such as triethylamine and pyridine, and a suitable acylating agent, such as acetic anhydride, acetyl chloride and benzoyl chloride.

The conversion of the general formula (19) and the general formula (20) into the general formula (1g) (Step 4-B) can be carried out by a known process (See, for example, Tetrahedron Letters 38(1997): 2645-2648).

For example, the reaction may be carried out at -20 to 80°C for 1 to 24 hours in a suitable solvent, such as dichloromethane, tetrahydrofuran, benzene, toluene, 1,4-dioxane and mixtures thereof, in the presence of a Lewis acid, such as magnesium perchlorate, magnesium bistrifluoromethanesulfonylimide, titanium tetrachloride and tin tetrachloride.

Of the compounds represented by the general formula (1), those shown by the general formula (1h) may be synthesized by Production Process 5 shown below.

[Production Process 5]

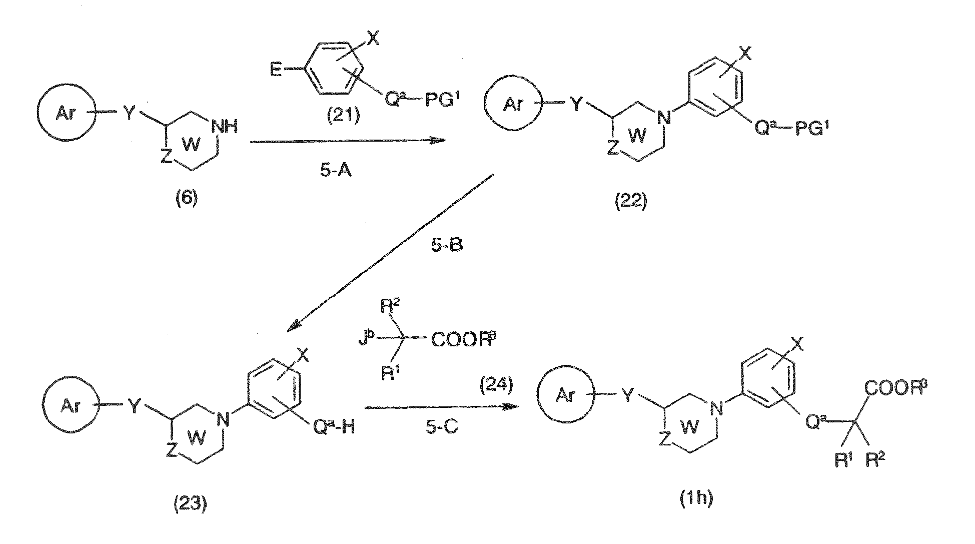

[wherein J^{b} represents a leaving group or a hydroxyl group; Q^{a} represents an oxygen atom or a sulfur atom; and the Ar, R¹, R², R³, W, X, Y, Z, E, PG¹ and wavy line are as defined above].

The leaving group J^{b} may be a halogen atom, a lower alkylsulfonyloxy group that may be substituted with a halogen atom(s), such as amethanesulfonyloxy group and a trifluoromethanesulfonyloxy group, or an arylsulfonyloxy group that may be substituted with a lower alkyl group, such as a phenylsulfonyloxy group and a p-tolylsulfonyloxy group.

The conversion of the general formula (6) and the general formula (21) into the general formula (22) (Step 5-A) can be carried out as in Step 1-A.

The conversion of the compound of the general formula (22), in which PG¹ represents a protective group, into the general formula (23) (Step 5-B) can be carried out as in Step 2-F.

When the substituent J^{b} in the general formula (24) is a leaving group, the conversion of the general formula (23) and the general formula (24) into the general formula (1h) (Step 5-C) can be carried out at room temperature to 150°C for 1 to 48 hours in a suitable solvent, such as methanol, ethanol, tetrahydrofuran, 1,4-dioxane, N,N-dimethylformamide, N,N-dimethylacetamide, N-methylpyrrolidone, dimethylsulfoxide and mixtures thereof, in the presence of abase, such as sodium hydroxide, potassium hydroxide, sodium carbonate, potassium carbonate, sodium hydride, sodium methoxide and potassium tert-butoxide.

When the substituent J^{b} in the general formula (24) is a hydroxyl group, the reaction can be carried out at 0 to 60°C for 3 to 24 hours in a suitable solvent, such as toluene, hexane, tetrahydrofuran and mixtures thereof, in the presence of an organic phosphorus compound, such as triphenylphosphine and tributylphosphine, and in the presence of an electrophile, such as diethyl azodicarboxylate, diisopropyl azodicarboxylate and dipiperidine azodicarboxylate. Alternatively, the reaction may be carried out at room temperature to 120°C for 1 to 24 hours in a suitable solvent, such as toluene, benzene, hexane, tetrahydrofuran and mixtures thereof, in the presence of a phosphorane compound, such as cyanomethylene tributylphosphorane and cyanomethylene trimethylphosphorane.

Of the compounds represented by the general formula (1), those shown by the general formula (1i) may be synthesized by Production Process 6 shown below.

[Production Process 6]

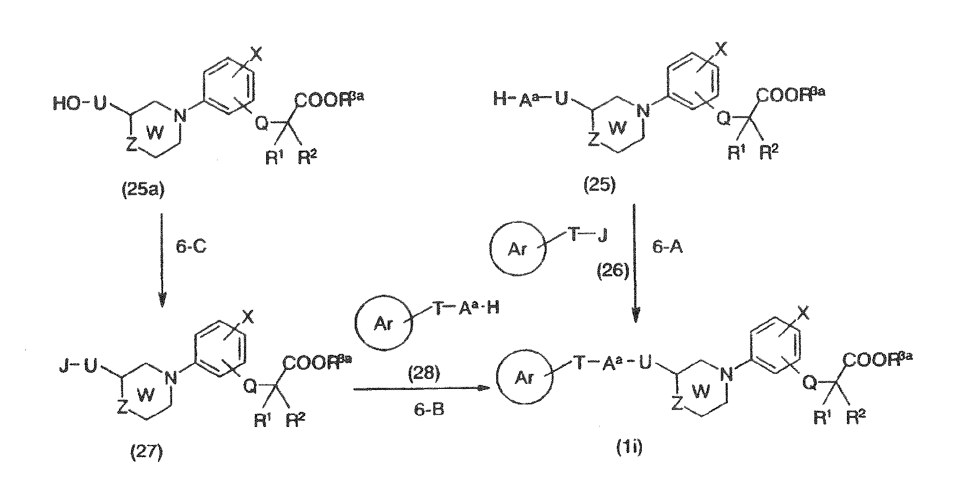

[wherein A^{a} is an oxygen atom, a sulfur atom or -NR⁴-; and the Ar, J, Q, T, U, W, X, Z, R¹, R², R^{3a} and R⁴ are as defined above].

The conversion of the general formula (25) and the general formula (26) into the general formula (1i) (Step 6-A) can be carried out at 15 to 120°C for 1 to 24 hours in a suitable solvent, such as toluene, hexane, tetrahydrofuran, diethyl ether, dichloromethane, N,N-dimethylformamide, dimethylsulfoxide, acetone and mixtures thereof, in the presence of a base, such as sodium hydroxide, potassium hydroxide, sodium carbonate, potassium carbonate, sodium hydride, sodium methoxide, potassium tert-butoxide, pyridine, triethylamine and N,N-dimethylaniline. When necessary, a suitable iodide, such as sodium iodide and tetrabutylammonium iodide, may be added.

The conversion of the general formula (27) and the general formula (28) into the general formula (1i) (Step 6-B) can be carried out as in Step 6-A.

The conversion of the general formula (25a) into the general formula (27) (Step 6-C) can be carried out at -20 to 80°C for 0.5 to 6 hours in the absence or in the presence of a suitable solvent, such as dichloromethane, chloroform, tetrahydrofuran, benzene and mixtures thereof, and in the presence of a halogenating agent, such as thionyl chloride, phosphorus oxychloride and thionyl bromide. When necessary, abase, suchaspyridine, maybeused. Alternatively the reaction may be carried out at -20 to 60°C for 0.5 to 3 hours in a suitable solvent, such as dichloromethane, tetrahydrofuran, diethyl ether, 1,4-dioxane, N,N-dimethylformamide and mixtures thereof, in the presence of a bave, such as triethylamine and pyridine, and a suitable sulfonylating agent, such as methanesulfonyl chloride and trifluoromethanesulfonic anhydride.

Alternatively, the reaction may be carried out at -20 to 60°C for 0.5 to 6 hours in the absence or in the presence of a suitable solvent, such as dichloromethane, chloroform, tetrahydrofuran, toluene, benzene and mixtures thereof, and in the presence of triphenylphosphine (when necessary, imidazole may be added), and carbon tetrabromide, carbon tetrachloride or iodine.

Of the compounds represented by the general formula (1), those shown by the general formula (1j) may be synthesized by Production Process 7 shown below.
[Production Process 7]

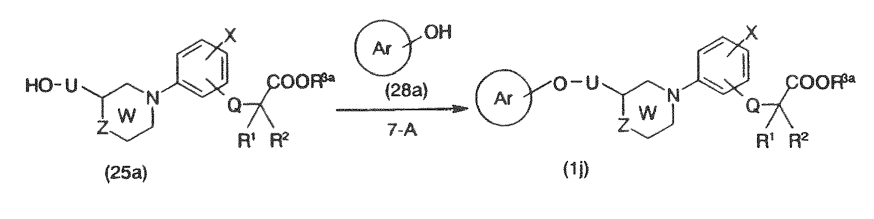

[wherein the ring Ar, Q, U, W, X, Z, R¹, R² and R^{3a} are as defined above].
The conversion of the general formula (25a) and the general formula (28a) into the general formula (1j) (Step 7-A) can be carried out at 0 to 60°C for 3 to 24 hours in a suitable solvent, such as toluene, hexane, tetrahydrofuran, N,N-dimethylformamide, N,N-dimethylacetamide and mixtures thereof, in the presence of an organic phosphorus compound, such as triphenylphosphine and tributylphosphine, and in the presence of an electrophile, such as diethyl azodicarboxylate, diisopropyl azodicarboxylate and dipiperidine azodicarboxylate. Alternatively, the reaction may be carried out at room temperature to 120°C for 1 to 24 hours in a suitable solvent, such as toluene, benzene, hexane, tetrahydrofuran and mixtures thereof, in the presence of a phosphorane compound, such as cyanomethylene tributylphosphorane and cyanomethylene trimethylphosphorane.

Of the compounds represented by the general formula (1), those shown by the general formula (1k) may be synthesized by Production Process 8 shown below.

[Production Process 8]

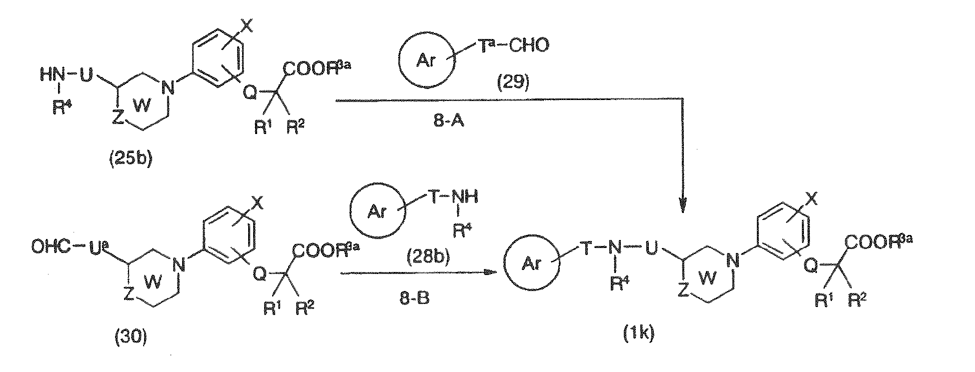

[wherein T^{a} represents a single bond, C₁-C₃ alkylene, C₂-C₃ alkenylene or C₂-C₃ alkynylene; U^{a} represents a single bond, C₁-C₃ alkylene or C₂-C₃ alkenylene; and the ring Ar, Q, T, U, W, X, Z, R¹, R², R^{3a} and R⁴ are as defined above].

The conversion of the general formula (25b) and the general formula (29) into the general formula (1k) (Step 8-A) can be carried out at 0 to 60 °C for 1~24 hours in a suitable solvent, such as methanol, ethanol, dichloromethane, chloroform, tetrahydrofuran and mixtures thereof, in the presence of a reducing agent, such as lithium borohydride, sodium borohydride, sodium cyanoborohydride and sodium triacetoxyborohydride. When necessary, an acid, such as hydrochloric acid, hydrobromic acid and acetic acid, or a Lewis acid, such as aluminum chloride and zinc chloride, may be added.

The conversion of the general formula (30) and the general formula (28b) into the general formula (1k) (Step 8-B) canbe carried out as in Step 8-A.

Of the compounds represented by the general formula (1), those shown by the general formula (11) or (1m) may be synthesized by Production Process 9 shown below.

[Production Process 9]

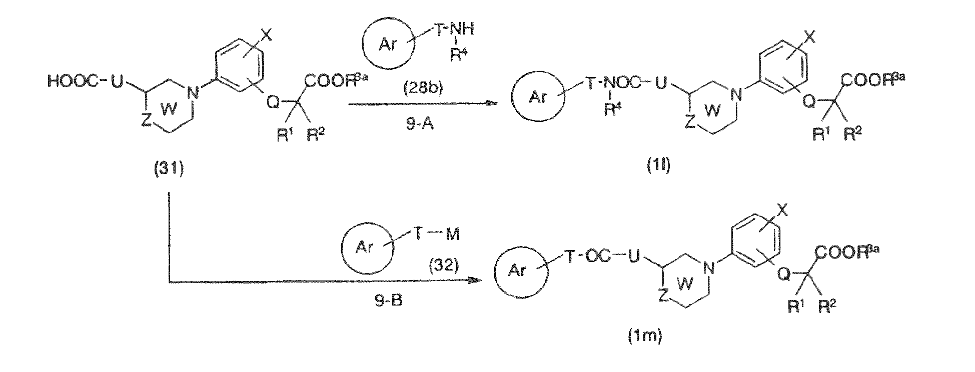

[wherein M represents a lithium atom, a copper atom or -M^{g} X¹ (where X¹ represents a halogen atom); and the ring Ar, Q, T, U, W, X, Z, R¹ R², R^{3a} and R⁴ are as defined above].

The conversion of the general formula (31) and the general formula (28b) into the general formula (11) (Step 9-A) can be carried out at -15 to 120°C for 1 to 24 hours. Specifically, the reaction is carried out in a suitable solvent, such as dichloromethane, chloroform, tetrahydrofuran, diethyl ether, N,N-dimethylformamide and mixtures thereof, in the presence of a base, such as pyridine, triethylamine, N-methylmorpholine and 4-(dimethylamino)pyridine, and a condensing agent, such as dicyclohexylcarbodiimide, 3-(3-dimethylaminopropyl)-1-ethylcarbodiimide hydrochloride, diethyl cyanophosphate, diphenylphosphoric acid azide and carbonyl diimidazole. When necessary, a reaction aid, such as N-hydroxybenzotriazole, N-hydroxysucclnimide and 3,4-dihydro-3-hydroxy-4-oxo-1,2,3-benzotriazine, may be added.

Alternatively, the reaction may be carried out as follows: the general formula (31) is first reacted with thionyl chloride, thionyl bromide, acetic anhydride or ethyl chlorocarbonate at -15 to 50°C for 5min to 3 hours to form an acid chloride, an acid bromide or an acid anhydride. This converts the carboxyl group into a reactive derivatizing group. The reaction can be carried out in the absence or in the presence of a suitable solvent, such as toluene, tetrahydrofuran, dichloromethane, N,N-dimethylformamide and mixtures thereof, and, when necessary, in the presence of a base, such as pyridine and triethylamine. Subsequently, the reaction product was reacted with the general formula (28b) at -15 to 50°C for 30 min to 6 hours in a suitable solvent, such as toluene, tetrahydrofuran, dichloromethane, N,N-dimethylformamide and mixtures thereof, in the presence of a base, such as pyridine, triethylamine and 4-(dimethylamino)pyridine.

The conversion of the general formula (31) and the general formula (32) into the general formula (1m) (Step 9-B) can be carried out as follows: The general formula (31) is first reacted with thionyl chloride, thionylbromide, acetic anhydride or ethyl chlorocarbonate at -15 to 50°C for 5 min to 3 hours to form an acid chloride, an acid bromide or an acid anhydride. This converts the carboxyl group into a reactive derivatizing group. The reaction may be carried out in the absence or in the presence of a suitable solvent, such as toluene, tetrahydrofuran, dichloromethane and mixtures thereof, and when necessary, in the presence of a base, such as pyridine and triethylamine. Subsequently, the reaction product is reacted with the general formula (32) at -78 to 50°C for 0.5 to 12 hours in a suitable solvent, such as toluene, benzene, tetrahydrofuran, diethyl ether, 1,4-dioxane and mixtures thereof.

Of the compounds represented by the general formula (1), those shown by the general formula (1m) may be synthesized by Production Process 10 shown below.

[Production Process 10]

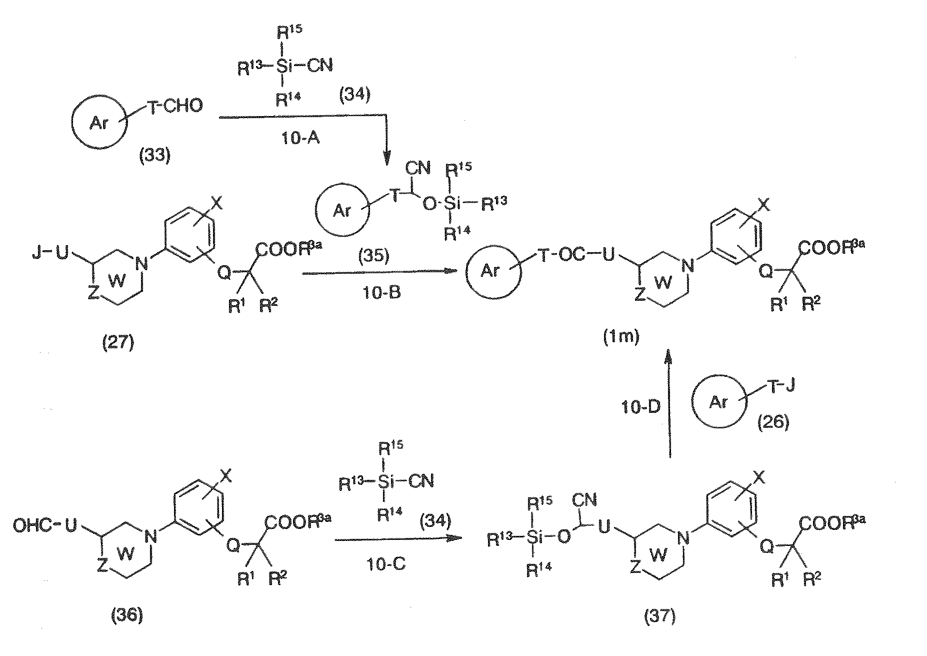

[wherein R¹³, R¹⁴ and R¹⁵ are each independently a lower alkyl group or an aryl group; and the ring Ar, Q, T, U, W, X, Z, R¹, R², R^{3a} and J are as defined above].

The conversion of the general formula (33) and the general formula (34) into the general formula (35) (Step 10-A) can be carried out at 0 to 60°C for 3 to 24 hours in the absence or in the presence of a suitable solvent, such as dichloromethane, chloroform, tetrahydrofuran, diethyl ether, 1,4-dioxane, acetonitrile and mixtures thereof, and when necessary, in the presence of a Lewis acid, such as zinc iodide.

The conversion of the general formula (27) and the general formula (35) into the general formula (1m) (Step 10-B) can be carried out as follows: The general formula (35) is first reacted with the general formula (27) at -78 to 60°C for 1 to 12 hours in a suitable solvent, such as benzene, toluene, tetrahydrofuran, diethyl ether, 1,4-dioxane, N,N-dimethylformamide and mixtures thereof, and in the presence of a base, such as sodium hydride, lithium diisopropylamide, lithium bis(trimethylsilyl)amide and potassium tert-butoxide. Subsequently, the reaction product is reacted with an acid, such as hydrochloric acid, sulfuric acid and nitric acid, at 0 to 100°C for 1 to 48 hours in the absence or in the presence of a suitable solvent, such as water, acetic acid, methanol, ethanol, ethylene glycol, tetrahydrofuran, 1,4-dioxane and mixtures thereof.

The conversion of the general formula (36) and the general formula (34) into the general formula (37) (Step 10-C) can be carried out as in Step 10-A.

The conversion of the general formula (37) and the general formula (26) into the general formula (1m) (Step 10-D) can be carried out as in Step 10-B.

Of the compounds represented by the general formula (1), those shown by the general formula (In) may be synthesized by Production Process 11 shown below.

[Production Process 11]

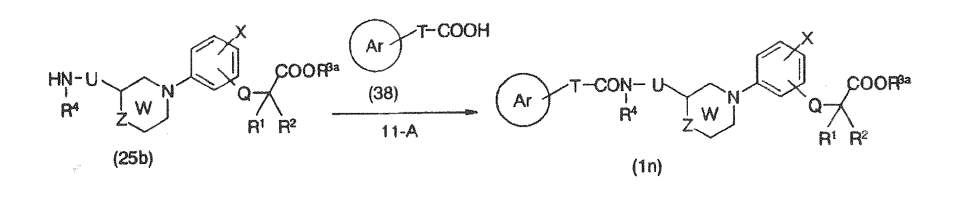

[wherein the ring Ar, Q, T, U, W, X, Z, R¹, R², R^{3a} and R⁴ are as defined above].
The conversion of the general formula (25b) and the general formula (38) into the general formula (1n) (Step 11-A) can be carried out as in Step 9-A.

Of the compounds represented by the general formula (1), those shown by the general formula (1o) may be synthesized by Production Process 12 shown below.

[Production Process 12]

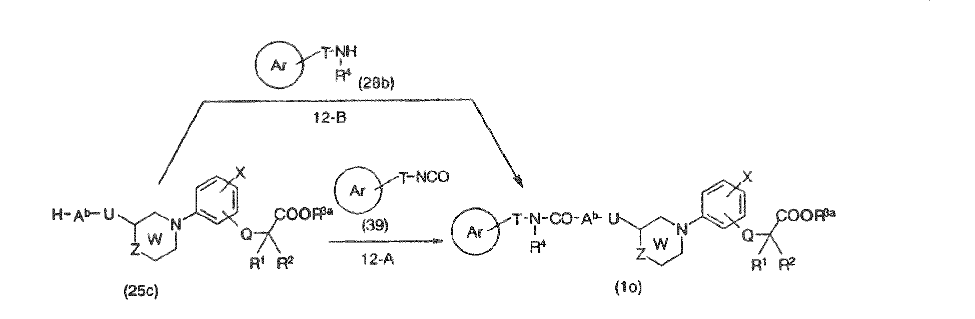

[wherein A^{b} represents an oxygen atom or -NR⁴-; and the ring Ar, Q, T, U, W, X, Z, R¹, R², R^{3a} and R⁴ are as defined above].

The conversion of the general formula (25c) and the general formula (39) into the general formula (1o) (Step 12-A) can be carried out at 0 to 100°C for 0.5 to 12 hours in a suitable solvent, such as ethyl acetate, benzene, toluene, tetrahydrofuran, diethyl ether, 1,4-dioxane, dichloromethane, N,N-dimethylformamide, N,N-dimethylacetamide and mixtures thereof, and when necessary, in the presence of a base, such as pyridine and triethylamine.

The conversion of the general formula (25c) and the general formula (28b) into the general formula (1o) (Step 12-B) can be carried out at 0 to 60°C for 0.5 to 12 hours in a suitable solvent, such as ethyl acetate, benzene, toluene, tetrahydrofuran, diethyl ether, 1,4-dioxane, dichloromethane and mixtures thereof, in the presence of carbonyl diimidazole and when necessary, in the presence of a base, such as pyridine, and triethylamine.

Of the compounds represented by the general formula (1), those shown by the general formula (1p) may be synthesized by Production Process 13 shown below.

[Production Process 13]

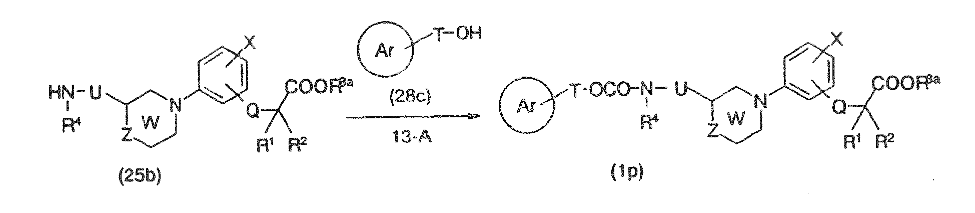

[wherein the ring Ar, Q, T, U, W, X, Z, R¹, R², R^{3a}, and R⁴ are as defined above].

The conversion of the general formula (25b) and the general formula (28c) into the general formula (1p) (Step 13-A) can be carried out as in Stop 12-B.

Of the compounds represented by the general formula (1), those shown by the general formula (1q) or (1r) may be synthesized by Production Process 14 shown below.

[Production Process 14]

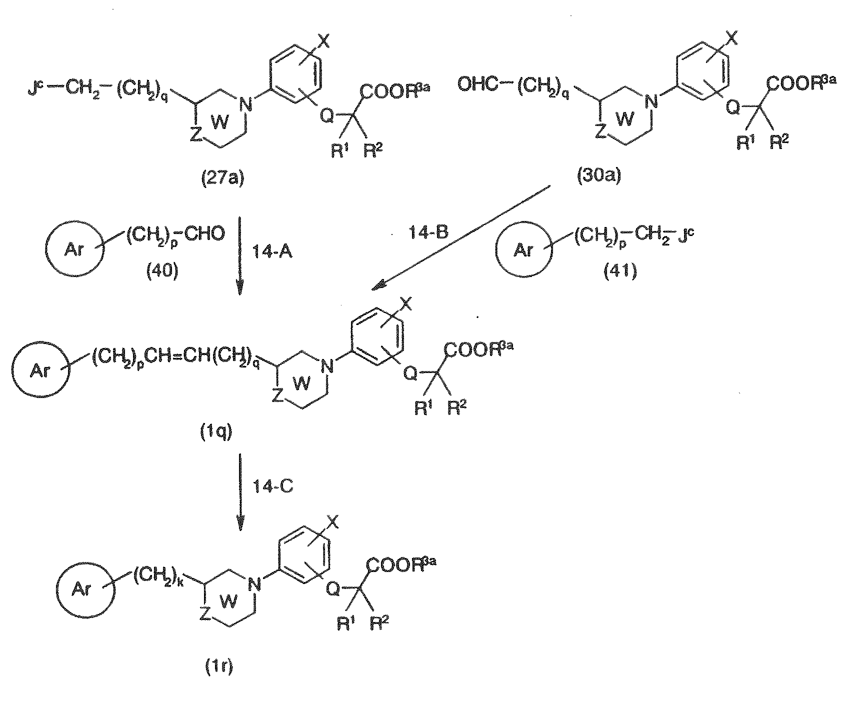

[wherein J^{c} represents a halogen atom; p and q each independently represent an integer of 0, 1 or 2 (with the proviso that p + q = 0, 1 or 2); k represents an integer of 2, 3 or 4; and the ring Ar, Q, W, X, Z, R¹, R² and R^{3a} are as defined above].

The conversion of the general formula (27a) and the general formula (40) into the general formula (1q) (Step 14-A) can be carried out as follows: The general formula (27a) is first reacted with an organic phosphorus compound, such as triethyl phosphite and triphenylphosphine, at 0 to 120°C for 1 to 12 hours, in a suitable solvent, such as toluene, tetrahydrofuran, benzene and mixtures thereof. Subsequently, the reaction product was reacted with the general formula (40) at -78 to 120°C for 1 to 12 hours in a suitable solvent, such as benzene, toluene, tetrahydrofuran, 1,4-dioxane, diethyl ether, dimethylsulfoxide and mixtures thereof, in the presence of a base, such as sodium hydride, n-butyllithium, lithium diisopropylamide, lithium bis(trimethylsilyl)amide, potassium tert-butoxide and sodium hydroxide.

The conversion of the general formula (30a) and the general formula (41) into the general formula (1q) (Step 14-B) can be carried out as in Step 14-A.

The conversion of the general formula (1q) into the general formula (1r) (Step 14-C) can be carried out as in Step 3-B.

Of the compounds represented by the general formula (1), those shown by the general formula (1s) may be synthesized by Production Process 15 shown below.

[Production Process 15]

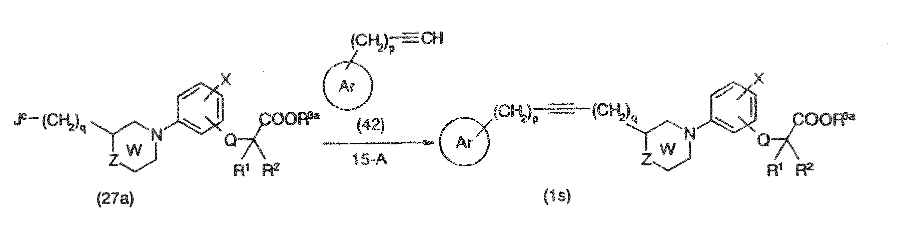

[wherein the ring Ar, Q, W, X, Z, R¹ R², R^{3a}, J^{c}, p and q are as defined above].

The conversion of the general formula (27a) and the general formula (42) into the general formula (1s) (Step 15-A) can be carried out at -78°C to room temperature for 1 to 12 hours. Specifically, the reaction is carried out in the presence of a suitable solvent, such as toluene, tetrahydrofuran, diethyl ether, dimethylsulfoxide, hexamethylphosphoric triamide and mixtures thereof, in the presence of a base, such as sodium hydride, n-butyllithium, lithium amide and potassium carbonate. When necessary, sodium iodide, copper (I) iodide or tetrabutylammonium iodide was added.

Of the compounds represented by the general formula (6) in Production Processes 1, 2 and 5, those shown by the general formula (6a) may be synthesized by Production Process 16 shown below.

[Production Process 16]

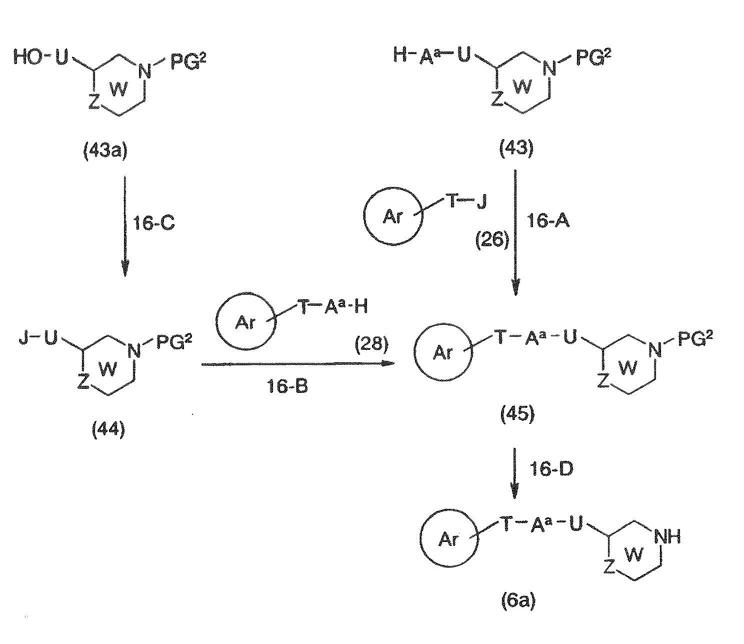

[wherein PG² represents a protective group; and the ring Ar, T, U, W, Z, A^{a} and J are as defined above].

The protective group PG² may be an acyl group, such as an acetyl group and a trifluoroacetyl group, a lower alkoxycarbonyl group, such as a tert-butoxycarbonyl group, a substituted or unsubstituted aryloxycarbonyl group, such as a benzyloxycarbornyl group, or a substituted or unsubstituted aralkyl group, such as p-methoxybenzyl group.

The conversion of the general formula (43) and the general formula (26) into the general formula (45) (Step 16-A) can be carried out as in Step 6-A.

The conversion of the general formula (44) and the general formula (28) into the general formula (45) (Step 16-B) can be carried out as in Step 6-B.

The conversion of the general formula (43a) into the general formula (44) (Step 16-C) can be carried out as in Step 6-C.

The conversion of the general formula (45) into the general formula (6a) (Step 16-D) can be carried out by a common deprotection process (for example, see, Protecting Groups in Organic Synthesis (John Wily and Sons(1999)).

Among known deprotection processes are those involving an acid, a base, ultraviolet rays, hydrazine, tetrabutylammonium fluoride and trimethylsilyl iodide, and reduction.

Of the compounds represented by the general formula (45) in Production Processes 16, those shown by the general formula (45a) may be synthesized by Production Process 17 shown below.

[Production Process 17]

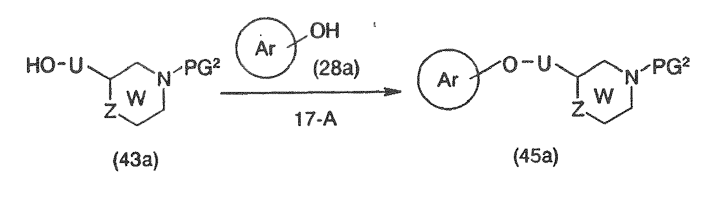

[wherein the ring Ar, U, W, Z and PG² are as defined above].

The conversion of the general formula (43a) and the general formula (28a) into the general formula (45a) (Step 17-A) can be carried out as in Step 7-A.

Of the compounds represented by the general formula (45) in Production Processes 16, those shown by the general formula (45b) may be synthesized by Production Process 18 shown below.

[Production Process 18]

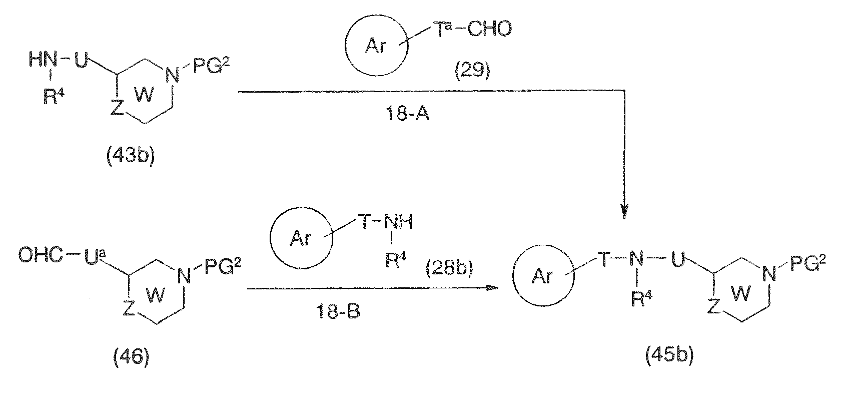

[wherein the ring Ar, T, T^{a}, U, U^{a}, W, Z, R⁴ and PG² are as defined above].

The conversion of the general formula (43b) and the general formula (29) into the general formula (45b) (Step 18-A) canbe carried out as in Step 8-A.

The conversion of the general formula (46) and the general formula (28b) into the general formula (45b) (Step 18-B) can be carried out as in Step 8-B.

Of the compounds represented by the general formula (6) in Production Processes 1, 2 and 5, those shown by the general formula (6b) may be synthesized by Production Process 19 shown below.

[Production Process 19]

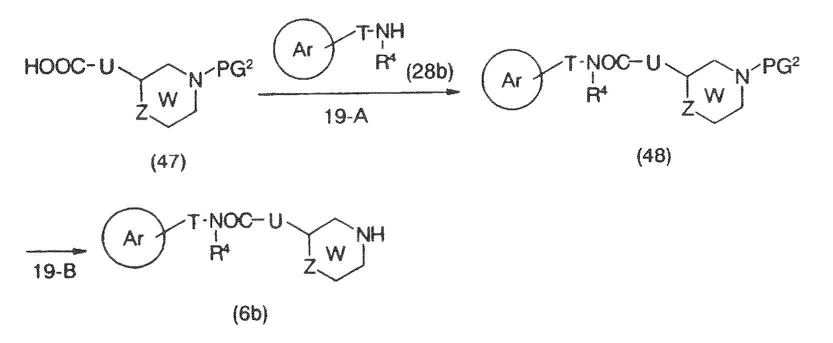

[wherein the ring Ar, T, U, W, Z, R⁴ and PG² are as defined above].

The conversion of the general formula (47) and the general formula (28b) into the general formula (48) (Step 19-A) can be carried out as in Step 9-A.

The conversion of the general formula (48) into the general formula (6b) (Step 19-B) can be carried out as in Step 16-D.

Of the compounds represented by the general formula (6) in Production Processes 1, 2 and 5, those shown by the general formula (6c) may be synthesized by Production Process 20 shown below.

[Production Process 20]

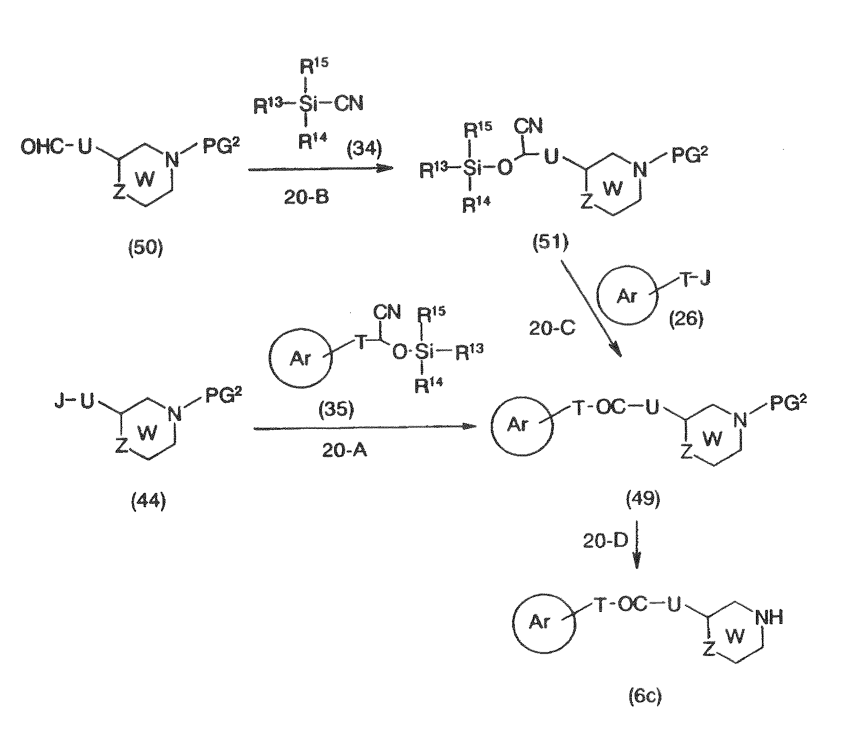

[wherein the ring Ar, T, U, W, Z, J, R¹³, R¹⁴, R¹⁵ and PG² are as defined above].

The conversion of the general formula (44) and the general formula (35) into the general formula (49) (Step 20-A) can be carried out as in Step 10-B.

The conversion of the general formula (50) and the general formula (34) into the general formula (51) (Step 20-B) can be carried out as in Step 10-C.

The conversion of the general formula (51) and the general formula (26) into the general formula (49) (Step 20-C) can be carried out as in Step 10-D.

The conversion of the general formula (49) into the general formula (6C) (Step 20-D) can be carried out as in Step 16-D.

Of the compounds represented by the general formula (6) in Production Processes 1, 2 and 5, those shown by the general formula (6d) may be synthesized by Production Process 21 shown below.

[Production Process 21]

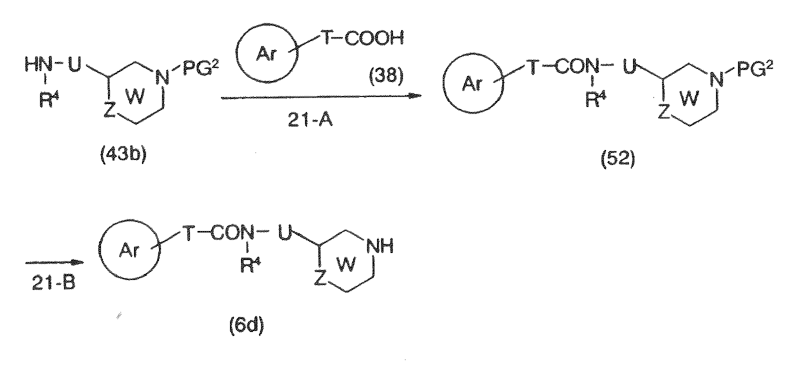

[wherein the ring Ar, T, U, W, Z, R⁴ and PG² are as defined above].

The conversion of the general formula (43b) and the general formula (38) into the general formula (52) (Step 21-A) can be carried out as in Step 11-A.

The conversion of the general formula (52) into the general formula (6d) (Step 21-B) can be carried out as in Step 16-D.

Of the compounds represented by the general formula (6) in Production Processes 1, 2 and 5, those shown by the general formula (6e) may be synthesized by Production Process 22 shown below.

[Production Process 22]

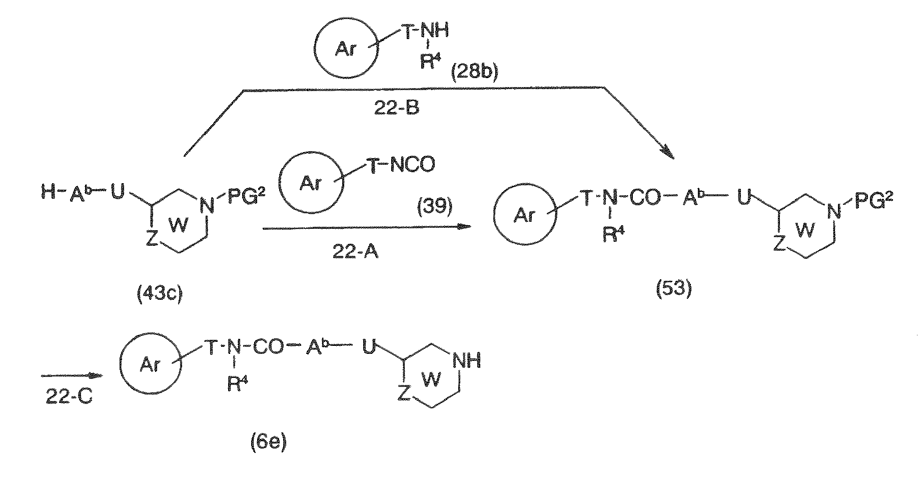

[wherein the ring Ar, A^{b}, T, U, W, Z, R⁴ and PG² are as defined above].

The conversion of the general formula (43c) and the general formula (39) into the general formula (53) (Step22-A) can be carried out as in Step 12-A.

The conversion of the general formula (43c) and the general formula (28b) into the general formula (53) (Step 22-B) canbe carried out as in Step 12-B.

The conversion of the general formula (53) into the general formula (6e) (Step 22-C) can be carried out as in Step 16-D.

Of the compounds represented by the general formula (6) in Production Processes 1, 2 and 5, those shown by the general formula (6f) may be synthesized by Production Process 23 shown below.

[Production Process 23]

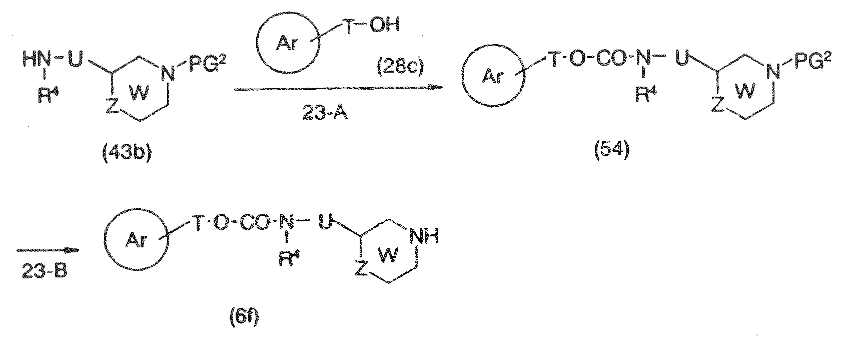

[wherein the ring Ar, T, U, W, Z, R⁴ and PG² are as defined above].

The conversion of the general formula (43b) and the general formula (28c) into the general formula (54) (Step23-A) canbe carried out as in Step 13-A.

The conversion of the general formula (54) into the general formula (6f) (Step 23-B) can be carried out as in Step 16-D.

Of the compounds represented by the general formula (6) in Production Processes 1, 2 and 5, those shown by the general formula (6g) or (6h) may be synthesized by Production Process 24 shown below.

[Production Process 24]

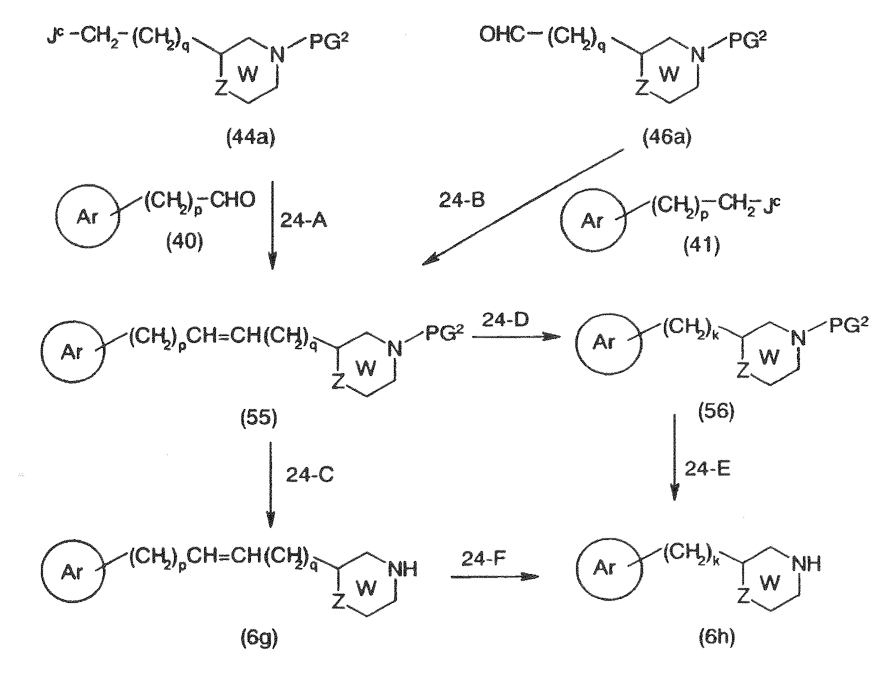

[wherein the ring Ar, W, X, Z, J^{c}, PG², p, q and k are as defined above].

The conversion of the general formula (44a) and the general formula (40) into the general formula (55) (Step 24-A) can be carried out as in Step 14-A.

The conversion of the general formula (46a) and the general formula (41) into the general formula (55) (Step 24-B) can be carried out as in Step 14-B.

The conversion of the general formula (55) into the general formula (6g) (Step 24-C) can be carried out as in Step 16-D.

The conversion of the general formula (55) into the general formula (56) (Step 24-D) can be carried out as in Step 14-C.

The conversion of the general formula (56) into the general formula (6h) (Step 24-E) can be carried out as in Step 16-D.

The conversion of the general formula (6g) into the general formula (6h) (Step 24-F) can be carried out as in Step 14-C.

Of the compounds represented by the general formula (6) in Production Processes 1, 2 and 5, those shown by the general formula (6i) may be synthesized by Production Process 25 shown below.

[Production Process 25]

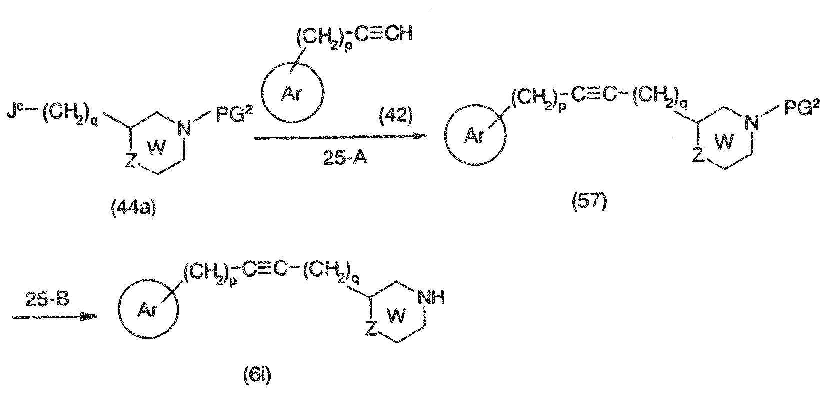

[wherein the ring Ar, W, X, Z, R^{a}, J^{c}, PG², p and q are as defined above].

The conversion of the general formula (44a) and the general formula (42) into the general formula (57) (Step 25-A) can be carried out as in Step 15-A.

The conversion of the general formula (57) into the general formula (6i) (Step 25-B) can be carried out as in Step 16-D.

The compounds represented by the general formula (60) or (61) in Production Processes 6 through 15 may be synthesized by Production Process 26 shown below.

[Production Process 26]

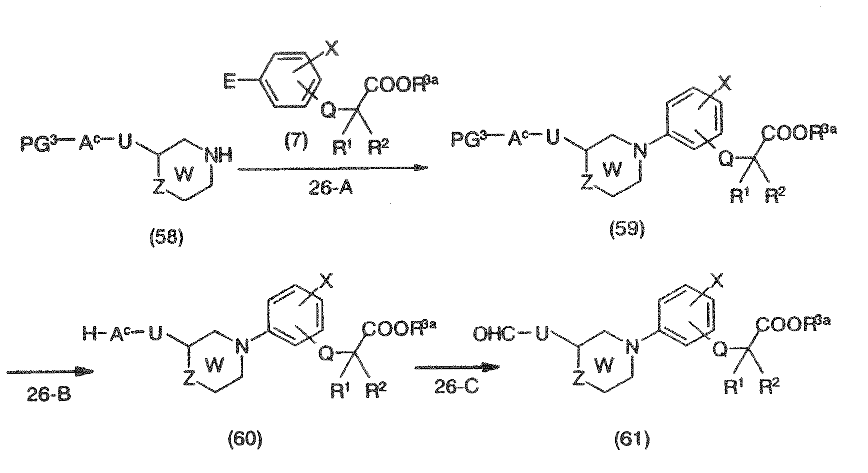

[wherein PG³ represents a protective group or a hydrogen atom; A^{c} represents an oxygen atom, a sulfur atom, -NR⁴⁻, -OOC- or -OCH₂-; and Q, U, W, X, Z, R¹, R², R^{3a}, R⁴ and E are as defined above].

The protective group PG³ may be an acyl group, such as an acetyl group and a trifluoroacetyl group, a lower alkoxycarbonyl group, such as a tert-butoxycarbonyl group, a substituted or unsubstituted aralkyloxycarbonyl group, such as a benzyloxycarbonyl group, a substituted or unsubstituted aralkyl group, such as a benzyl group, a p-methoxybenzyl group or a triphenylmethyl group, a silyl group, such as a trimethylsilyl group and a tert-butyldimethylsilyl group, or a phthaloyl group.

The conversion of the general formula (58) and the general formula (7) into the general formula (59) (Step 26-A) can be carried out as in Step 1-A.

The conversion of the compound of the general formula (59), in which PG³ represents a protective group, into the general formula (60) (Step 26-B) can be carried out by a common deprotection process (for example, see, Protecting Groups in Organic Synthesis (John Wily and Sons (1999)).

Among known deprotection processes are those involving an acid, a base, ultraviolet rays, hydrazine, tetrabutylammonium fluoride and trimethylsilyl iodide, and reduction.

The conversion of the compound of the general formula (60) with A^{C} representing -OCH₂- into the general formula (61) (Step 26-C) can be carried out at -78 to 50°C for 15 min to 6 hours in a suitable solvent, such as dichloromethane, tetrahydrofuran, diethyl ether and mixtures thereof, in the presence of a base, such as triethylamine, pyridine and diisopropylethylamine, or dimethylsulfoxide (and when necessary, the presence of an acid such as trifluoroacetic acid), and in the presence of an electrophile, such as oxalyl chloride, dicyclohexylcarbodiimide, sulfur trioxide and acetic anhydride.

Alternatively, the compound of the general formula (60) with A^{C} representing -OCH₂- may be converted into the general formula (61) by reacting it with Dess-Martin periodinane(1,1,1-tris(acetoxy)-1,1-dihydro-1,2-benziodoxol-3-(1H) -one), or with tetrapropylammonium perruthenate in the presence of 4-methylmorpholine N-oxide, in a suitable solvent, such as chloroform, dichloromethane and acetonitrile at 0 to 50°C for 10 min to 36 hours.

The compound of the general formula (60) with A^{c} representing -OCH₂- may also be converted into the general formula (61) by reacting it with 2,2,6,6-tetramethylpiperidine N-oxide at 0 to 50 °C for 0.5 to 6 hours in a suitable solvent, such as chloroform, dichloromethane, benzene and toluene, in the presence of a co-oxidizing agent, such as sodium hypochlorite, a reaction aid, such as potassium bromide, and a buffer, such as an aqueous sodium bicarbonate solution. The compounds of the general formula (60) with A^{c} representing an oxygen atom can be converted into compounds with A^{c} representing NH by a known process such as Gabriel reaction (see, for example, "Experimental Chemistry" Maruzen).

Of the compounds represented by the general formula (59) in Production Process 26, those shown by the general formula (59a) or (59b) may be synthesized by Production Process 27 shown below.

[Production Process 27]

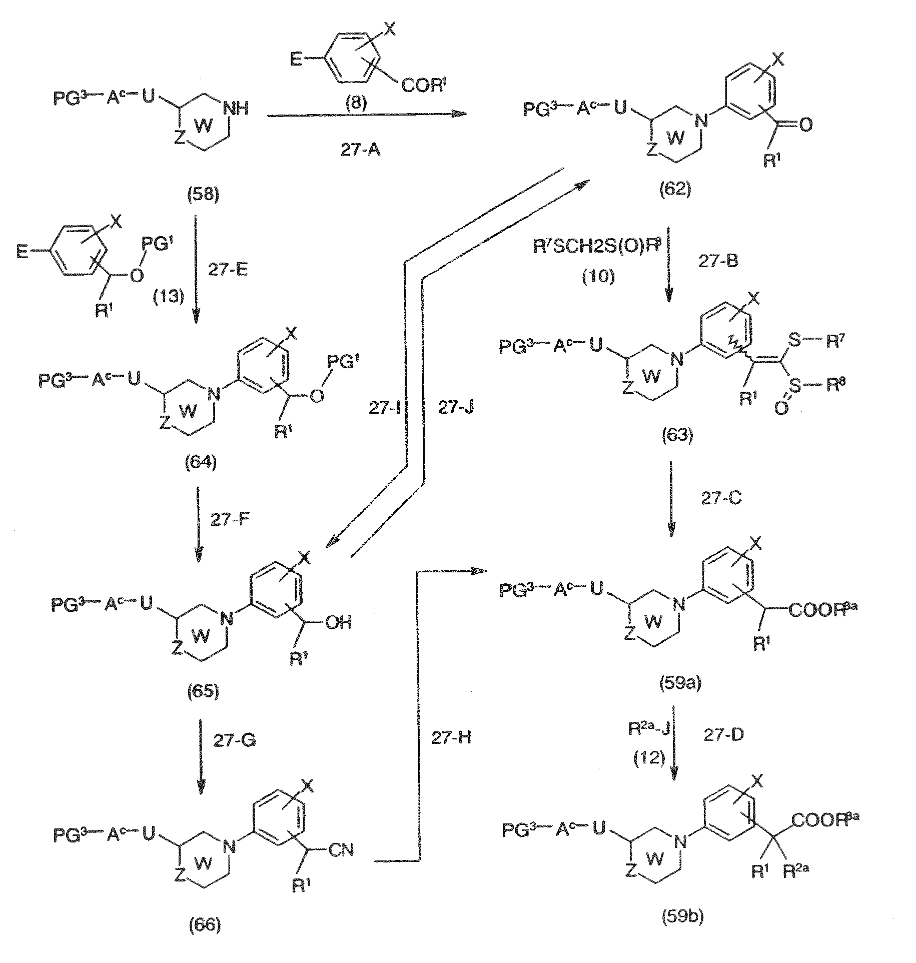

[wherein A^{c}, U, W, X, Z, R¹, R^{2a}, R^{3a}, R⁷, R⁸, E, J, PG³ and the wavy line are as defined above].

The conversion of the general formula (58) and the general formula (8) into the general formula (62) (Step 27-A) can be carried out as in Step 2-A.

The conversion of the general formula (62) and the general formula (10) into the general formula (63) (Step 27-B) can be carried out as in Step 2-B.

The conversion of the general formula (63) into the general formula (59a) (Step 27-C) can be carried out as in Step 2-C.

The conversion of the general formula (59a) and the general formula (12) into the general formula (59b) (Step27-D) can be carried out as in Step 2-D.

The conversion of the general formula (58) and the general formula (13) into the general formula (64) (Step 27-E) can be carried out as in Step 2-E.

The conversion of the general formula (64) into the general formula (65) (Step 27-F) can be carried out as in Step 2-F.

The conversion of the general formula (65) into the general formula (66) (Step 27-G) can be carried out as in Step 2-G.

The conversion of the general formula (66) into the general formula (59a) (Step 27-H) can be carried out as follows: The general formula (66) is first hydrolyzed at 0 to 200°C for 1 to 48 hours in the absence or in the presence of a suitable solvent, such as water, acetic acid, methanol, ethanol, ethylene glycol, tetrahydrofuran, 1,4-dioxane and mixtures thereof, and in the presence of an acid such as hydrochloric acid, sulfuric acid and nitric acid or in the presence of a base, such as lithium hydroxide, sodium hydroxide, potassium hydroxide, sodium carbonate and potassium carbonate. Subsequently, the reaction product is esterified with an acid, such as sulfuric acid and hydrochloric acid, at room temperature to 100°C 0.5 to 12 hours in an alcohol, such as methanol and ethanol. Alternatively, the reaction product may be esterified with a lower alkyl halide, such as methyl iodide and ethyl iodide, at 0 to 100°C for 0.5 to 12 hours in a suitable solvent, such as N,N-dimethylformamide, N,N-dimethylacetamide and acetone, and in the presence of a base, such as lithium hydroxide, sodium hydroxide, potassium hydroxide, sodium carbonate, potassium carbonate and sodium hydride.

The conversion of the general formula (65) into the general formula (62) (Step 27-J) can be carried out as in Step 2-J.

The conversion of the general formula (62) into the general formula (65) (Step 27-I) can be carried out as In Step 2-I.

Of the compounds represented by the general formula (59) in Production Process 26, those shown by the general formula (59c) or (59d) may be synthesized by Production Process 28 shown below.

[Production Process 28]

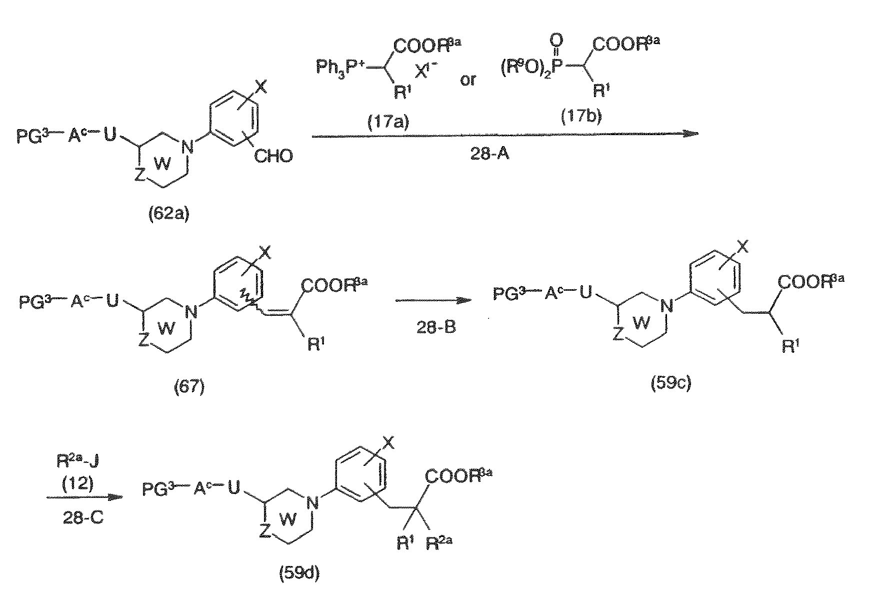

[wherein A^{c}, U, W, X, X¹, Z, R¹, R^{2a}, R^{3a}, R⁹, PG³ and the wavy line are as defined above].

The conversion of the general formula (62a) into the general formula (67) (Step 28-A) can be carried out as in Step 3-A.

The conversion of the general formula (67) into the general formula (59c) (Step 23-B) can be carried out as in Step 3-B.

The conversion of the general formula (59c) into the general formula (59d) (Step 28-C) can be carried out as in Step 3-C.

Of the compounds represented by the general formula (59) in Production Process 26, those shown by the general formula (59e) may be synthesized by Production Process 29 shown below.

[Production Process 29]

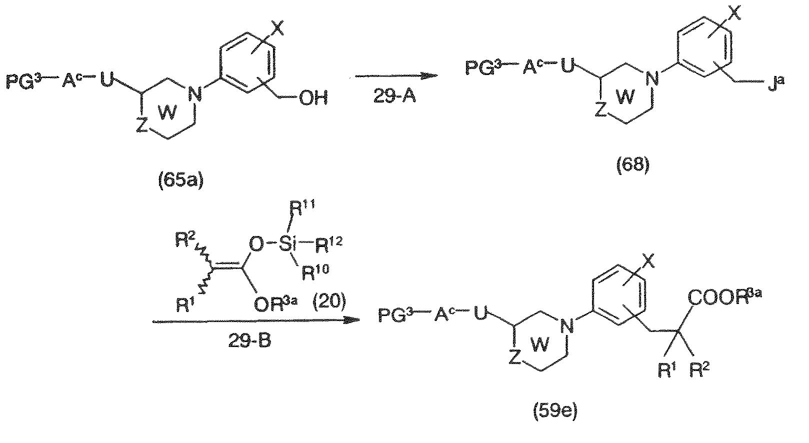

[wherein A^{c}, J^{a}, U, W, X, Z, R¹, R², R^{3a}, R¹⁰, R¹¹, R¹², PG³ and the wavy line are as defined above].

The conversion of the general formula (65a) into the general formula (68) (Step 29-A) can be carried out as in Step 4-A.

The conversion of the general formula (68) and the general formula (20) into the general formula (50e) (Step 29-B) can be carried out as in Step 4-B.

Of the compounds represented by the general formula (59) in Production Process 26, those shown by the general formula (59f) may be synthesized by Production Process 30 shown below.

[Production Process 30]

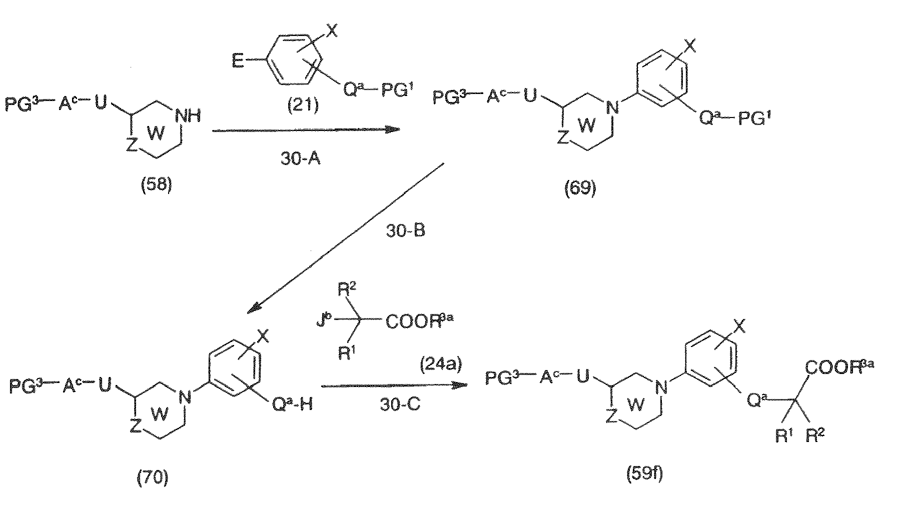

[wherein A^{c}, E, J^{b}, Q^{a}, U, W, X, Z, R¹, R², R^{3a}, PG¹ and PG³ are as defined above].

The conversion of the general formula (58) and the general formula (21) into the general formula (69) (Step 30-A) can be carried out as in Step 5-A.

The conversion of the general formula (69) into the general formula (70) (Step 30-B) can be carried out as in Step 5-B.

The conversion of the general formula (70) and the general formula (24a) into the general formula (59f) (Step 30-C) can be carried out as in Step 5-C.

Of the compounds represented by the general formula (1), those shown by the general formula (It) may be synthesized by Production Process 31 shown below.

[Production Process 31]

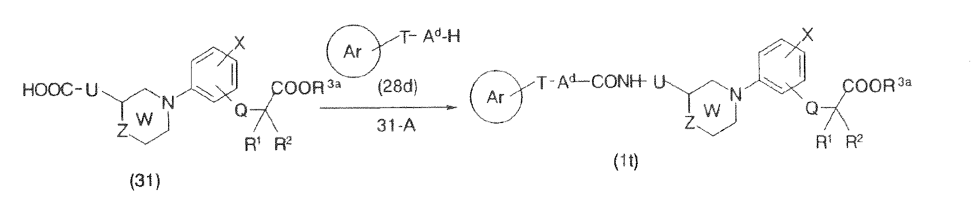

[wherein A^{d} represents an oxygen atom or -NR⁴-; and the ring Ar, Q, T, U, W, X, Z, R¹, R¹, R², R^{3a} and R⁴ are as defined above].

The conversion of the general formula (31) and the general formula (28d) into the general formula (1t) (Step31-A) can be carried out as follows: The general formula (31) is first reacted with a phophonic azide, such as diphenylphosphonic azide, at room temperature to 150°C for 3 to 12 hours in a suitable solvent, such as benzene, toluene, tetrahydrofuran, 1,4-dioxane and mixtures thereof, and in the presence of a base, such as pyridine, triethylamine, N-methylmorpholine and 4-(dimethylamino)pyridine. This converts the carboxyl group into an isocyanate group. Subsequently, the reaction product is reacted with the general formula (28d) at 0 to 150°C for 0.5 to 12 hours in a suitable solvent, such as benzene, toluene, tetrahydrofuran, 1,4-dioxane and mixtures thereof, and when necessary, in the presence of a base, such as pyridine, triethylamine, N-methylmorpholine and 4-(dimethylamino)pyridine.

Alternatively, the general formula (31) may be reacted with thionyl chloride, thionyl bromide, acetic anhydride or ethyl chlorocarbonate at -15 to 50°C for 5min to 3 hours to form an acid chloride, an acid bromide or an acid anhydride. This converts the carboxyl group into a reactive derivatizing group. This reaction can be carried out in the absence or in the presence of a suitable solvent, such as benzene, toluene, tetrahydrofuran, dichloromethane and mixtures thereof, and when necessary, in the presence of a base, such as pyridine and triethylamine. Subsequently, the reaction product is reacted with an azide, such as sodium azide, or a silyl azide, such as trimthylsilyl azide, at 0 to 50 °C for 0.5 to 6 hours in a suitable solvent, such as water, acetone, 1,4-dioxane, tetrahydrofuran, benzene, toluene, dichloromethane and mixtures thereof, and when necessary, in the presence of a reaction aid, such as 18-crown-6. The resulting product is reacted at room temperature to 150°C for 3 to 12 hours in a suitable solvent, such as benzene, toluene, tetrahydrofuran, 1,4-dioxane and mixtures thereof, to introduce an isocyanate group. The reaction product is then reacted with the general formula (28d) at 0 to 150°C for 0.5 to 12 hours in a suitable solvent, such as benzene, toluene, tetrahydrofuran, 1,4-dioxane and mixtures thereof, and when necessary, in the presence of a base, such as pyridine, triethylamine, N-methylmorpholine and 4-(dimethylamino)pyridine. Of the compounds represented by the general formula (1), those shown by the general formula (1v) may be synthesized by Production Process 32 shown below.

[Production Process 32]

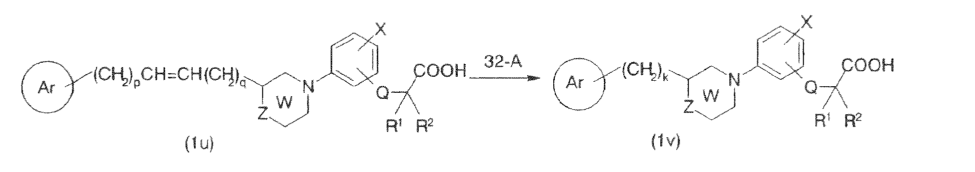

[wherein p, q, k, the ring Ar, Q, W, X, Z, R¹ and R² are as defined above].

The conversion of the general formula (lu) into the general formula (1v) (Step 32-A) can be carried out as in Step 14-C.

Of the compounds represented by the general formula (6) in Production Processes 1, 2 and 5, those shown by the general formula (6j) may be synthesized by Production Process 33 shown below.

[Production Process 33]

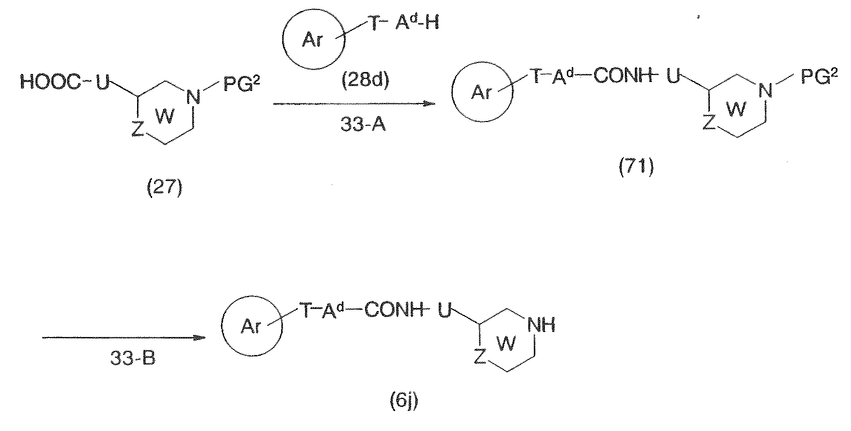

[wherein the ring Ar, T, U, W, Z, A^{d} and PG² are as defined above].

The conversion of the general formula (27) and the general formula (28d) into the general formula (71) (Step 33-A) can be carried out as in Step 31-A. The conversion of the general formula (71) into the general formula (6j) (Step 33-B) can be carried out as in Step 16-D.

The compounds having ring Ar and represented by the general formulas (26), (28), (29), (32), (33), and (38) through (42) in Production Processes 1 through 33 can be synthesized by known processes (See, for example, Heterocyclic Chemistry (Chapman and Hall (1995)); Synthetic Communication, 20(16), 2537-2547(1990); Heterocycles, 47(2), 857-864(1998); Journal of Organic Chemistry, 61(19), 6496-6497(1996); Journal of American Chemical Society, 71, 2328(1949); and Synthesis Communication, 19(16),2921-2924(1989)).

The substituents X and R⁴ in the compounds shown in Production Processes 1 through 33 can be converted by known processes (See, for example, "Experimental Chemistry" Maruzen).

For example, the compounds with substituent X representing a nitro group can be converted into compounds with X representing an amino group by a metal-based reduction process involving iron powder or zinc powder, or by a reduction process involving palladium/active carbon. The compounds with the substituent R⁴ representing a hydrogen atom can be converted into compounds with R⁴ representing an alkyl group by an alkylation process involving a halogenated alkyl, such as methyl iodide, or by a reductive amination process involving an aldehyde, such as acetaldehyde, and a reductant, such as sodium borohydride.

Optical isomers of the compounds represented by the general formula (1) can be synthesized by Production Processes 1 through 33 using optically active starting materials.

Alternatively, these optical isomers may be obtained by subjecting a racemic mixture of the general formula (1) to fractional crystallization using an optically active acid or base, or by reacting the racemic mixture with an optically active alcohol or an optically active oxazolidinone derivative to form a diastereomeric ester or oxazolidinone derivative, and subjecting these derivatives to fractional crystallization or chromatography, followed by hydrolysis.

The optical isomers may also be obtained by separating a racemic mixture of the general formula (1) by chromatography using a chiral support.

### Examples

The present invention will now be described with reference to Examples, which are not intended to limit the scope of the invention in any way.

<Example 1>
Methyl 2-[3-[[2-(4-chlorophenyl)-4-methylthiazol-5-yl]carbonylaminome thyl]piperidin-1-yl]phenylacetate

Step 1a) N-[(1-tert-butoxycarbonyl)piperidin-3-yl methyl]-2-(4-chlorophenyl)-4-methylthiazole-5-carboxamide

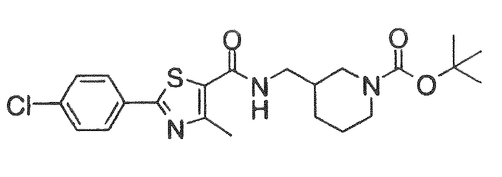

2-(4-chlorophenyl)-4-methylthiazole-5-carboxylic acid (507 mg, 2.00 mmol), 1-(tert-butoxycarbonyl)piperidin-3-yl methylamine (429 mg, 2.00 mmol) and 1-hydroxyberizotriazole monohydrate (368 mg, 2.40 mmol) were dissolved in N,N-dimethylformamide (10 mL). The solution was cooled in an ice bath and 3-(3-dimethylaminopropyl)-1-ethylcarbodiimidehydrochloride (460 mg, 2.40 mmol) and N-methylmorpholine (0.528 mL, 4.80 mmol) were added. The mixture was stirred at room temperature for 4 hours. Subsequently, a 5% aqueous citric acid was added and the mixture was extracted with ethyl acetate. The extract was washed sequentially with a saturated aqueous sodium bicarbonate solution and a saturated brine, dried over magnesium sulfate and evaporated. Purification of the resulting residue by silica gel column chromatography (hexane:ethyl acetate = 10:1 --> 2:1) afforded the title compound as a colorless amorphous material (851 mg, 95 %). ¹H NMR (400 MHz, CDCl₃) δ 1.42-1.51 (11H, m), 1.60-1.72 (1H, m), 1.78-1.96 (2H, m), 2.75 (3H, s), 3.05-3.86 (6H, m), 6.59 (1H, brs), 7.42 (2H, d, J = 8.6 Hz), 7.87 (2H, d, J = 8.6 Hz).

Step 1b) N-(piperidin-3-yl methyl)-2-(4-chlorophenyl)-4-methylthiazole-5-carboxamide

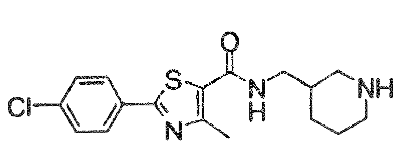

N-[(1-tert-butoxycarbonyl)piperidin-3-yl methyl]-2-(4-chlorophenyl)-4-methylthiazole-5-carboxamide (2.78 g, 6.18 mmol) was dissolved in methanol (30 mL). While the solution was stirred in an ice bath, a 5.7 mol/L hydrogen chloride-methanol solution (50 mL) was added. The mixture was stirred in an ice bath for 10 min and at room temperature for 3 hours. Subsequently, the reaction mixture was concentrated and water was added to the resulting residue. A mol/L aqueous sodium hydroxide solution was added to neutralize the mixture. Sodium chloride was then added to saturation and the mixture was extracted with ethyl acetate. The extract was washed with a saturated brine and dried over magnesium sulfate. Evaporation of the solvent afforded the title compound as a colorless powder (2.09 g, 97%).

¹H NMR (400 MHz, DMSO-d₆) δ 1.00-1.10 (1H, m), 1.26-1.37 (1H, m), 1.53-1.74 (3H, m), 2.18 (1H, dd, J = 9.8, 11.6 Hz), 2.40 (1H, dt, J= 2.4, 11.6 Hz), 2.59 (3H, s), 2.77-2.83 (1H, m), 2.91 (1H, dd, J = 2.4, 11.6 Hz), 3.68 (2H, t, J= 6.1 Hz), 7.42 (2H, d, J= 8.6 Hz), 7.87 (2H, d, J = 8.6 Hz), 8.30 (1H, t, J = 5.5 Hz).

Step 1c) N-[1-(2-formylphenyl)piperidin-3-yl methyl]-2-(4-chlorophenyl)-4-methylthiazole-5-carboxamide

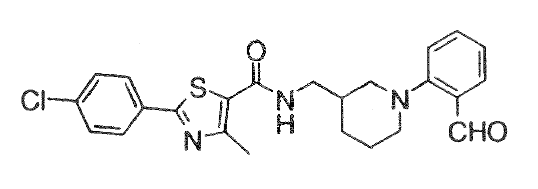

Potassium carbonate (138 mg, 1.00 mmol) and tetrabutylammonium iodide (18.5 mg, 0.0500 mmol) were added to a solution of N-(piperidin-3-yl methyl)-2-(4-chlorophenyl)-4-methylthiazole-5-carboxamide (175 mg, 0.500 mmol) and 2-fluorobenzaldehyde (0.109 mL, 1.00 mmol) in N,N-dimethylformamide (5mL). The mixture was stirred at 140°C for 4 hours. Subsequently, a saturated aqueous sodium bicarbonate solution was added to adjust the pH to less than 8. The mixture was then extracted with ethyl acetate. The extract was washed with a saturated brine, dried over magnesium sulfate, and evaporated. Purification of the resulting residue by silica gel column chromatography (hexane:ethyl acetate = 4: 1 --> 1:1) afforded the title compound as a yellow powder (139 mg, 61%).

¹H NMR (400 MHz, CDCl₃) δ 1.21-1.33 (1H, m), 1.76-1.95 (3H, m), 2.11-2.21 (1H, m), 2.68-2.73 (1H, m), 2.73 (3H, s), 2.89 (1H, td, J = 11.6, 3.1 Hz), 3.22-3.25 (1H, m), 3.28-3.32 (1H, m), 3.43-3.47 (2H, m), 6.00 (1H, t, J = 5. 5 Hz), 7.09-7.12 (2H, m), 7.41 (2H, d, J = 8.6 Hz), 7.51 (1H, td, J = 7.9, 1.8 Hz), 7.78 (1H, dd, J = 7.9, 1.8 Hz), 7.86 (2H, d, J = 8.6 Hz), 10.26 (1H, s).
FAB⁺(m/z) : 454 (M+H).

Step 1d) (E)-N-[1-[2-(2-methylthio-2-methylsulfinylethenyl)phenyl]piper idin-3-yl methyl]-2-(4-chlorophenyl)-4-methylthiazole-5-carboxamide and (Z)-N-[1-[2-(2-methylthio-2-methylsulfinylethenyl)phenyl]piper idin-3-yl methyl]-2-(4-chlorophenyl)-4-methylthiazole-5-carboxamide

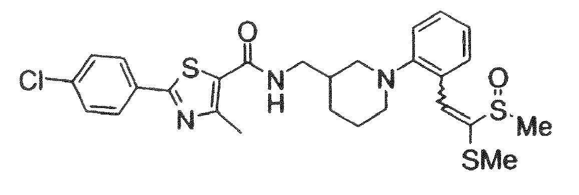

Methyl methylsulfinylmethylsulfide (0.0456 mL, 0.367 mmol) and a 40% benzyltrimethylammonium hydroxide/methanol solution (0.167 mL, 0.367 mmol) were added to a solution of N-[1-(2-formylphenyl)piperidin-3-yl methyl]-2-(4-chlorophenyl)-4-methylthlazole-5-carboxamide (139 mg, 0.306 mmol) in tetrahydrofuran (3 mL). The mixture was stirred for 3 hours under reflux. Subsequently, water was added and the mixture was extracted with ethyl acetate. The extract was washed with a saturated brine, dried over magnesium sulfate, and evaporated. Purification of the resulting residue by silica gel column chromatography (CHROMATOREX NH-DM2035, Fuji Silysia Chemical Ltd., hexane:ethyl acotate = 2:1) afforded the title compounds as a pale yellow oil (Z-form, 56.0mg, 33%) and as a pale yellow powder (E-form, 59.3 mg, 35%), respectively.

(Z)-N-[1-[2-(2-methylthio-2-methylsulfinylethenyl)phenyl]piper idin-3-yl methyl]-2-(4-chlorophenyl)-4-methylthiazole-5-carboxamide ¹HNMR (400MHz, CDCl₃) δ 1.14-1.22 (1H, m), 1.80-1.90 (3H, m), 2.00 (3H s), 2.30-2.36 (2H, m), 2.70 (3H, s), 2.73 (3H, s), 2.89 (1H, td, J = 11.6, 3.1 Hz), 3.06-3.09 (1H, m), 3.24-3.26 (2H, m), 3.57-3.65 (1H, m), 7.00-7.08 (3H, m), 7.33 (1H, td, J = 7.9, 1.2 Hz), 7.43 (2H, d, J = 8.6 Hz), 7.88 (2H, d, J = 8.6 Hz), 7.95(1H, s), 8.09(1H, dd, J = 7.9, 1.2 Hz).

(E)-N-[1-[2-(2-methylthio-2-methylsulfinylethenyl)phenyl]piper indin-3-yl methyl]-2-(4-chlorophenyl)-4-methylthiazole-5-carboxamide ¹H NMR (400 MHz, CDCl₃ δ 1.38-1.48 (1H, m), 1.77-1.84 (3H, m), 2.19-2.26 (1H, m), 2.28(3H, s), 2.70-2.74(7H, m), 2.78-2.84 (1H, m), 2.97-3.05 (2H, m), 3.39 (1H, dt, J = 13.4, 4.9 Hz), 3.55-3.63 (1H, m), 6.88 (1H, brs), 7.09-7.13 (2H, m), 7.37(1H, td, J = 7.9, 1.2 Hz), 7.42 (2H, d, J = 8.6 Hz), 7.86 (2H, d, J = 8.6 Hz), 8.03 (1H, s), 8.13 (1H, dd, J = 7.9, 1.2 Hz).

Step 1e) Methyl 2-[3-[[2-(4-chlorophenyl)-4-methylthiazol-5-yl]carbonylaminome thyl]piperidin-1-yl]phenylacetate

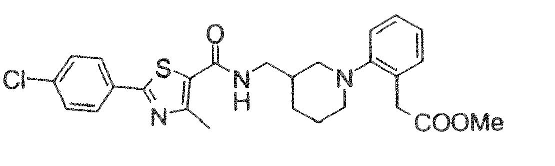

A solution of (Z)-N-[1-[2-(2-methylthio-2-methylsulfinylethenyl)phenyl]piper idin-3-yl methyl]-2-(4-chlorophenyl)-4-methylthiazole-5-carboxamide (56.0 mg, 0.100 mmol) in a 10% hydrochloric acid/methanol solution (2 mL) was stirred at room temperature for 6 hours. The solvent was evaporated and a saturated aqueous sodium bicarbonate solution was added to the resulting residue to adjust the pH to less than 8. The mixture was extracted with ethyl acetate. The extract was then washed with a saturated brine, dried over magnesium sulfate, and evaporated. Purification of the resulting residue by silica gel column chromatography (hexane:ethyl acetate = 10:1 --> 2:1) afforded the title compound as a colorless oil (36.7 mg, 74%).

¹H NMR (400 MHz, CDCl₃) δ 1.24-1.35 (1H, m), 1.65-1.72 (1H, m), 1.78-1.88 (2H, m), 2.05-2.11 (1H, m), 2.51-2.56 (1H, m), 2.69-2.75 (4H, m), 2.84-2.90 (1H, m), 3.05 (1H, dd, J = 11.6, 2.4 Hz), 3.31-3.38 (1H, m), 3.46-3.52 (1H, m), 3.65 (3H,s), 3. 68 (1H, d, J = 15.3 Hz), 3.74 (1H, d, J = 15.3 Hz), 6.20 (1H, brs), 7.09 (1H, t, J = 7.3 Hz), 7.17 (1H, d, J = 7.3 Hz), 7.22-7.30(2H, m), 7.40 (2H, d, J =8.6 Hz), 7.85 (2H, d, J = 8.6 Hz).
FAB⁺ (m/z) : 498 (M+H).

<Example 2>
Methyl 3-[3-[[2-(4-chlorophenyl)-4-methylthiazol-5-yl]carbonylaminome thyl]piperidin-1-yl]phenylacetate

Step2a) N-[1-(3-formylphenyl)piperidin-3-yl methyl]-2-(4-chlorophenyl)-4-methylthiazole-5-carboxamide

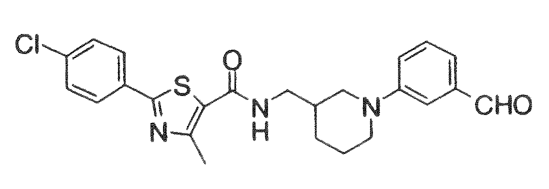

Triethylamine (0.32 mL, 2.30 mmol), 3-formylphenylboricacid (348 mg, 2.32 mmol), copper acetate (210 mg, 1.16 mmol) and molecular sieve powder (45 mg) were added to a solution of N-(piperidin-3-yl methyl)-2-(4-chlorophenyl)-4-methylthiazoie-5-carboxamide (390 mg, 1.16 mmol) in an anhydrous dichloromethane solution (15 mL). The mixture was stirred at room temperature for 6 hours. Subsequently, the mixture was filtered through Celite. The filtrate was diluted with ethyl acetate and was washed with a saturated aqueous sodium bicarbonate solution, water and a saturated brine. The organic layer was dried over anhydrous sodium sulfate and the solvent was evaporated. Purification of the resulting residue by silica gel chromatography (hexane:ethyl acetate = 3:1) afforded the title compound as a yellow powder (202 mg, 38%).

¹H-NMR (CDCl₃-d, 400 MHz) δ 1.24-1.35 (1H, m), 1.68-1.79 (1H, m), 1.83-1.95 (2H,m), 2.00-2.09 (1H, m), 2.70 (1H, dd, J=12.2, 9.8 Hz), 2.75 3H, s), 2.85-2.91 (1H, m), 3.40-3.51 (2H, m), 3.59-3.68 (2H, m), 5.96 (1H, br), 7.20 (1H, dd, J=8.6, 2.4 Hz), 7.31 (1H, d, J=7.3 Hz), 7.39-7.43 (4H, m), 7.87 (2H, d, J=8.6 Hz), 9.96 (1H, s).
FAB⁺(m/z): 454(M+H).

Step 2b) N-[1-[3-(2-methylthio-2-methylsulfinylethenyl)phenyl]piperidin -3-yl methyl]-2-(4-chlorophenyl)-4-methylthiazole-5-carboxamide

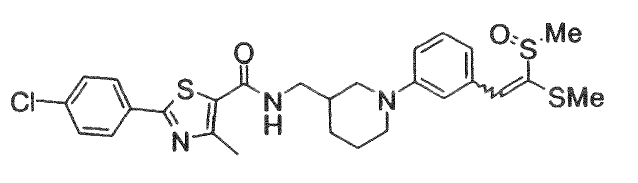

Using N-[1-(3-formylphenyl)piperidin-3-yl methyl]-2-(4-chlorophenyl)-4-methylthiazole-5-carboxamide (145 mg, 0.319 mmol), the same procedure was followed as in Step 1d of Example 1 to afford the title compound as a yellow powder (106 mg, 59%).

Step 2c) Methyl 3-[3-[[2-(4-chlorophenyl)-4-methylthiazol-5-yl]carbonylaminome thyl]piperidin-1-yl]phenylacetate

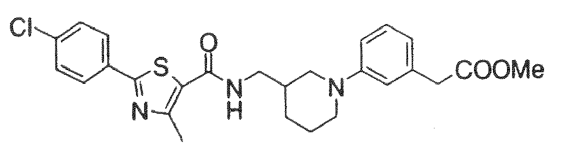

Using N-[1-[3-(2-methylthio-2-methylsulfinylethenyl)phenyl]piperidin -3-yl methyl]-2-(4-chlorophenyl)-4-methylthiazole-5-carboxamide (104 mg, 0.186 mmol), the same procedure was followed as in Step 1e of Example 1 to afford the title compound as a pale yellow powder (73.3 mg, 79%).

¹H-NMR (CDCl₃-d, 400 MHz) δ 1.24-1.28 (1H, m), 1.65-1.75 (1H, m), 1.80-1.93 (2H,m), 2.01-2.10 (1H, m), 2.66 (1H, dd, J = 12.2, 9.8 Hz), 2.68 (3H, s), 2.79-2.87 (1H, m), 3.42-3.54 (3H, m), 3.55-3.61 (3H, m), 3.67 (3H, s), 5.96-6.01 (1H, m), 6.76 (1H, d, J = 7.3 Hz), 6.82-6.88 (2H, m), 7.20 (1H, t, J = 7.3 Hz), 7.42 (2H, d, J = 8.6 Hz), 7.87 (2H, d, J = 8.6 Hz).
EI⁺ (m/z) : 497(M+).

<Example 3>
Methyl 2-[3-[[2-(4-chlorophenyl)-4-methylthiazol-5-yl]carbonylamino]p iperidin-1-yl]phenylacetate

Step 3a) N-[(1-tert-butoxycarbonyl)piperidin-3-yl]-2-(4-chlorophenyl)-4-methylthiazol-5-yl carboxamide

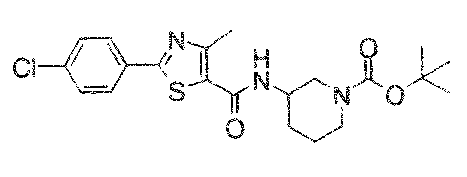

Using 2-(4-chlorophenyl)-4-methylthiazole-5-carboxylic acid (1.52 g, 5.99 mmol) and 1-(tert-butoxycarbonyl)piperidin-3-yl amine (1.20 g, 5.99 mmol), the same procedure was followed as in Step 1a of Example 1 to afford the title compound as a colorless powder (2.30 g, 88%).

¹H NMR (400 MHz, CDCl₃) δ 1.47 (9H, s), 1.56-1.74 (2H, m), 1.78-2.03 (2H, m), 2.73 (3H, s), 3.12-3.24 (1H, m), 3.34-3.50 (1H, m) 3. 68-3.71 (2H, m), 4.13-4.19 (1H, m), 6.04 (1H, m), 7.42 (3H, d, J=8.6 Hz), 7.86 (2H, d, J = 8.6 Hz).
FAB⁺(m/z): 436(M+H).

Step 3b) N-(piperidin-3-yl)-2-(4-chlorophenyl)-4-methylthiazol-5-yl carboxamide

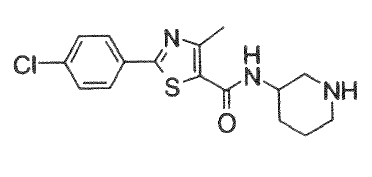

Using N-[(1-tert-butoxycarbonyl)piperidine-3-yl]-2-(4-chlorophenyl)-4-methylthiazol-5-yl carboxamide (2.30 g, 5.28 mmol), the same procedure was followed as in Step 1b of Example 1 to afford the title compound as a colorless powder (1.75 g, 99%).

¹H NMR (400 MHz, DMSO-d₆) δ 1.33-1.49 (2H, m), 1.57-1.61 (1H, m), 1.80-1.84 (1H, m), 2.37-2.42 (2H, m), 2.57 (3H, s), 2.72-2.77 (1H, m), 2.93 (1H, dd, J = 3.1, 11.6 Hz), 3.70-3.79 (1H, m), 7.57 (2H, d, J = 8.6 Hz), 7.94 (2H, d, J = 8.6Hz), 8.05 (1H, d, J = 7. 9 Hz).

Step 3c) 2-[3-[[2-(4-chlorophenyl)-4-methylthiazol-5-yl]carbonylamino]p iperidin-1-yl]benzaldehyde

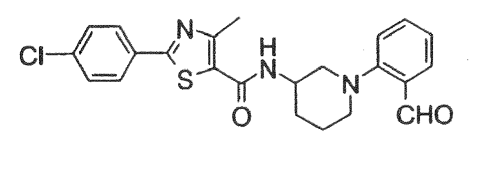

Using N-(piperidin-3-yl)-2-(4-chlorophenyl)-4-methylthlazol-5-ylcarb oxamide (67.2 mg, 0.200 mmol) and 2-fluorobenzaldehyde (0.211 mL, 2.00 mmol), the same procedure was followed as in Step 1c of Example 1 to afford the title compound as a colorless powder (14.9 mg, 17%).

¹H NMR (400 MHz, CDCl₃) δ 1.68-1.81 (2H, m), 2.01-2.11 (2H, m), 2.76 (3H, s), 2.92-2.99 (1H, m), 3.13 (1H, dd, J=2.4, 12. 2 Hz), 3.24-3.30 (2H, m), 4.36-4.43 (1H, m), 7.15 (1H, d, J = 8.6 Hz), 7.20 (1H, t, J= 7.3 Hz), 7.40-7.46 (3H, m), 7.56 (1H, dt, J = 1.8, 7.3 Hz), 7.79 (1H, dd, J = 1.8, 7.9 Hz), 7.88 (2H, d, J = 8.6 Hz), 10.14 (1H, s).

Step 3d) N-[1-[2-(2-methylthio-2-methylsulfinylethenyl)phenyl]piperidin -3-yl]-2-(4-chlorophenyl)-4-methylthiazole-5-carboxamide

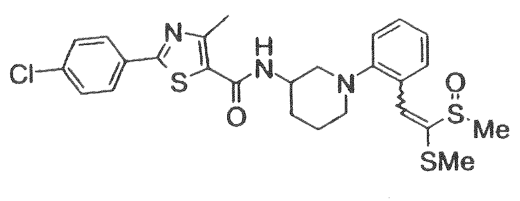

Using 2-[3-[[2-(4-chlorophenyl)-4-methylthiazol-5-yl]carbonylamino]p iperidin-1-yl]benzaldehyde (44.3mg, 0.101 mmol), the same procedure was followed as in Step 1d of Example 1 to afford the title compound as a brown amorphous material (46.7 mg, 85%).

Step 3e) Methyl 2-[3-[[2-(4-chlorophenyl)-4-methylthiazol-5-yl]carbonylamino]p iperidin-1-yl]phenylacetate

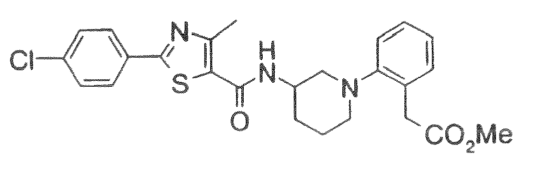

Using N-[1-[2-(2-methylthio-2-methylsulfinylethenyl)phenyl]piperidin -3-yl]-2-(4-chlorophenyl)-4-methylthiazole-5-carboxamide (44.9 mg, 0.0822 mmol), the same procedure was followed as in Step 1e of Example 1 to afford the title compound as a pale yellow oil (31.0 mg, 28%).

¹H-NMR (CDCl₃-d, 400 MHz) δ 1.72-1.80 (2H, m), 1.80-1.97 (2H, m), 2.03-2.10 (111,m), 2.75-2.80 (4H, m), 2.85-2.93 (1H, m), 3.10 (1H, d, J = 12.2 Hz) 3.59 (3H, s), 3. 69 (1H, d, J = 15.3 Hz), 3.74 (1H, d, J = 15.3 Hz), 4.43 (1H, br), 7.14 (1H, t, J= 7.3 Hz), 7.19 (1H, t, J = 6.8 Hz), 7.25-7.33 (3H, m), 7.42 (2H, d, J = 8.6 Hz), 7. 89 (2H, d, J = 8.6 Hz).
FAB⁺(m/z): 484(M+H).

<Example 4>
Methyl 3-[3-[[2-(4-chlorophenyl)-4-methylthiazol-5-yl]carbonylamino]p iperidin-1-yl]phenylacetate

Step 4a) 3-[3-[[2-(4-chlorophenyl)-4-methylthiazol-5-yl]carbonylamino]p iperidin-1-yl]benzaldehyde

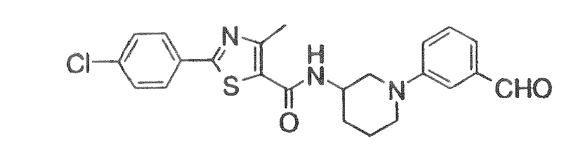

Using N-(piperidin-3-yl)-2-(4-chlorophenyl)-4-methylthiazol-5-yl carboxamide (712 mg, 2.12 mmol) and 3-formylphenylboric acid (640 mg, 4.27 mmol), the same procedure was followed as in Step 2a of Example 2 to afford the title compound as a yellow white crystal (81.3 mg, 8.7%).

¹H-HMR (CDCl₃-d, 400 MHz) δ 1.78-1.94 (4H, m), 2.72 (3H, s), 3.13-3.21 (1H, m), 3.29 (1H, dd, J = 12.2, 6.1 Hz), 3.34-3.42 (1H, m), 3.45 (1H, dd, J = 12.2, 3.1 Hz), 4.36-4.44 (1H, m), 6.22 (1H, d, J = 7.9 Hz), 7.24-7.27 (1H, m), 7.37-7.48 (5H,m), 7.87 (2H, d, J = 8.6 Hz), 9.97 (1H, s).
FAB⁺(m/z): 440(M+H).

Step 4b) N-[1-[3-(2-methylthio-2-methylsulfinylethenyl)phenyl]piperidin -3-yl]-2-(4-chlorophenyl)-4-methylthiazole-5-carboxamide

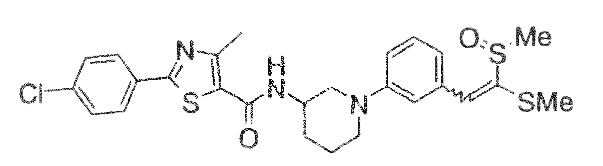

Using 3-[3-[[2-(4-chlorophenyl)-4-methylthiazol-5-yl]carbonylamino]p iperidin-1-yl]benzaldehyde (79.1mg, 0.18 mmol), the same procedure was followed as in Step 1d of Example 1 to afford the title compound as a brown oil (0.1 mg, 41%).

Step 4c) Methyl 3-[3-[[2-(4-chlorophenyl)-4-methylthiazol-5-yl]carbonylamino]p iperidin-1-yl]phenylacetate

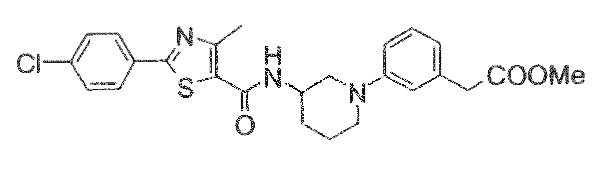

Using N-[1-[3-(2-methylthio-2-methylsulfinylethenyl)phenyl]piperidin -3-yl]-2-(4-chlorophenyl)-4-methylthiazole-5-carboxamide (38.0 mg, 0.0696 mmol), the same procedure was followed as in Step 1d of Example 1 to afford the title compound as a yellow white crystal (18.1 mg, 54%).

¹H-NMR (CDCl₃-d, 400MHz) δ 1.77-1.94 (4H, m), 2.72 (3H, s), 2.94-3.02 (1H, m), 3.27 (2H, td, J = 12.2, 3.0 Hz), 3.32-3.42 (1H, m), 3.59 (2H, s), 3.69 (3H, s), 4.40 (1N, br), 6.39 (1H, d, J = 7.9 Hz), 6. 83 (1-H, d, J = 7.9 Hz), 6.88-6.89 (2H, m), 7.21-7.24 (1H, m), 7.41 (2H, d, J = 8.6 Hz), 7.87 (2H, d, J = 8.6 Hz).
FAB⁺(m/z): 484 (M+H).

<Example 5>
Methyl 3-[3-[[2-(4-chlorophenyl)-4-methylthiazol-5-yl]carbonylamino]p iperidin-1-yl]phenoxyacetate

Step 5a) Ethyl 1-(3-benzyloxyphenyl)nipecotate

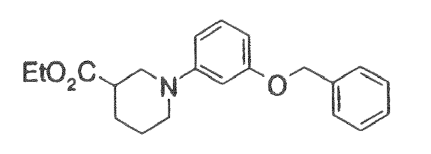

Using ethyl nipecotate (812 mg, 5.17 mmol) and 3-benzyloxyphenylboric acid (2.30g, 10.1 mmol), the same procedure was followed as in Step 2a of Example 2 to afford the title compound as a yellow oil (603 mg, 34%).

¹H-NMR (CDCl₃-d, 400 MHz) δ 1.26 (3H, t, J = 7.3 Hz), 1.62-1.72 (2H, m), 1.73-1.34(1H, m), 1.97-2.06 (1H, m), 2.59-2.68 (1H, m), 2.75-2.84 (1H, m), 3.00 (1H, dd, J = 12.8, 9.8 Hz), 3.48 (1H, d, J = 12.8 Hz), 3.71 (1H, dd, J = 12.8, 3.7 Hz), 4.15 (2H, q, J = 7.3 Hz), 5.03 (2H, s), 6.45 (1H, dd, J = 7.3, 1.8 Hz), 6.55-6.60 (2H, m), 7.14 (1H, t, J = 8.6 Hz), 7.30 (1H, t, J = 7.3 Hz), 7.37 (2H, t, J = 7.3 Hz), 7.42 (2H, d, J = 7.3 Hz).
EI⁺(m/z): 339(M+)

Step 5b) 1-(3-benzyloxyphenyl)nipecotic acid

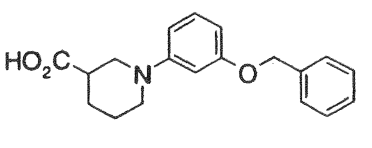

A 1 mol/L aqueous sodium hydroxide solution (3 mL) was added to a solution of ethyl 1-(3-benzyloxyphenyl)nipecotate (327 mg, 0.963 mmol) in ethanol (5 mL). The mixture was stirred at room temperature for 4 hours. Subsequently, the reaction mixture was concentrated and water was added to the resulting residue. 1 mol/L aqueous hydrochloric acid was added to make the mixture acidic. The mixture was extracted with ethyl acetate and the organic layer was washed with a saturated brine and dried over anhydrous sodium sulfate. Evaporation of the solvent afforded the title compound as a brown oil (302 mg, quantitative).

¹H-NMR (DMSO-d₆, 400 MHz) δ 1.52-1.80 (3H, m), 1.83-1.97 (1H, m), 2.53-2.70 (1H,m), 2.81-3.18 (2H, m), 3.60-3.70 (2H, m), 5.06 (2H, s), 6.48-6.75 (3H, m), 7.15(1H, t, J= 7.9 Hz), 7.31-7.35 (1H, m), 7.38 (2H, t, J = 7.3 Hz), 7.47 (2H, d, J= 7.9 Hz), 12.37 (1H, br). EI⁺(m/z) : 311(M+)

Step 5c) 1-(3-benzyloxyphenyl)-3-tert-butoxycarbonylaminopiperidine

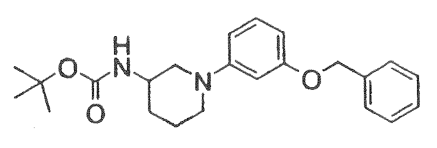

Triethylamine (0.14 mL, 1.00 mmol) and diphenylphosphoryl azide (0.21 mL, 0.997 mmol) were added to 1-(3-benzyloxyphenyl)nipecotic acid (299 mg, 0.960 mmol) in tert-butanol (5 mL). The mixture was refluxed for 9 hours. Subsequently, the reaction, mixture was concentrated and ethyl acetate was added to the residue. The mixture was then washed with 5% aqueous citric acid, a saturated aqueous sodium bicarbonate solution and a saturated brine. The organic layer was dried over anhydrous sodium sulfate and the solvent was evaporated. Purification of the resulting residue by silica gel chromatography (hexane:ethyl acetate = 10:1) afforded the title compound as a colorless oil (120 mg, 33%).

¹H-NMR (CDCl_{3-d}, 400 MHz) δ 1.40-1.52 (10H, m), 1.65-1.73 (1H, m), 1.73-1.85 (2H, m), 2.97-3.17 (3H, m), 3.31 (1H, d, J = 10.4 Hz), 3.85 (1H, br), 4.87-4.94 (1H, m), 5.04 (2H, s), 6.48 (1H, dd, J = 7.9, 1.8 Hz), 6.55-6.58 (2H, m), 7.15 (1H, t, J = 8.6 Hz), 7.32 (1H, m), 7.38 (2H, t, J=8.6Hz), 7.43 (2H, d, J=6.7 Hz).
FAB⁺(m/z): 383(M+H)

Step 5d) 3-tert-butoxycarbonylamino-1-(3-hydroxyphenyl)piperidine

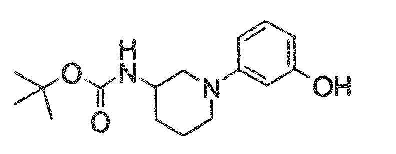

10% palladium carbon (49.2 mg) was added to 1-(3-benzyloxyphenyl)-3-tert-butoxycarbonylaminopiperidine (107 mg, 0.281 mmol) in a mixture of ethanol/tetrahydrofuran (1:1, 6 mL). The mixture was hydrogenated for 3 hours in a hydrogen atmosphere under 0.39 MPa at 50°C. Subsequently, the reaction mixture was filtered through Ce life and the filtrate was concentrated. Purification of the-resulting residue by silica gel chromatography (hexane:ethyl acetate = 1:1) afforded the title compound as a colorless amorphous material (65.0 mg, 79%).

¹H-NMR (CDCl₃-d, 400MHz) δ 1.46 (9H, s), 1.52-1.58 (1H, m), 1.64-1.73 (1H, m), 1.75-1.83 (2H, m), 2.92-3.01 (1H, m), 3.06-3.20 (1H, m), 3.40 (1H, d, J = 12.2 Hz), 3.79-3.88 (1H, m), 4.82-4.91 (1H, m), 6.32 (1H, dd, J = 7.9, 1.8 Hz), 6.43 (1H, s), 6.50 (1H, dd, J = 7.9, 1.8 Hz), 7.09 (1H, t, J = 7.9 Hz).
FAB⁺(m/z): 292(M+)

Step 5e) Methyl 3-(3-tert-butoxycarbonylamino-piperidin-1-yl)phenoxyacetate

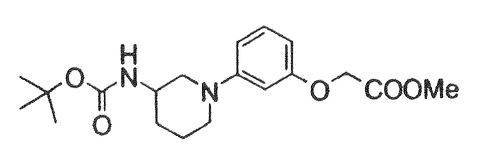

Potassium carbonate (50.0 mg, 0.362 mmol) and methyl bromoacetate (0.023 mL, 0.243 mmol) were added to a solution of 3-tert-butoxycarbonylamino-1-(3-hydroxyphenyl)piperidine (60.0 mg, 0.205 mmol) in anhydrous N,N-dimethylformamide (2 mL). The mixture was stirred at room temperature for 4 hours. Subsequently, the reaction mixture was diluted with ethyl acetate and washed with water and a saturated brine. The organic layer was dried over anhydrous sodium sulfate and the solvent was evaporated. Purification of the resulting residue by silica gel chromatography (CHROMATOREX NH-DM2035, Fuji Silysia Chemical Ltd., hexane:ethyl acetate = 3:1) afforded the title compound as a colorless oil (68.6 mg, 92%).

¹H-NMR (CDCl₃-d, 400 MHz) δ 1.46 (9H, s), 1.52-1.58 (1H, m), 1.65-1.73 (1H, m), 1.73-1.84 (2H, m), 2.95-3.04 (1H, m), 3.06-3.22 (2H, m), 3.34 (1H, d, J = 11.0 Hz), 3.78-3.88 (4H, m), 4.62 (2H, s), 4.86 (1H, br), 6.34 (1H, dd, J = 7.9, 2.4 Hz), 6.53 (1H, s), 6.60 (1H, d, J = 8.6 Hz), 7.15 (1H, t, J = 7.9 Hz).

Step 5f) Methyl 3-(3-aminopiperidin-1-yl)phenoxyacetate

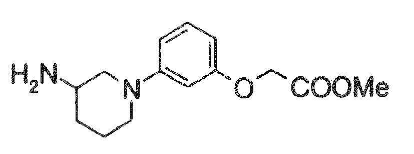

Trifluoroacetic acid (0.30 mL, 3.95 mmol) was added to methyl 3-(3-tert-butoxycarbonylamino-piperidin-1-yl)phenoxyacetate (65.9 mg, 0.181 mmol) in anhydrous dichloromethane (0.4 mL) under stirring at 0°C. The mixture was continuously stirred for 2 hours. Subsequently, the reaction mixture was concentrated and water was added to the resulting residue. This was followed by a saturated aqueous sodium bicarbonate solution to neutralize the mixture. The mixture was extracted with ethyl acetate. The organic layer was washed with a saturated brine and dried over anhydrous sodium sulfate. Evaporation of the solvent afforded the title compound as a brown oil (42.2 mg, 88%).

¹H-NMR (CDCl₃-d, 400 MHz) δ 1.65-1.72 (1H, m), 1.75-1.86 (1H, m), 1.90-1.98 (1H, m), 2.59 (1H, dd, J = 12.2, 8.6 Hz), 2.73-2.84 (1H, m), 2.93-3.15 (2H, m), 3.35-3.45 (1H, m), 3.55 (1H, d, J = 13.5 Hz), 3.81 (3H, s), 4.62(2H, s), 6.32 (1H, dd, J = 8.6, 2.4 Hz), 6.53 (2H, t, J = 2.4 Hz), 6.60 (1H, dd, J = 8.6, 1.8 Hz), 7.15 (1H, t, J = 7.9 Hz).

Step 5g) Methyl 3-[3-[[2-(4-chlorophanyl)-4-znethylthiazol-5-yl]carbonylamino]p iperidin-1-yl]phenoxyacetate

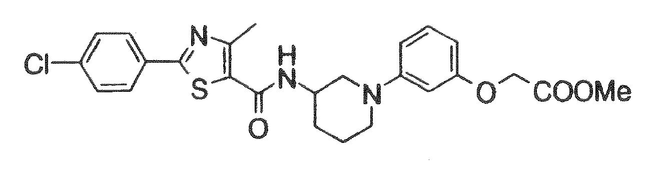

2-(4-chlorophenyl)-4-methylthiazole-5-carboxylic acid (46.8 mg, 0.184 mmol), 1-hydroxybenzotriazole (28.5 mg, 0.186 mmol), N-methylmorpholine (0.040 mL, 0.364 mmol) and 3-(3-dimethylaminopropyl)-1-ethylcarbodiimide hydrochloride (35.0 mg, 0.183 mmol) were added to a solution of methyl 3-(3-aminopiperidin-1-yl)phenoxyacetate (42.1 mg, 0.159 mmol) in anhydrous N,N-dimethylformamide (2 mL) under stirring at 0°C. The mixture was stirred for 20 min and was continuously stirred for additional 4 hours at roomtemperature. Subsequently, ethyl acetate was added and the mixture was washed with 5% aqueous citric acid, a saturated aqueous sodium bicarbonate solution, water and a saturated brine. The organic layer was dried over anhydrous sodium sulfate and the solvent was evaporated. Purification of the resulting residue by silica gel chromatography (hexane : ethyl acetate = 2:1) afforded the title compound as a pale yellow crystal (34.2 mg, 43%).

¹H-NMR (CDCl₃-d, 400 MHz) δ 1.72-1.92 (4H, m), 2.71 (3H, s), 2.95-3.04 (1H, m), 3.26-3.40 (3H, m), 3.80 (3H, s), 4.35-4.42 (1H, m), 4.63 (2H, s), 6.33 (1H, d, J = 8.6Hz), 6.39 (1H, dd, J = 8.6, 1.8 Hz), 6.58 (1H, t, J=2.4 Hz), 6.64 (1H, dd, J = 8.6, 1.8 Hz), 7.18 (1H, t, J = 8.6 Hz), 7.41 (2H, d, J = 8.6 Hz), 7.87 (2H, d, J = 8.6 Hz).
FAB⁺ (m/z) : 500 (M+H)

<Example 6>
Ethyl 2-[3-[3-[[2-(4-chlorophenyl)-4-methylthiazol-5-yl]carbonylamin o]piperidin-1-yl]phenoxy]-2-methylpropionate

Step 6a) Ethyl 2-[3-(3-tert-1-butoxycarbonylamino-piperidin-1-yl)phenoxy]-2-met hylpropionate

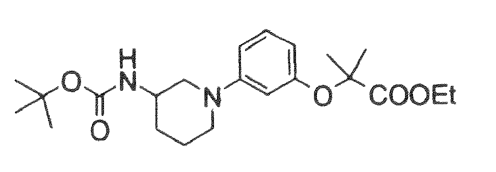

Using 3-tert-butoxycarbonylamino-1-(3-hydroxyphenyl)piperidine (82.4 mg, 0.282 mmol) and ethyl 2-bromo-2-methylpropionate, the same procedure was followed as in Step 5e of Example 5 to afford the title compound as a colorless oil (51.2 mg, 45%).

¹H-NMR (CDCl₃-d, 400 MHz) δ 1.28 (3H, t, J = 6.7 Hz), 1.46 (9H, s), 1.57-1.73 (8H, m), 1.75-1.87 (2H, m), 2.97-3.18 (3H, m), 3.31 (1H, d, J = 12.8 Hz), 3.83-3.92 (1H, m), 4.26 (2H, q, J = 6.7 Hz), 4.90-4.96 (1H, m), 6.31 (1H, dd, J = 7.9, 1.8 Hz), 6.47-6.52 (1H, m), 6.61 (1H, d, J = 7.9 Hz), 7.11 (1H, t, J = 7.9 Hz).
FAB⁺(m/z): 407 (M+H)

Step 6b) Ethyl 2-[3-(3-amino-piperidin-1-yl)-pherioxy]-2-methylpropionate

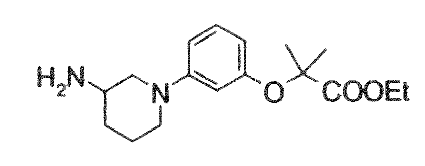

Using ethyl 2-[3-(3-tert-butoxycarbonylaminopiperidin-1-yl)-phenoxy] -2-met hylpropionate (46.7 mg, 0.117 mmol), the same procedure was followed as in Step 5f of Example 5 to afford the title compound as a yellow oil (33.0 mg, 92%).

¹H-NMR (CDCl₃-d, 400 MHz) δ 1.28 (3H, t, J = 6.1 Hz), 1.62 (6H, s), 1.65-1.74 (1H, m), 1.79-1.87 (1H, m), 1.92-1.99 (1H, m), 2.53-2.63 (1H, m), 2.75-2.85 (1H, m), 2.94-3.05 (1H, m), 5.37-3.44 (1H, m), 3.53 (1H, d, J = 12.2 Hz), 3.80 (1H, s), 4.25 (2H, q, J = 6.1 Hz), 6.26-6.28 (1H, m), 6.47-6.53 (1H, m), 6.61 (1H, dd, J = 7.9, 2.4 Hz), 7.07-7.18 (1H, m).
FAB⁺(m/z) : 307 (M+H)

Step 6c) Ethyl 2-[3-[3-[[2-(4-chlorophenyl)-4-methylthiazol-5-yl]carbonylamin o]piperidin-1-yl]phenoxy]-2-methylpropionate

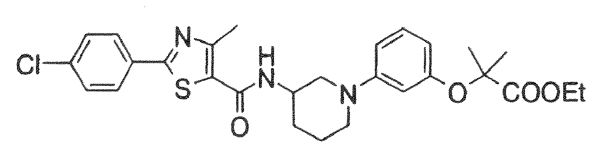

Using ethyl 2-[3-(3-amino-piperidin-1-yl)-phenoxy]-2-methylpropionate (30.8 mg, 0.101 mmol), the same procedure was followed as in Step 5g of Example 5 to afford the title compound as a colorless crystal (35.8 mg, 65%).

¹H-NMR (CDCl₃-d, 400 MHz) δ 1.24 (3H, t, J = 7.3 Hz), 1.60 (6H, s), 1.72-1.92 (4H, m), 2.72 (3H, s), 2.90-2.98 (1H, m), 3.20-3.37 (3H, m), 4.33 (2H, q, J = 7.3 Hz), 4.35-4.41 (1H, m), 6.28-6.39 (2H, m), 6.49-6.54 (1H, m), 6.59-6.64 (1H, in), 7.11 (1H, t, J=7.9 Hz), 7.42 (2H, d, J = 8.6 Hz), 7.87 (2H, d, J = 8.6 Hz).
FAB⁺(m/z):542 (M+H)

<Example 7>
Methyl 2-[3-[[2-(4-chlorophenyl)-4-methylthiazol-5-yl]carbonylamino]p iperidin-1-yl]phenoxyacetate

Step 7a) Methyl 2-(3-tert-butoxycarbonylamino-piperidin-1-yl)phenoxyacetate

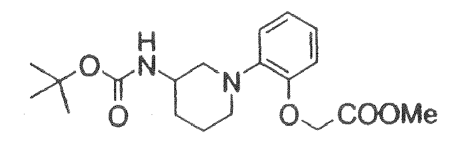

Potassium carbonate (660 mg, 7.48 mmol), copper iodide (37.3 mg, 0.196 mmol) and L-proline (45.8 mg, 0.398 mmol) 1) were added to 3-tert-butoxycarbonylaminopiperidine (373mg, 1.86 mmol) and methyl 2-iodophenoxyacetate (542 mg, 1.86 mmol) in dimethylsulfoxide (4 mL). The mixture was stirred at 80°C for 10hours. Subsequently, the reaction mixture was poured into ice water. The mixture was then extracted with ethyl acetate. The organic layer was washed with a saturated brine, dried over anhydrous sodium sulfate, and evaporated. Purification of the resulting residue by silica gel chromatography (hexane:ethyl acetate = 4:1) afforded the title compound as a yellow oil (26.2 mg, 3.9%).

¹H-NMR (CDCl₃-d, 400 MHz) δ 1.49 (9H, s), 1.61-1.72 (2H, m), 1.72-1.79 (1H, m), 1.85-1.94 (1H, m), 2.92-3.16 (4H, m), 3.80 (3H, s), 3.82-3.87 (1H, m), 4.69 (2H, s), 5.52 (1H, br), 6.74-6.80 (1H, m), 6.91-6.97 (3H, m).

Step 7b) Methyl 2-(3-amino-piperidin-1-yl)phenoxyacetate

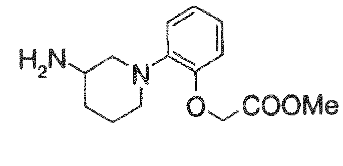

Using methyl 2-(3-tert-butoxycarbonylaminopiperidin-1-yl)phenoxyacetate (23.8 mg, 0.0653 mmol), the same procedure was followed as in Step 5f of Example 5 to afford the title compound as a yellow oil (18.5 mg, quantitative).

¹H-NMR (CDCl₃-d, 400 MHz) δ 1.74-1.87 (2H, m), 1.92-2.01 (1H, m), 2.96-3.25 (4H, m), 3.43-3.47 (1H, m) 3.82 (3H, s) 3.96-4.08 (1H, m), 4.64 (1H, d, J = 15.3 Hz), 4.69 (1H, d, J= 15.3 Hz), 6.74-6.80 (1H, m), 6.95-7.04 (3H, m).

Step 7c) Methyl 2-[3-[[2-(4-chlorophenyl)-4-methylthiazole-5-yl]carbonylamino] piperidin-1-yl]phenoxyacetate

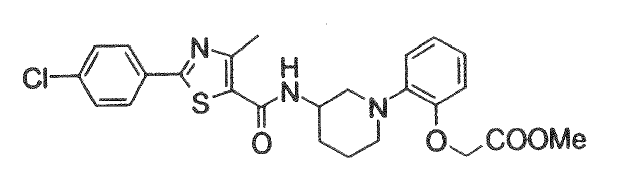

Using methyl 2-(3-amino-piperidin-1-yl)phenoxyacetate (18.5 mg, 0.0700 mmol), the same procedure was followed as in Step 5g of Example 5 to afford the title compound as a colorless crystal (14.0 mg, 40%).

¹H-NMR (CDCl₃-d, 400 MHz) δ 1.62-1.67 (1H, m), 1.73-1.83 (1H, m), 1.93-2.04 (1H, m), 2.12-2.19 (1H, m), 2.79 (3H, s), 2.83-2.97 (2H, m), 3.28-3.34 (1H, m), 3.56 (1H, d, J = 14. 1 Hz), 3.67 (3H, s), 4.35-4.40 (1H, m), 4.62 (1H, d, J = 15.9 Hz), 4.69 (1H, d, J = 15.9 Hz), 6.71-6.77 (1H, m), 6.98-7.04 (3H, m), 7.32-7.38 (1H, m), 7.44 (2H, d, J = 8.6 Hz), 7.91 (2H, d, J = 8.6 Hz).

<Example 8>
2-[3-[[2-(4-chlorophenyl)-4-methylthiazol-5-yl]carbonylaminome thyl]piperidin-1-yl]phenylacetic acid

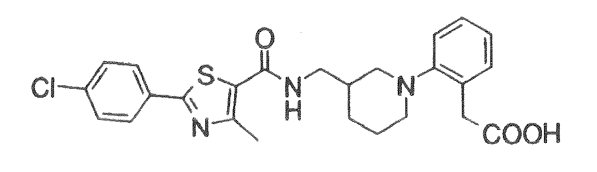

A 1 mol/L aqueous potassium hydroxide solution (1 mL) was added to a solution of methyl 2-[3-[[2-(4-chlorophenyl)-4-methylthiazol-5-yl]carbonylaminome thyl]piperidin-1-yl]phenylacetate(73.2mg, 0.147mmol) in methanol (2 mL). The mixture was stirred for 1 hour under reflux. After cooling, the solvent was evaporated and purified water was added to the resulting residue. 2 mol/L hydrochloric acid was added to make the mixture acidic. The resulting crystals were collected by filtration and washed with water to afford the title compound as a colorless powder (55.6 mg, 78%).

¹H NMR (400 MHz, DMSO-d₆) δ 1.06-1.16 (1H, m), 1.53-1.65 (1H, m), 1.69-1.79 (2H, m), 1.89-2.00 (1H, m), 2.37-2.43 (1H, m), 2.53-2. 59 (4H, m), 2.32-2.85 (1H, m), 2.93-2.97 (1H, m), 3.11-3.17 (1H, m), 3.24-3.26 (1H, m), 3.55 (1H, d, J= 15.9 Hz), 3.60 (1H, d, J= 15.9 Hz), 7.03 (1H, td, J = 7.3, 1.2 Hz), 7.13 (1H, dd, J = 7.9, 1.2 Hz), 7.19-7.24 (2H, m), 7.56 (2H, d, J = 8.6 Hz), 7.93 (2H, d, J = 8.6 Hz), 8.33(1H, t, J = 5.5 Hz).
HR-FAB⁺: 484.1472(+1.0 mmu).
Elemental analysis (%): Calcd. for C₂₅H₂₆ClN₃O₃S, 3/10H₂O: C 61.35, H 5.48, N 8.59; Found: C 61.35, H 5.37, N 8.29.

<Examples 9 through 14>
The compounds of Examples 2 through 7 were reacted as in Example 8 to obtain compounds shown in Table 2 below.

**[Table 2]**

| | | | | | |
|---|---|---|---|---|---|
| 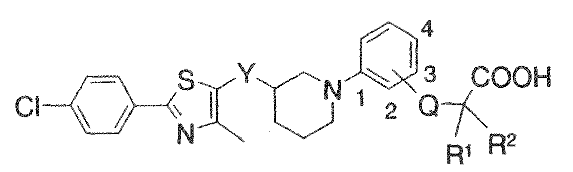 | | | | | |

| | Y | Q | R¹ | R² | Position of QGR¹R²COOH |
|---|---|---|---|---|---|
| Example 9 | CONHCH₂ | - | H | H | Position 3 |
| Example 10 | CONH | - | H | H | Position 2 |
| Example 11 | CONH | - | H | H | Position 3 |
| Example 12 | CONH | O | H | H | Position 2 |
| Example 13 | CONH | O | H | H | Position 3 |
| Example 14 | CONH | O | Me | Me | Position 3 |

(Compound, of Example 9)
Pale yellow powder
¹H-NMR (DMSO-d₆, 400 MHz) δ 1.21-1.32 (1H, m), 1.75-1.90 (3H, m), 2.13-2.24 (1H, m), 2.59 (3H, s), 2.85-3.08 (2H, m), 3.15-3.21 (3H, m), 3.55-3.58 (2H, m), 7.26-7.38 (3H, m), 7.57 (2H, d, J= 8.6 Hz), 7.94 (2H, d, J = 8.6 Hz), 8.44 (1H, t, J = 5.5 Hz).
HR-FAB⁺: 484.1488(+2.6 mmu).

(Compound of Example 10)
Colorless powder
¹H-NMR (DMSO-d₆, 400 MHz) δ 1.47-1.57 (1H, m), 1.62-1.72 (1H, m), 1.76-1.82 (1H, m), 1.85-1.95 (1H, m), 2.55-2.68 (5H, m), 2.82-2.88 (1H, m), 3.02-3.07 (1H, m), 3.61 (1H, d, J = 15.9 Hz), 3.66 (1H, d, J = 15.9 Hz), 4.00-4.11 (1H, m), 7.06 (1H, t, J = 7.3 Hz), 7.14 (1H, d, J = 7.3 Hz), 7.22-7.27 (2H, m), 7.58 (2H, d, J = 8.6 Hz), 7.95 (2H, d, J = 8.6 Hz), 8.19 (1H, br), 12.24 (1H, br).
HR-FAB⁺ : 470.1289(-1.6 mmu).

(Compound of Example 11)
Colorless powder
¹H-NMR (DMSO-d₆, 400 MHz) δ 1.48-1.68 (2H, m), 1.76-1.84 (1H, m), 1.87-1.94 (1H, m), 2.59 (3H, s), 2.65-2.75 (2H, m), 3.45-3.58 (3H, m), 3.67 (1H, d, J = 7.9 Hz), 3.95 (1H, br), 6.65 (1H, d, J = 7.3 Hz), 6.82-6.85 (2H, m), 7.13 (1H, t, J = 7.3 Hz), 7.57 (2H, d, J = 8.6 Hz), 7.95 (2H, d, J = 8.6 Hz), 8.24 (1H, d, J = 7.9 Hz), 12.24 (1H, br).
HR-FAB⁺: 470.1322(+1.7 mmu).

(Compound of Example 12)
Colorless crystal
¹H-NMR (DMSO-d₆, 400 MHz) δ 1.60-1.70 (2H, m), 1.78-1.87 (2H, m), 2.62 (3H, s), 2.65-2.78 (1H, m), 2.84-2.94 (1H, m), 3.44-3.60 (2H, m), 3.99-4.08 (1H, m), 4.68 (2H, s), 6.82-6.86 (1H, m), 6.88-7.00 (3H, m), 7.58 (2H, d, J = 8.6 Hz), 7.96 (2H, d, J = 8.6 Hz), 8.02 (1H, d, J = 6.7 Hz).
HR-FAB⁺: 486.1291(+3.6 mmu).

(Compound of Example 13)
Pale yellow powder
¹H-NMR (DMSO-d₆, 400 MHz) 5 1.50-1.68 (2H, m), 1.73-1.82 (1H, m), 1.85-1.94 (1H,m), 2.59 (3H, s), 2.69-2.83 (2H, m), 3.64-3.70 (2H, m), 3.86-3.99 (1H, m), 4.60(2H, s), 6.27 (1H, dd, J = 7.9, 2.4 Hz), 6.46 (1H, s), 6.55 (1H, d, J = 8.6 Hz), 7.08 (1H, t, J = 7.9 Hz), 7. 57 (2H, d, J = 8.6 Hz), 7.95 (2H, d, J = 8.6 Hz), 8.24 (1H, d, J = 7.3 Hz).
HR-FAB⁺: 486.1239(-1.5 mmu).

(Compound of Example 14)
Colorless crystal
¹H-NMR (DMSO-d₆, 400 MHz) δ 1.45-1.65 (8H, m), 1.75-1.83 (1H, m), 1.88-1.95 (1H, m), 2.70 (3H, s), 2.65-2.82 (2H, m), 3.50 (1H, d, J = 12.2 Hz), 3.65 (1H, d, J = 12.2 Hz), 3.88-4.03 (1H, m), 6.21 (1H, dd, J = 8.6, 1.8 Hz), 6.40 (1H, s), 6.59 (1H, d, J = 8.6 Hz), 7.07 (1H, t, J = 7.3 Hz), 7.59 (2H, d, J *=* 8.6 Hz), 7.97 (2H, d, J = 8.6 Hz), 8.24 (1H, d, J = 7.3 Hz), 12.99 (1H, br).
HR-FAB⁺: 514.1570(-0.5 mmu). Elemental analysis (%): Calcd. for C₂₆H₂₈ClN₃O₄S, 4/5H₂O: C 59.09, H 5.64, N 7.95; Found: C 58.90, H 5.34, N 7.68.

<Example 15>
Methyl [2-[3-[[2-(4-chlorophenyl)-4-methylthiazol-5-yl]methoxy]piperi din-1-yl]-phenyl]-acetate

Step 15a) 1-(tert-butoxycarbonyl)-3-[[2-(4-chlorophenyl)-4-methylthiazol -5-yl]methoxymethyl]piperidine

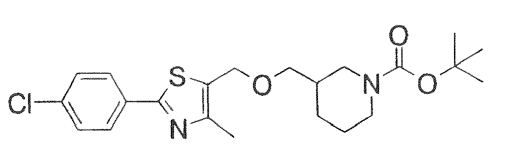

1-(tert-butoxycarbonyl)piperidin-3-ylmethanol (215mg, 1.00 mmol) was dissolved in tetrahydrofuran (5 mL). While the solution was cooled in an ice bath, 60% sodium hydride (oil dispersion) (44.0 mg, 1.10 mmol) was added and the mixture was stirred for 20 min. To this product, a solution of 5-chloromethyl-2-(4-chlorophenyl)-4-methylthiazole (258 mg, 1.00 mmol) in tetrahydrofuran (5 mL) and sodium iodide (15 mg, 0.100 mmol) were added. The resulting mixture was stirred for 2 hours in an ice bath and then 6 hours at room temperature. Subsequently, ethyl acetate was added and the mixture was washed sequentially with 5% aqueous citric acid and a saturated brine, dried over magnesium sulfate, and evaporated. Purification of the resulting residue by silica gel column chromatography (hexane:ethyl acetate = 20:1 --> 5:1) afforded the title compound as a colorless oil (258 mg, 59%).

¹H NMR (400 MHz, CDCl₃) δ 1.17-1.29 (1H, m), 1.40-1.49 (10H, m), 1.60- 1.67 (1H, m), 1.74-1.86 (2H, m), 2.44 (3H, s), 2.64 (1H, m), 2.77-2.84 (1H, m), 3.32-3.39 (2H, m), 3.87-3.90 (1H, m), 4.01 (1H, m), 4.62 (2H, s), 7. 39 (2H, d, J *=* 8.6 Hz), 7.84 (2H, d, J = 8.6 Hz).

Step 15b) 3-[[2-(4-chlorophenyl)-4-methylthiazol-5-yl]methoxymethyl]pipe ridine

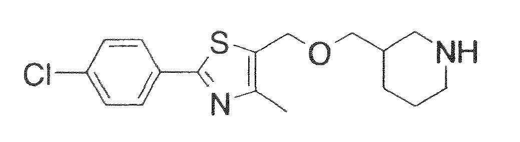

Using 1- (tert-butoxycarbonyl)-3-[[2-(4-chlorophenyl)-4-methylthiazol -5-yl]methoxymethyl]piperidine (258 mg, 0.590 mmol), the same procedure was followed as in Step 1b of Example 1 to afford the title compound as a colorless oil (169 mg, 85%).

¹H NMR (400 MHz, CDCl₃) δ 1.08-1.18 (1H, m), 1.40-1.51 (1H, m), 1.62-1.69 (1H, m), 1.75-1.86 (2H, m), 2.35 (1H, dd, J = 9.8, 12.2 Hz), 2.44 (3H, s), 2.55 (1H, dt, J = 3.1, J = 11.6 Hz), 2.97-3.02 (1H, m), 3.12-3.16 (1H, m), 3.30-3.36 (2H, m), 4.62 (2H, s), 7.39 (2H, d, J = 8.6 Hz), 7.84 (2H, d, J = 8.6 Hz).

Step 15c) 2-[3-[[2-(4-chlorophenyl)-4-methylthiazol-5-yl]methoxymethyl] piperidin-1-yl]benzaldehyde

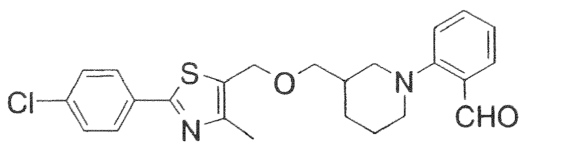

Using 3-[[2-(4-chlorophenyl)-4-methylthiazol-5-yl]methoxymethyl]pipe ridine (106 mg, 0.315 mmol) and 2-fluorobenzaldehyde (0.0329 mL, 0.315 mmol), the same procedure was followed as in Step 1c of Example 1 to afford the title compound as a colorless oil (45.2 mg, 32%).

¹H NMR (400 MHz, CDCl₃) δ 1.19-1.28 (1H, m), 1.73-1.87 (3H, m), 2.11-2.21 (1H, m), 2.43 (3H, s), 2.68-2.73 (1H, m), 2.82-2.89 (1H, m), 3.19-3.21 (1H, m), 3.33-3.37 (1H, m), 3.41-3.48 (2H, m), 4.63 (2H, m), 7.06-7.11 (2H, m), 7.38 (2H, d, J = 8.6 Hz), 7.47-7.51 (1H, m), 7.77-7.84 (3H, m), 10.29 (1H, s).

Step 15d) 1-[2-(2-methylthio-2-methylsulfinylethenyl)phenyl]-3-[2-(4-chl orophenyl)-4-methylthiazole]methoxymethyl]piperidine

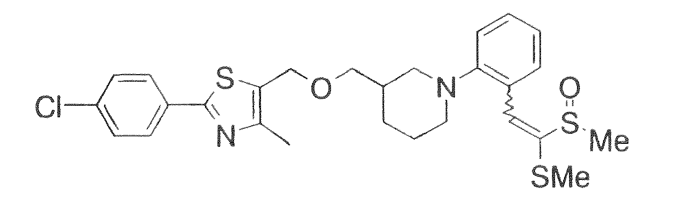

Using 2-[3-[[2-(4-chlorophenyl)-4-methylthiazol-5-yl]methoxymethyl]p iperidin-1-yl]benzaldehyde (882 mg, 2.00 mmol) and methyl methylsulfinylmethylsulfide (244 µL, 2. 40 mmol), the same procedure was followed as in Step 1d of Example 1 to afford the title compound as a yellow amorphous material (922 mg, 84%).
FAB⁺(m/z): 547(M+H).

Step 15e) Methyl 2-[3-[[2-(4-chlorophenyl)-4-methylthiazol-5-yl]methoxymethyl]p iperidin-1-yl]phenylacetate

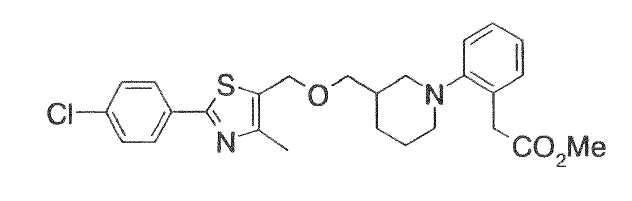

Using 1-[2-(2-methylthio-2-methylsulfinylethenyl)phenyl]-3-[2-(4-chl orophenyl)-4-methylthiazole]methoxymethyl]piperidine (922 mg, 1. 68 mmol), the same procedure was followed as in Step 1e of Example 1 to afford the title compound as a pale yellow oil (509 mg, 62%).

¹H NMR (400 MHz, CDCl₃) δ 1.10-1.25 (1H, m), 1.62-1.83 (3H, m), 2.00-2.20 (1H, m), 2.43 (3H, s), 2.42-2.50 (1H, m), 2.58-2.64 (1H, m), 2.86-2.93 (1H, m), 3.05 (1H, d, J = 11.0 Hz), 3.39-3.44 (2H, m), 3.67 (3H, s), 3.70 (2H, s), 4.62 (2H, s), 7.07 (1H, t, J = 7.3 Hz), 7.13 (1H, d, J = 7.3 Hz), 7.21-7.26 (2H, m), 7.38 (2H, d, J = 8.6 Hz), 7.83 (2H, d, J = 8.6 Hz).
FAB⁺ (m/z) : 486 (M+H).

<Example 16>
Methyl 3-[3-[[2-(4-chlorophenyl)-4-methylthiazol-5-yl]methoxy]piperid in-1-yl]phenylacetate

Step 16a) 1-(tert-butoxycarbonyl)-3-[[2-(4-chlorophenyl)-4-methylthiazol -5-yl]methoxy]piperidine

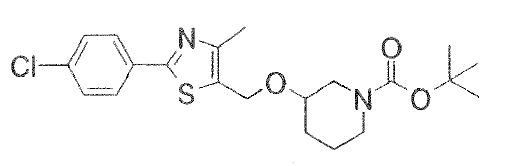

Using 1-(tert-butoxycarbonyl)-3-hydroxypiperidine (201 mg, 1.00 mmol) and 5-chloromethyl-2-(4-chlorophenyl)-4-methylthiazole (258 mg, 1.00 mmol), the same procedure was followed as in Step 15a of Example 15 to afford the title compound as a colorless oil (187 mg, 44%).

¹H NMR (400 MHz, CDCl₃) δ 1.41-1.62 (11H, m), 1.71-1.82 (1H, m), 1.88-1.97 (1H, m) 2.44 (3H, s), 3.12-3.22 (2H, m), 3.46 (1H, m), 3.53-3.56 (1H, m), 3.79 (1H, m), 4.64-4.81 (2H, m), 7.38 (2H, d, J = 8.6 Hz), 7.83 (2H, d, J = 8.6 Hz).

Step 16b) 3-[[2-(4-chlorophenyl)-4-methylthiazol-5-yl]methoxy]piperidine

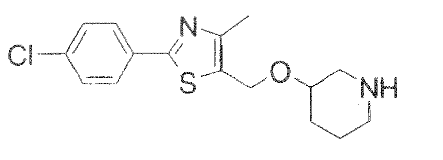

Using 1-(tert-butoxycarbonyl)-3-[[2-(4-chlorophenyl)-4-methylthiazol -5-yl]methoxy]piperidine (187 mg, 0.442 mmol), the same procedure was followed as in Step 1b of Example 1 to afford the title compound as a colorless oil (119 mg, 83%).

¹H NMR (400 MHz, CDCl₃) δ 1.39-1.49 (1H, m), 1.53-1.61 (1H, m), 1.72-1.80 (1H, m), 1.91-2.00 (1H, m), 2.44 (3H, s), 2.64-2.70 (2H, m), 2.81-2.86 (1H, m), 3.10 (1H, dd, J=2.4, J=12.2 Hz), 3.41-3.46 (1H, m), 4.65-4.72 (2H, m), 7.39 (2H, d, J = 8.6 Hz), 7.83 (2H, d, J = 8.6 Hz).

Step 16c) 3-[3-[[2-(4-chlorophenyl)-4-methylthiazol-5-yl]methoxy]piperid in-1-yl]benzaldehyde

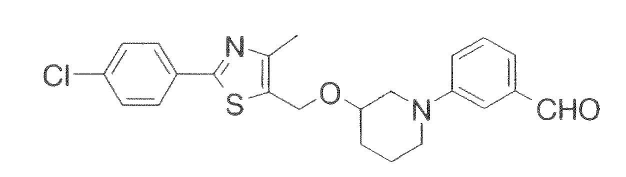

Using 3-[[2-(4-chlorophenyl)-4-methylthiazol-5-yl]methoxy]piperidine (255 mg, 0.790 mmol) and 3-formylphenylboric acid (237 mg, 1.58 mmol), the same procedure was followed as in Step 2a of Example 2 to afford the title compound as an orange oil (95 mg, 28%).

¹H NMR (400 MHz, CDCl₃) δ1.62-1.74 (2H, m), 1.89-1.98 (1H, m), 2.03-2.13 (1H, m), 2.46 (3H, s), 2.89-2.99 (2H, m), 3.46-3.52 (1H, m), 3.63-3.74 (2H, m), 4.73 (1H, d, J = 12.2 Hz), 4.78 (1H, d, J = 12.2 Hz), 7.19 (1H, d, J = 7.9 Hz), 7.31 (1H, d, J = 7.3 Hz), 7.36-7.45 (4H, m), 7.84 (2H, d, J = 8.6 Hz), 9.96 (1H, s).
EI⁺(m/z): 426(M+).

Step 16d) 1-[2-(-methhio-2-methysulfinylethenyl)phenyl]-3-[2-(4-chl orophenyl)-4-methylthiazole]methoxy]piperidine

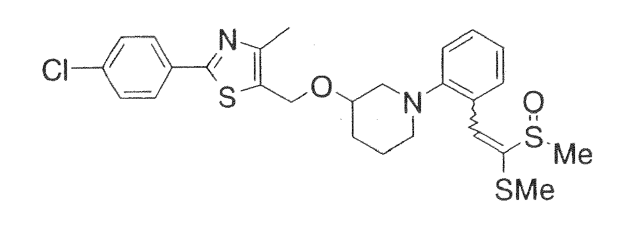

Using 3-[3-[[2-(4-chlorophenyl)-4-methylthiazol-5-yl]methoxy]piperid in-1-yl]benzaldehyde (95.0 mg, 0.220 mmol) and methyl methylsulfinylmethylsulfide (27 µL, 0.260 mmol), the same procedure was followed as in Step 1d of Example 1 to afford the title compound as an orange solid (75 mg, 64%).

FAB⁺(m/z) : 533(M+H).

Step 16e) Methyl 3-[3-[[2-(4-chlorophenyl)-4-methylthiazol-5-yl]methoxy]piperid in-1-yl]phenylacetate

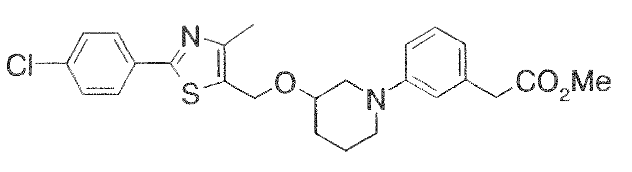

Using 1-[2-(2-methylthio-2-methylsulfinylethenyl)phenyl]-3-[2-(4-chl orophenly)-4-methylthiazole]methoxy]piperidine (75 mg, 0.141 mmol), the same procedure was followed as in Step 1e of Example 1 to afford the title compound as a reddish brown oil (35 mg, 53%).

¹H NMR (400 MHz, CDCl₃) δ 1.42-1.59 (1H, m), 1.60-1.73 (1H, m), 1.85-1.93 (1H, m), 2.06-2.13 (1H, m), 2.45 (3H, s), 2.76-2.86 (2H, m), 3.40-3.46 (1H, m), 3.57 (2H, s), 3.62-3.69 (2H, m), 3.68 (3H, s), 4.73 (1H, d, J = 12.2 Hz), 4.77 (1H,d, J = 12.2 Hz), 6.76 (1H, d, J = 7.3 Hz), 6.81-6.85 (2H, m), 7.17-7.22 (1H, m), 7.39 (2H, d, J = 8.6 Hz), 7.84 (2H, d, J = 8.6 Hz).
FAB⁺ (m/z) : 471 (M+H).

<Example 17>
Methyl 2-[3-[[2-(4-chlorophenyl)-4-methylthiazol-5-yl]methylaminocarb onyl]piperidin-1-yl]phenylacetate

Step 17a) 1-(tert-butoxycarbonyl)-N-[[2-(4-chlorophenyl)-4-methylthiazol 5-]methyl]piperidine-3-carboxamide

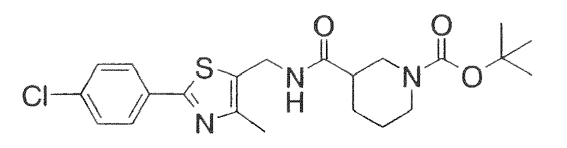

2-(4-chlorophenyl)-4-methylthiazol-5-ylmethylamine (477 mg, 2.00 mmol), 1-tert-butoxycarbonylnipecotic acid (459 mg, 2.00 mmol) and 1-hydroxybenzotriazole monohydrate (368 mg, 2.40 mmol) were dissolved in N,N-dimethylformamide (10 mL). While the solution was cooled in an ice bath, 3-(3-dimethylaminopropyl)-1-ethylcarbodiimidehydrochloride (460 mg, 2.40 mmol) and N-methylmorpholine (0.484 mL, 4.40 mmol) were added and the mixture was stirred at room temperature for 6 hours. Subsequently, 5% aqueous citric acid was added and the mixture was extracted with ethyl acetate. The organic layer was washed sequentially with a saturated aqueous sodium bicarbonate solution and a saturated brine, dried over magnesium sulfate, and evaporated. Purification of the resulting residue by silica gel column chromatography (hexane:ethyl acetate = 5:1 --> 1:1) afforded the title compound as a colorless powder (916 mg, 100%).

¹H NMR (400 MHz, CDCl₃) δ 1.36-1.51 (10H, m), 1.56-1.62 (1H, m), 1.67-1.85 (1H, m), 2.28-2.40 (1H, m), 2.45 (3H, s), 2.93-3.95 (5H, m), 4.56-4.60 (2H, m), 7.38 (2H, d, J = 8.6 Hz), 7.81 (2H, d, J = 8.6 Hz).

Step 17b) N-[[2-(4-chlorophenyl)-4-methylthiazol-5-yl]methyl]piperidine-3-carboxamide

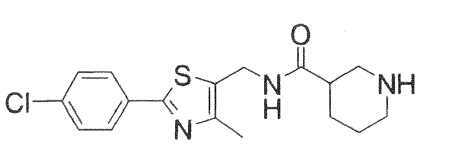

Using 1-tert-butoxycarbonyl-N-[[2-(4-chlorophenyl)-4-methylthiazol-5 -yl]methyl]piperidine-3-carboxamide (195 mg, 0.430 mmol), the same procedure was carried out as in Step 1b of Example 1 to afford the title compound as a pale yellow solid (137 mg, 90%).

¹H NMR (400 MHz, CDCl₃) δ 1.50-1.60 (1H, m), 1.65-1.81 (2H, m), 1.88-1.97 (1H, m), 2.46 (3H, s), 2.49-2.52 (1H, m), 2.78-2.84 (1H, m), 2.90-3.01 (2H, m), 3.11 (H, dd, J = 12.2, 5.5 Hz), 4.57-4.59 (2H, m), 7.38 (2H, d, J = 8.6 Hz), 7.81 (2H, d, J = 8.6 Hz), 8.12 (1H, brs).
FAB⁺(m/z) : 350 (M+H).

Step 17c) 2-[3-[[2-(4-chlorophenyl)-4-methylthiazol-5-yl]methylaminocarb onyl]piperidin-1-yl]benzaldehyde

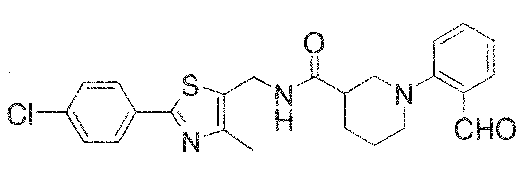

Using N-[[2-(4-chlorophenyl)-4-methylthiazol-5-yl]methyl]piperidine-3-carboxamide (137 mg, 0.392 mmol) and 2-fluoroaldehyde (62.0 µL, 0.588 mmol), the same procedure was followed as in Step 1c of Example 1 to afford the title compound as a pale yellow solid (94.0 mg, 53%).

¹H NMR (400 MHz, CDCl₃) δ 1.66-1.76 (1H, m), 1.80-1.96 (2H, m), 2.00-2.12 (1H, m), 2.42 (3H, s), 2.68-2.77 (1H, m), 2.89-2.97 (2H, m), 3.02-3.09 (1H, m), 3.18-3.2 (2H, m), 4.53 (1H, dd, J = 15.3, 4.9 Hz), 4.59 (1H, dd, J = 15.3, 4.9 Hz), 7.13 (1H, d, J = 7.9 Hz), 7.17 (1H, t, J = 7.9 Hz), 7.36 (2H, d, J = 8.6 Hz), 7.52-7.56 (1H, m), 7.74 (1H, dd, J= 7.9, 1.2 Hz), 7.77 (2H, d, J = H.6 Hz), 10.09 (1H, s).
FAB⁺ (m/z) : 454 (M+H).

Step 17d) N-[[2-(4-chlorophenyl)-4-methylthiazol-5-yl]methyl]-1-[2-(2-me thylthio-2-methylsulfinylethenyl)phenyl]piperidine-3-carboxaml de

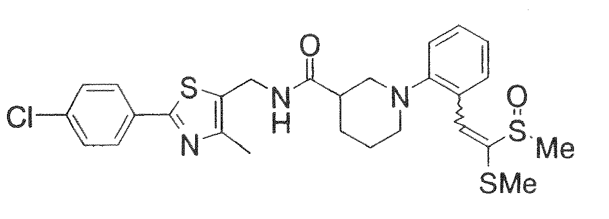

Using 2-[3-[[2-(4-chlorophenyl)-4-methylthiazol-5-yl]methylaminocarb onyl]piperidin-1-yl]benxaldehyde (94 mg, 0.207 mmol) and methyl methylsulfinylmethylsulfide (26 µL, 0.255 mmol), the same procedure was followed as in Step 1d of Example 1 to afford the title compound as a pale yellow solid (22 mg, 19%).

FAB⁺(m/z): 560(M+H).

Step 17e) Methyl 2-[3-[[2-(4-chlorophenyl)-4-methylthiazol-5-yl]methylaminocarb onyl]piperidin-1-yl]phenylacetate

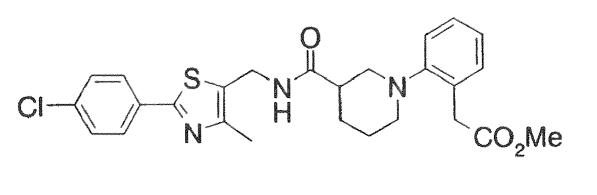

Using N-[[2-(4-chlorophenyl)-4-methylthiazol-5-yl]methyl]-1-[2-(2-me thylthio-2-methylsulfinylethenyl)phenyl]piperidine-3-casboxaml de (22 mg, 0.0392 mmol), the same procedure was followed as in Step 1e of Example 1 to afford the title compound as a colorless oil (21 mg, quantitative)).

¹H NMR (400 MHz, CDCl₃) δ 1.60-1.70 (1H, m), 1.80-1.90 (3H, m), 2.45 (3H, s), 2.60-2.68 (1H, m), 2.76-2.97 (3H, m), 3.12 (1H, dd, J = 12.2, 3.7 Hz), 3.60 (1H, d, J = 15.3 Hz), 3.61 (3H, s), 3.67 (1H, d, J = 15.3 Hz), 4.53 (1H, dd, J = 15.9, 5.5 Hz), 4.63 (1H, dd, J = 15.9, 5.5 Hz), 7.10 (1H, td, J = 7.3, 1.2 Hz), 7.15 (1H, dd, J = 7.3, 1.2 Hz), 7.24-7.29 (2H, m), 7.37 (2H, d, J = 8.6 Hz), 7.80 (2H, d, J = 8.6 Hz).
FAB⁺(m/z): 498(M+H).

<Example 18>
Methyl 2-[3-[[2-phenyl-4-methylthiazol-5-yl]carbonylaminomethyl]piper idin-1-yl]phenylacetate

Step 18a) 2-[3-(hydroxymethyl)piperidine-1-yl]benzaldehyde

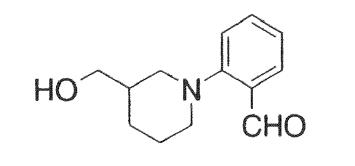

Potassium carbonate (13.8 g, 100 mmol) and tetrabutylammonium iodide (1.85 g, 5.00 mmol) were added to a solution of 3-piperidinemethanol (5.76 g, 50.0 mmol) and 2-fluorobenzaldehyde (7.90 g, 75.0 mmol) in N,N-dimethylformamide (250 mL). The mixture was stirred at 130°C for 5 hours. After cooling, water was added and the mixture was extracted with ethyl acetate. The organic layer was washed with water and a saturated brine, dried over sodium sulfate, and evaporated. Purification of the resulting residue by silica gel column chromatography (hexane:ethyl acetate = 5:1 --> 2:1) afforded the title compound as a yellow oil (9.03 g, 82%).

¹H NMR (400 MHz, CDCl₃) δ 1.17-1.28 (1H, m), 1.42 (1H, t, J = 5.5 Hz), 1.74-1.90 (3H, m), 1.99-2.09 (1H, m), 2.72 (1H, dd, J = 11. 6, 9.8 Hz), 2.83-2.92 (1H, m), 3.18-3.24 (1H, m), 3.34-3.41 (1H, m), 3.55-3.69 (2H, m), 7.06-7.14 (2H, m), 7.51 (1H, td, J = 7.9, 1.8 Hz), 7.80 (1H, dd, J = 7.9, 1.8 Hz), 10.29 (1H, s).

Step 18b) 2-[3-(tert-butyldimethylsilyloxymethyl)piperidin-1-yl]benzalde hyde

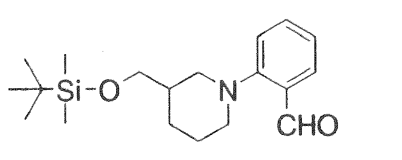

t-butyldimethylchlorosilane(7.43g, 49.3 mmol) and imidable (3.36 g, 49.3 mmol) were added to a solution of 2-[3-(hydroxymethyl)piperidin-1-yl]benzaldehyde (9. 03 g, 41.1 mmol) in N,N-dimethylformamide (45 mL). The mixture was stirred at room temperature for 12 hours. Subsequently, water was added and the mixture was extracted with ethyl acetate. The organic layer was washed with water and a saturated brine, dried over sodium sulfate, and evaporated. Purification of the resulting residue by silica a gel column chromatography (hexane:ethyl acetate = 15:1) afforded the title compound as a yellow oil (13.5 g, 98%).

¹H NMR (400 MHz, CDCl₃) δ 0.03 (3H, s), 0.04 (3H, s), 0.88 (9H, s), 1.12-1.20 (1H, m), 1.75-1.85 (3H, m), 1.94-2.06 (1H, m), 2.65 (1H, dd, J=11.6, 9.8 Hz), 2.79-2.87 (1H, m), 3.18-3.26 (1H, m), 3.34-3.39 (1H, m), 3.49 (1H, dd, J= 9.8, 7.6 Hz), 3.57 (1H, dd, 9.8, 5.5Hz), 7.05-7.13 (2H, m), 7.46-7.52 (1H, m), 7.80 (1H, dd, J = 7.9, 1.8 Hz), 10.29 (1H, brs).
EI⁺ (m/z): 333 (M+).

Step 18c) Methyl 2-[3-(hydroxymethyl)piperldin-1-yl]phenylacetate

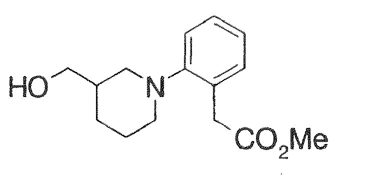

Methyl methylsulfinylmethylsulfide (2.44 mL, 24.0 mmol) and benzyltrimethylammonium hydroxide (9.09 mL, 20.0 mmol) were added to a solution of 2-[3-(tert-butyldimethylsilyloxymethyl)piperidin-1-yl]benzalde hyde (6.67 g, 20.0 mmol) in tetrahydrofuran (70 mL). The mixture was refluxed for 7 hours. After cooling, ethyl acetate was added and the organic layer was washed with water and a saturated brine, dried over sodium sulfate, and evaporated. The resulting residue was purified by silica gel column chromatography (CHROMATOREX NH-DM2035, Fuji Silysia Chemical Ltd., hexane :ethyl acetate = 20:1--> 10:1--> 5:1). The purified product was further purified by silica gel column chromatography (hexane :ethyl acetate = 1:4 --> ethyl acetate) to give a crude reddish brown oil (1.30 g). This product was dissolved in a 10% hydrochloric acid/methanol solution (10 mL). The solution was stirred for 2 hours and was allowed to stand for 3 days. Subsequently, concentrated hydrochloric acid (10 mL) was added and the mixture was allowed to stand for another day. The mixture was then concentrated and 5 mol/L sodium hydroxide was added to the resulting reside tomake it basic. The resulting mixture was extracted with ethyl acetate. The organic layer was washed with water and a saturated brine, dried over sodiumsulfate, and evaporated. Purification of the resulting residue by silica gel column chromatography (hexane:ethyl acetate = 4:1 --> 1:1 --> 1:4) afforded the title compound as a yellow solid (255 mg, 5%).

¹H NMR (400 MHz, CDCl₃) δ 1.17-1.24 (1H, m), 1.64-1.82 (3H, m), 1.89-1.98 (1H, m), 2.52 (1H, t, J = 9.8 Hz), 2.63-2.71 (1H, m), 2.86-2.92 (1H, m), 3.03-3.09 (1H, m), 3.57-3.61 (2H, m), 3.69 (1H, d, J = 15.9 Hz), 3.70 (3H, s), 3.74 (1H, d, J = 15.9 Hz), 7.07 (1H, td, J = 7.3, 1.2 Hz), 7.15 (1H, d, J = 6.7 Hz), 7.22-7.30 (2H, m).
EI⁺(m/z): 263(M+).

Step 18d) N-[[1-[(2-methoxycarbonylmethyl)phenyl]piperidin-3-yl]methyl]p hthalimide

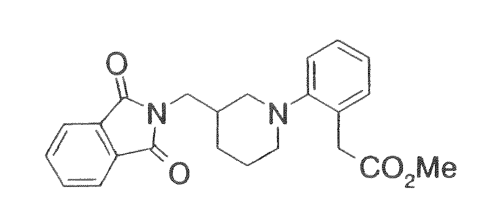

Phthalimide (200 mg, 1.36 mmol) and triphenylphosphine (305 mg, 1.16 mmol) were added to a solution of methyl 2-[3-(hydroxymethyl)piperidin-1-yl]phenylacetate (255 mg, 0.968 mmol) in tetrahydrofuran (20 mL). The mixture was stirred for 30 min and diethyl azodicarboxylate (661 µL, 1.46 mmol) was added while the mixture was cooled in an ice bath. The mixture was then stirred at room temperature for 4 hours. Subsequently, water was added and the mixture was extracted with ethyl acetate. The organic layer was washed with water and a saturated brine, dried over sodium sulfate, and evaporated. Purification of the resulting residue by silica gel column chromatography (hexane:ethyl acetate = 3:1) afforded the title compound as a pale yellow solid (381 mg, quantitative).

FAB⁺ (m/z): 393 (M+H).

Step 18e) Methyl 2-[3-(aminomethyl)piperidin-1-yl]phenylacetate

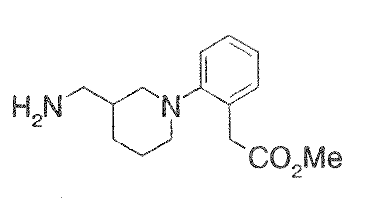

N-[1-[(2-methoxycarbonylmethyl)phenyl]piperidin-3-yl]met hyl]phthalimide (381 mg, 0.97 mmol) was dissolved in methanol (4 mL). To this solution, hydrazine monohydrate (94 µL, 1.94 mmol) was added and the mixture was refluxed for 6 hours. After cooling, the mixture was concentrated and ethyl acetate was added to the residue. The resulting mixture was filtered. The filtrate was washed with a 1 mol/L aqueous sodium hydroxide solution and a saturated brine, and dried over sodium sulfate. Evaporation of the solvent afforded the title compound as a yellow oil (182 mg, 72%).

¹H NMR (400 MHz, CDCl₃) δ 1.02-1.14 (1H, m), 1.70-1.80 (4H, m), 1.81-1.89 (1H, m), 2.37 (1H, t, J = 9.8 Hz), 2.61-2.67 (3H, m), 2.87-2.94 (1H, m), 3.02-3.08 (1H, m), 3.70 (3H, s), 3.71 (2H, s), 7.07 (1H, td, J= 7.3, 1.2 Hz), 7.14 (1H, d, J= 7.9 Hz), 7.21-7.24 (2H, m).
FAB⁺ (m/z): 263(M+H).

Step 18f) Methyl 2-[3-[[2-phenyl-4-methylthiazol-5-yl]carbonylaminomethyl]piper idin-1-yl]phenylacetate

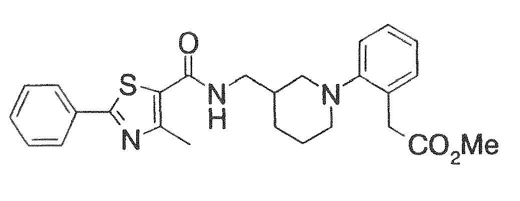

3-(3-dimethylaminopropyl)-1-ethylcarbodiimide hydrochloride (52.0 mg, 0.270 mmol) and N-methylmorpholine (51.0 µL, 0.464 mmol) were added to a solution of methyl 2-[3-(aminomethyl)piperidin-1-yl]phenylacetate (60 mg, 0.228 mmol), 2-phenyl-4-methylthlazole-5-carboxylic acid (50 mg, 0.228 mmol) and 1-hydroxybenzotrsazolo monohydrate (41 mg, 0.267 mmol) in N,N-dimethylacetamide (20 mL) while the solution was stirred in an ice bath. The mixture was stirred at room temperature for 48 hours. Subsequently, ethyl acetate was added. This mixture was washed sequentially with a 5% aqueous citric acid, a saturated aqueous sodium bicarbonate solution, and a saturated brine, dried over magnesium sulfate, and evaporated. Purification of the resulting residue by silica gel column chromatography (hexane:ethyl acetate = 4:1 --> 2:1) afforded the title compound as a colorless oil (15 mg, 14%).

¹H NMR (400 MHz, CDCl₃) δ 1.22-1.36 (1H, m), 1.63-1.76 (1H, m), 1.77-1.90 (2H, m), 2.02-2.16 (1H, m), 2.48-2. 60 (1H, m), 2.70-2.78 (1H,m), 2. 73 (3H,s), 2.83-2.95 (1H, m), 3.01-3.12 (1H, m), 3.32-3.40 (1H, m), 3.46-3.52 (1H, m), 3.66 (3H, s), 3.70-3.75 (2H, m), 6.17 (1H, brs), 7.09 (1H, d, J=7.3 Hz), 7.17 (1H, d, J = 7.3Hz), 7.30 (2H, m), 7.42-7.46 (3H, m), 7.90-7.93 (2H, m).
FAB⁺(m/z): 464(M+H).

<Example 19>
Methyl 2-[3-[[2-(4-trifluoromethylphenyl)-4-methylthiazol-5-yl]carbon ylaminomethyl]piperidin-1-yl]phenylacetate

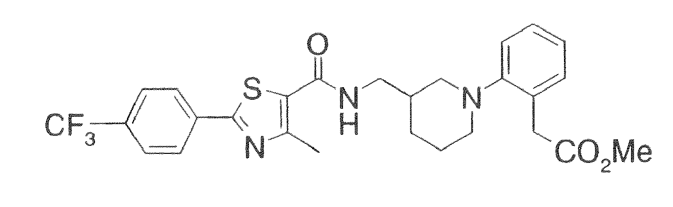

Using methyl 2-[3-(aminomethyl)piperidin-1-yl]phenylacetate (20.8 mg, 0.0793 mmol) and 2-[4-(trifluoromethyl)phenyl]-4-methylthiazle-5-carboxylic acid (22.8 mg, 0.0793 mmol), the same procedure was followed as in Step 18f of Example 18 to afford the title compound as a colorless oil (17 mg, 40%).

¹H NMR (400 MHz, CDCl₃) δ 1.19-1.38 (1H, m), 1.63-1.76 (1H, m), 1.76-1.90 (2H, m), 2.02-2.16 (1H, m), 2.49-2.62 (1H, m), 2.69-2.80 (4H, m), 2.83-2.92 (1H, m), 3.02-3.11 (1H, m), 3.30-3.40 (1H, m), 3.45-3.55 (1H, m), 3.66 (3H, s), 3.66-3.77 (2H, m), 6.26 (1H, brs), 7.10 (1H, t, J = 7.3 Hz), 7.18 (1H, d, J = 7.3 Hz), 7.22-7.30 (2H, m), 7.69 (2H, d, J = 7.9 Hz), 8.03 (2H, d, J = 7.9 Hz).
FAB⁺(m/z) : 532 (M+H).

<Example 20>
Methyl 2-[3-[[5-(4-chlorophenyl)-3-methylthiophen-2-yl]carbonylaminom ethyl]piperidin-1-yl]phenylacetate

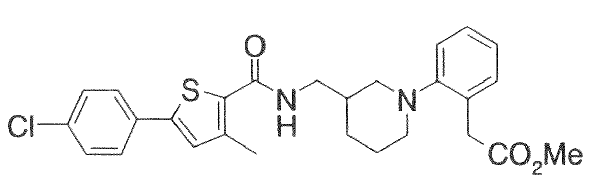

Using methyl 2-[3-(aminomethyl)piperidin-1-yl]pheriylacetate (52 mg, 0.198 mmol) and 5-(4-chlorophenyl)-3-methylthiophene-2-carboxylic acid (50 mg, 0.198 mmol), the same procedure was followed as in Step 18f of Example 18 to afford the title compound as a colorless oil (37 mg, 38%).

¹H NMR (400 MHz, CDCl₃) δ 1.20-1.30 (1H, m), 1.61-1.75 (1H, m), 1.75-1.90 (2H, m), 2.01-2.10 (1H, m), 2.45-2.55 (1H, m), 2.51 (3H, s), 2.69 (1H, t, J = 9.8 Hz), 2.83-2.92 (1H, m), 3.05 (1H, d, J = 10.4 Hz), 3.30-3.39 (1H, m), 3.40-3.50 (1H, m), 3.66 (3H, s), 3.69-3.75 (2H, m), 6.08 (1H, brs), 7.05 (1H, s), 7.09 (1H, t, J = 7.3 Hz), 7.16 (1H, d, J = 7.3 Hz), 7.23-7.28 (2H, m), 7.33 (2H, d, J = 8.6 Hz), 7.48 (2H, d, J = 8.6 Hz).
FAB⁺ (m/z): 497(M+H).

<Example 21>
Methyl 3-[2-[3-[[2-(4-chlorophenyl)-4-methylthiazol-5-yl]carbonylamin omethyl]piperidin-1-yl]phenyl]propionate

Step 21a) Methyl (E)-2-[3-(tert-butyldimethylsilyloxymethyl)piperidin-1-yl]cinn amate

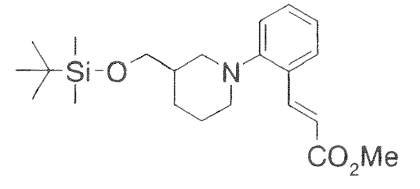

A solution of 2-[3-(tert-butyldimethylsilyloxymethyl)piperidin-1-yl]benzalde hyde (667 mg, 2.00 mmol) and methyl triphenylphosphoranilidene acetate (1.00 g, 3.00 mmol) in toluene (25 mL) was refluxed for 6 hours. After cooling, the solvent was evaporated and the residue was purified by silica gel column chromatography (hexane :ethyl acetate = 40:1) to afford the title compound as a yellow oil (638 mg, 82%).

¹H NMR (400 MHz, CDCl₃) δ 0.01 (3H, s), 0.02 (3H, s), 0.86 (9H, s), 1.04-1.16 (1H, m), 1.72-1.84 (3H, m), 1.98-2.06 (1H, m), 2.41 (1H, t, J = 10.4 Hz), 2.58-2.68 (1H, m), 3.04-3.12 (1H, m), 3.22-3.30 (1H, m), 3.45 (1H, dd, J = 9.8, 7.3 Hz), 3.57 (1H, dd, J = 9.8, 5.5 Hz), 3.80 (3H, s), 6.40 (1H, d, J = 15.9 Hz), 7.00-7.06 (2H, m), 7.30-7.35 (1H, m), 7.51 (1H, dd, J = 7.3, 1.2 Hz), 8.04 (1H, d, J = 15.9 Hz).
EI⁺ (m/z) : 389(M+).

Step 21b) Methyl 3-[2-[3-(tert-butyldmethylsilyloxymethyl)piperidin-1-yl]pheny 1]propionate

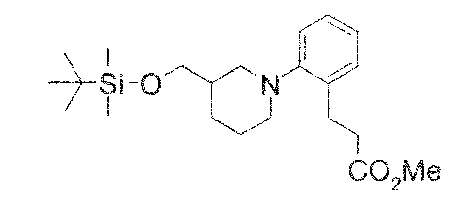

10% palladium/carbon (64 mg) was added to a solution of methyl (E)-2-[3-(tert-butyldimethylsilyloxymethyl)piperidin-1-yl]cinn amate (638mg, 1.64 mmol) in methanol (15mL). The mixture was stirred in a hydrogen atmosphere at room temperature for 3 hours. Subsequently, the mixture was filtered through Celite and the filtrate was concentrated. Purification of the resulting residue by silica gel column chromatography (hexane:ethyl acetate = 20:1) afforded the title compound as a colorless oil (575 mg, 90%).

¹H NMR (400 MHz, CDCl₃) δ 0.02 (3H, s), 0.04 (3H, s), 0.88 (9H, s), 1.06-1.16 (1H, m), 1.66-1.80 (3H, m), 1.88-1.98 (1H, m), 2.41 (1H, t, J = 10.4 Hz), 2.58-2.70 (3H, m), 2.92-3.02 (3H, m), 3.08-3.14 (1H, m), 3.48 (1H, dd, J = 9. 8, 7.9 Hz), 3.57 (1H, dd, J = 9.8, 5.5 Hz), 3.68 (3H, s), 7.02 (1H, t, J = 7.3 Hz), 7.09 (1H, d, J = 7.3 Hz), 7.15-7.20 (2H, m).
EI⁺ (m/z): 391 (M+).

Step 21c) Methyl 3-[2-[3-(hydroxymethyl)piperidin-1-yl]phenyl]propionate

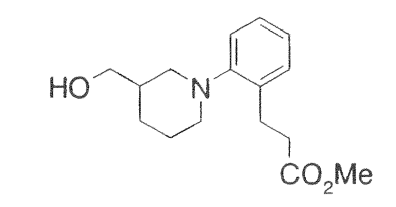

Methyl 3-[2-[3-(tert-butyldimethylsilyloxymethyl)piperidin-1-yl]pheny 1]propionate (575 mg, 1.47 mmol) was dissolved in a mixture of tetrahydrofuran (2 mL) and water (2 mL). To this solution, acetic acid (2 mL) was added and the mixture was stirred at room temperature for 2 hours. This was followed by addition of additional acetic acid (2 mL) and stirring for 12 hours. Subsequently, the mixture was further stirred at 50°C. for 5 hours. After cooling, the mixture was concentrated and water was added to the residue. A saturated aqueous bicarbonate solution was then added to make the mixture basic and the mixture was extracted with ethyl acetate. The organic layer was washed with water and a saturated brine, dried over sodium sulfate, and evaporated. Purification of the resulting residue by silica gel column chromatography (hexane:ethyl acetate = 5:1 --> 2:1) afforded the title compound as a yellow oil (320 mg, 78%).

¹H NMR (400 MHz, CDCl₃) 5 1.20-1.28 (1H, m), 1.66-1.84 (4H, m), 1.93-2.02 (1H, m), 2.58-2.75 (4H, m), 2.92-3.08 (4H, m), 3.59-3.72 (2H, m), 3.69 (3H, s), 7.03 (1H, td, J = 7.3, 1.2 Hz), 7.11 (1H, dd, J = 7.9, 1.2 Hz), 7.15-7.22 (2H, m).
EI⁺(m/z): 277 (M+).

Step 21d) N-[[1-[-[2-(methoxycarbonyl)ethyl]phenyl]piperidin-3-yl]methy l]phalimide

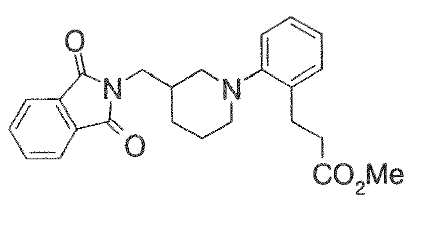

Using methyl 3-[2-[3-[hydroxymethyl)piperidin-1-yl]phenyl]propionate (320 mg, 1.15 mmol) and phthalimide (237 mg, 1.61 mmol), the same procedure was followed as in Step 18d of Example 18 to afford the title compound as a colorless solid (488 mg, quantitative).

¹H NMR (400 MHz, CDCl₃) δ 1.15-1.25 (1H, m), 1.66-1.76 (1H, m), 1.79-1.87 (2H, m), 2.22-2.30 (1H, m) 2.52-3.65 (4H, m), 2.85-3.03 (4H, m), 3.62 (1H, dd, J= 13.4, 7.9 Hz), 3.64 (3H, s), 3.71 (1H, dd, J = 13.4, 6.7 Hz), 7.00 (1H, td, J = 7.3, 1.2 Hz), 7.09 (1H, dd, J = 7.3, 1.2 Hz), 7.13 (1H, dd, J = 7.3, 1.8 Hz), 7.17 (1H, td, 7.3, 1.8 Hz), 7.70 (2H, dd, J = 5.5, 3.1 Hz), 7.83 (2H, dd, J = 5.5, 3.1 Hz).

Step 21e) Methyl 3-[2-[3-(aminomethyl)piperidiri-1-yl]phenyl]propionate

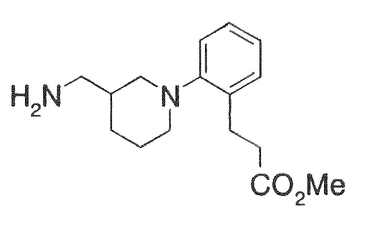

Using N-[[1-[2-[2-(methoxycarbonyl)ethyl]phenyl]piperidin-3-yl]methy 1]phthalimide (468 mg, 1.15 mmol), the same procedure was followed as in Step 18e of Example 18 to afford the title compound as a colorless oil (128 mg, 40%).

¹H NMR (400 MHz, CDCl₃) δ 1.02-1.18 (1H, m), 1.68-1.90 (5H, m), 2.41 (1H, t, J=10.4Hz), 2.60-2.72 (5H,m), 2.94-2.3.02 (3H, m), 3.06-3.11 (1H, m), 3.68 (3H, s), 6.99-7.04 (1H, m), 7.10 (1H, d, J = 7.3 Hz), 7.14-7.22 (2H, m).
FAB⁺ (m/z): 277(M+H).

Step 21f) Methyl 3-[2-[3-[[2-(4-chlorophenyl)-4-methylthiazol-5-yl]carbonylamin omethyl]piperidin-1-yl]phenyl]propionate

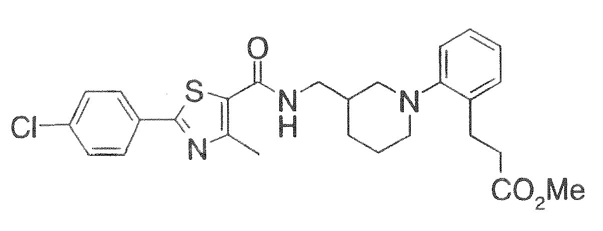

Using methyl 3-[2-[3-(aminomethyl)piperidin-1-yl]phenyl]propionate (128 mg, 0.463 mmol) and 2-(4-chlorophenyl)-4-methylthlazole-5-carboxylic acid (117 mg, 0.461 mmol), the same procedure was followed as in Step 18f of Example 18 to afford the title compound as a colorless amorphous material (165 mg, 70%).

¹H NMR (400 MHz, CDCl₃) δ 1.24-1.28 (1H, m), 1.66-1.92 (3H, m), 2.06-2.18 (1H, m), 2.54-2.76 (4H, m), 2.72 (3H, s), 2.92-3.08 (4H, m), 3.46 (2H, t, J = 6.7 Hz), 3.65 (3H, s), 6.13 (1H, brs), 7.04 (1H, t, J = 7.3 Hz), 7.11 (1H, d, J = 7.3 Hz), 7.15-7.20 (2H, m), 7.41 (2H, d, J = 8.6 Hz), 7.86 (2H, d, J = 8.6 Hz).
FAB⁺(m/z): 512(M+H).

<Example> 22>
Ethyl 2-[2-[3-[[2-(4-chlorophenyl)-4-methylthiazol-5-yl]carbonylamin omethyl]piperidin-1-yl]phenylmethyl]valerate

Step 22a) Ethyl 2-[2-[3-(hydroxymethyl)piperidin-1-yl]phenylmethyl]valerate

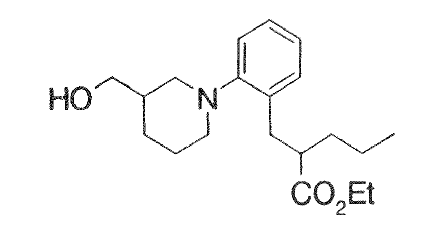

To an ice-cold tetrahydrofuran solution (15mL) of potassium t-butoxide (247 mg, 2.20 mmol), triethyl2-phosphonopentanoate (586 mg, 2.20mmol) was added dropwise in an argon atmosphere. The mixture was stirred for 30 min and a solution of 2-[3-(tert-butyldimethylsilyloxymethyl)piperidin-1-yl]benzalde hyde (667 mg, 2.00 mmol) in tetrahydrofuran (5 mL) was added dropwise. The mixture was stirred for 1 hour and then additional 6 hours at room temperature, followed by addition of ice water and then diluted hydrochloric acid to make the mixture acidic. The mixture was extracted with ethyl acetate and the organic layer was washed with a saturated brine, dried over sodium sulfate, and evaporated. The resulting residue was purified by silica gel column chromatography (hexane: ethyl acetate = 40:1, CHROMATOREX NH-DM2035, Fuji Silysia Chemical Ltd.) to give a crude yellow oil (690 mg). This product was dissolved in methanol (10 mL). To this solution, 10% palladium/carbon (69 mg) was added and the mixture was stirred at room temperature for 6 hours in a hydrogen atmosphere. Subsequently, the mixture was filtered through Celite and the filtrate was concentrated. The resulting residue was again purified by silica gel column chromatography (hexane :ethyl acetate = 10:1) to give a crude yellow oil (605 mg). The product was dissolved in tetrahydrofuran (1 mL) and a 1.0 mol/L solution of tetrabutylammonium fluoride in tetrahydrofuran (2 mL) was added to the solution. The resulting mixture was stirred at room temperature for 20 hours. Subsequently, water was added and the mixture was extracted with ethyl acetate. The organic layer was washed with water and a saturated brine, dried over sodium sulfate, and evaporated. Purification of the resulting residue by silica gel column chromatography (hexane:ethyl acetate = 8:1 --> 5:1) afforded the title compound as a yellow oil (323 mg, 48%).

¹H-NMR (400 MHz, CDCl₃) δ 0.89 (3H, t, J = 7.3 Hz), 1.10 (3H x 1/2, t, J = 7.3 Hz), 1.12 (3H x 1/2, t, J = 7.3 Hz), 1.18-1.50 (4H, m), 1.56-1.86 (4H, m), 1.92-2.02 (1H, m), 2.46-2.76 (2H, m), 2.82-2.96 (4H, m), 3.01-3.12 (1H, m), 3.58-3.80 (2H, m), 4.01 (2H x 1/2, q, J = 7. 3 Hz), 4.04 (2H x 1/2, q, J = 7.3 Hz), 7.00 (1H, t, J = 7.3 Hz), 7.09-7.20 (3H, m).
EI⁺(m/z): 333(M+).

Step 22b) N-[[1-[2-[2-(ethoxycarbonyl)pentyl]phenyl]piperidin-3-yl]methy 1]phthalimide

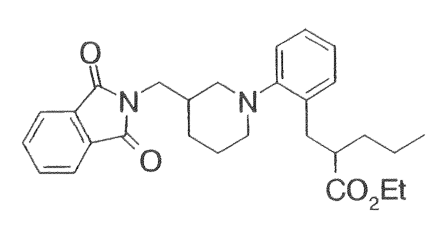

Using ethyl 2-[2-[3-(hydroxymethyl)piperidin-1-yl]phenylmethyl]valerate (323 mg, 0.970 mmol) and phthalimide (200 mg, 1.36 mmol), the same procedure was followed as in Step 18d of Example 18 to afford the title compound as a colorless oil (361 mg, 80%).

¹H-NMR (400 MHz, CDCl₃) δ 0.84 (3H, t, J = 7.3 Hz), 1.04 (3H × 1/2, t, J = 7.3 Hz), 1.08 (3H × 1/2, t, J = 7.3 Hz), 1.13-1.45 (4H, m), 1.52-2.05 (5H, m), 2.18-2.33 (1H, m), 2.46-3.00 (7H, m), 3.57-3.76 (2H, m), 3.94-4.15 (2H, m), 6.97 (1H, t, J = 7.3 Hz), 7.06-7.11 (2H, m), 7.16 (1H, td, J = 7.3, 1.2 Hz), 7.70 (2H, dd, J = 5.5, 3.1 Hz), 7.83 (2H, dd, J = 5.5, 3.1 Hz).

Step 22c) Ethyl 2-[2-[3-(aminomethyl)piperidin-1-yl]phenylmethyl]valerate

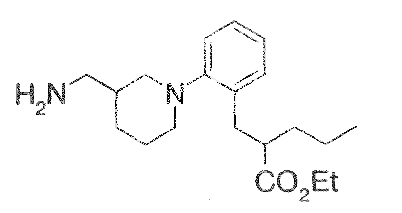

Using N-[[1-[2-[2-(ethoxycarbonyl)pentyl]phenyl]piperidin-3-yl]methy 1]phthalimide (361 mg, 78.0 mmol), the same procedure was followed as in Step 18e of Example 18 to afford the title compound as a colorless oil (236 mg, 91%).

¹H NMR (400 MHz, CDCl₃) δ 0.88 (3H, t, J = 7.3 Hz), 1.01-1.18 (1H, m), 1.12 (3H, t, J = 7.3 Hz), 1.23-1.48 (3H, m), 1.57-1.90 (7H, m), 2.35 (1H x 1/2, t, J = 10.4 Hz), 2.46 (1H x 1/2, t, J = 10.4 Hz), 2.53-2.74 (3H, m), 2.83-2.97 (4H, m), 3.03-3.09 (1H, m), 4.02 (2H, q, J = 7.3 Hz), 6.99 (1H, t, J = 7.3 Hz), 7.09-7.19 (3H, m).
FAB⁺ (m/z): 333(M+H).

Step 22d) Ethyl 2-[2-[3-[[2-(4-chlorophenyl)-4-methylthiazol-5-yl]carbonylamin omethyl]piperidin-1-yl]phenylmethyl]valerate

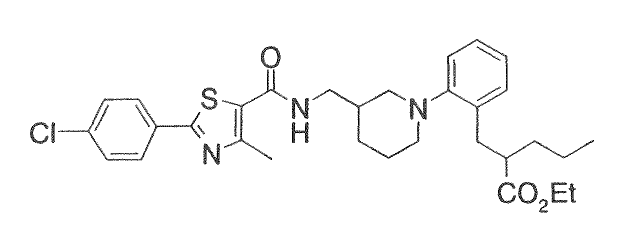

Using ethyl 2-[2-[3-(aminomethyl)piperidin-1-yl]phenylmethyl]valerate (236 mg, 0.710 mmol) and 2-(4-chlorophenyl)-4-methylthiazole-5-carboxylic acid (180 mg, 0.710 mmol), the same procedure was followed as in Step 18f of Example 18 to afford the title compound as a yellow amorphous material (277 mg, 69%).

¹H NMR (400 MHz, CDCl₃) δ 0.86 (3H x 1/2, t, J = 7.3 Hz), 0.89 (3H x 1/2, t, J = 7.3 Hz), 1.04 (3H x 1/2, t, J = 7.3 Hz), 1.09 (3H x 1/2, t, J = 7.3 Hz) 1.19-1.52 (4H, m), 1.59-1.91 (4H. m), 2.05-2.18 (1H, m), 2.43-3.10 (7H, m), 2.70 (3H x 1/2, s), 2.71 (3H x 1/2, s), 3.32-3.56 (2H, m), 3.96 (2H x 1/2, q, J *=* 7.3 Hz), 4.03 (2H x 1/2, q, J *=* 7.3 Hz), 6.16 (1H x 1/2, brs), 6.37 (1H x 1/2, brs), 6.98-7.03 (1H, m), 7.10-7.20 (3H, m), 7.40 (2H x 1/2, d, J = 8.6 Hz), 7.41 (2H x 1/2, d, J = 8.6 Hz), 7.83 (2H x 1/2, d, J = 8.6 Hz), 7.85 (2H x 1/2, d, J = 8.6 Hz).
FAB⁺(m/z): 568 (M+H).

<Example 23~30>
The compounds of Examples 14 through 22 were reacted as in Example 8 to obtain compounds shown in Table 3 below.

**[Table 3]**

| 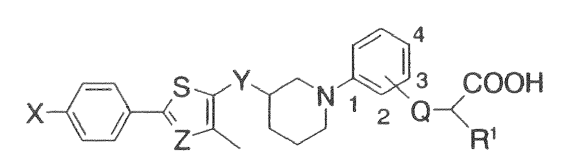 | | | | | | |
|---|---|---|---|---|---|---|
| | X | Y | **Z** | Q | R¹ | Position of QCR¹R²COOH |
| Example 33 | Cl | CH₂OCH₂ | N | - | H | Position 2 |
| Example 24 | Cl | CH₂O | N | - | H | Position 3 |
| Example 25 | Cl | CH₂NHCO | N | - | H | Position 2 |
| Example 26 | H | CONHCH₂ | N | - | H | Position 2 |
| Example 27 | CF₃ | CONHCH₂ | N | - | H | Position 2 |
| Example 28 | Cl | CONHCH₂ | CH | - | H | Position 2 |
| Example 29 | Cl | CONHCH₂ | N | CH₂ | H | Position 2 |
| Example 30 | Cl | CONHCH₂ | N | CH₂ | n-Pr | Position 2 |

(Compound of Example 23)
Colorless amorphous material
¹H NMR (400 MHz, DMSO-d₆) δ 1.08-1.20 (1H, m), 1.55-1.75 (3H, m), 1.92-2.02 (1H, m), 2.33-2.36 (1H, m), 2.37 (3H, s), 2.40-2.60 (2H, m), 2.80-2.88 (1H, m), 2.92-2.96 (1H, m), 3.42 (2H, d, J= 6.7 Hz), 3.58 (2H, s), 4.65 (2H, s), 7.03 (1H, t, J = 7.3 Hz), 7.11 (1H, d, J = 7.3 Hz), 7.17-7.24 (2H, m), 7.53 (2H, d, J = 8.6 Hz), 7.89 (2H, d, J = 8.6 Hz), 12.31 (1H, brs).
HR-FAB⁺: 471. 1526 (+1.7 mmu). Elemental analysis (%): Calcd. for C₂₅H₂₇ClN₂0₃S·4/10 H₂O: C 62.79, H 5.78, N 5.86; Found: C 62.65, H 5.82, N 5.64.

(Compound of Example 24)
Yellos solid
¹H NMR (400 MHz, DMSO-d₆) δ 1.46-2.06 (4H,m), 2.39 (3H, s), 2.68-2.82 (2H, m), 3.47 (2H, s), 3.55-3.72 (2H, m), 4.80 (2H, s), 6.64 (1H, d, J = 7.9 Hz), 6.80-6.85 (2H, m) 7.12 (1H, t, J = 7.9 Hz), 7.53 (2H, d, J = 8.6 Hz), 7.90 (2H, d, J = 8.6 Hz), 12.22 (1H, brs).
FAB⁺(m/z): 457 (M+H).

(Compound of Example 25)
Colorless solid
¹H NMR (400 MHz, DMSO-d₆) δ 1.40-1.50 (1H, m), 1.53-1.67 (1H, m), 1.67-1.75 (1H, m), 1.80-1.88 (1H, m), 2.36 (3H, s), 2.72 (1H, t, J = 11.0 Hz), 2.80-2.86 (1H, m), 2.91-2.97 (1H, m), 3.54 (1H, d, J = 15.9 Hz), 3.61 (1H, d, J = 15.9 Hz), 4.35 (1H, dd, J =15.3, 5.5 5 Hz), 4.40 (1H, dd, J =15.3, 5.5 Hz), 7.04 (1H, t, J= 7.3 Hz), 7.14 (1H, d, J = 7.3 Hz), 7.18-7.25 (2H, m), 7.51 (2H, d, J = 8.6 Hz), 7.85 (2H, d, J= 8.6 Hz), 8.50 (1H, t, J= 5.5 Hz), 12.19 (1H, brs).
HR-FAB⁺: 484.1461(-0.1 mmu).

(Compound of Example 26)
Colorless powder
¹H NMR (400 MHz, DMSO-d₆) δ 1.06-1.19 (1H, m), 1.54-1.67 (1H, m), 1.70-1.82 (2H, m), 1.90-2.03 (1H, m), 2.58-2.63 (4H, m), 2.82-2.89 (1H, m), 2,94-2.99 (1H, m), 3.10-3.19 (1H m), 3.57 (1H, d, J = 15.9 Hz), 3.63 (1H, d, J = 15.9 Hz), 7.04 (1H, td, J = 7.3, 1.2 Hz), 7.14 (1H, d, J = 6.7 Hz), 7.20-7.27 (2H, m), 7.49-7.53 (3H, m), 7.90-7.95 (2H, m), 8.31 (1H, t, J = 5.5 Hz), 12.25 (1H, s). HR-FAB⁺: 450.1872 (+2.0 mmu).
Elemental analysis (%): Calcd. for C₂₅H₂₇FN₃0₃S·4/10 H₂O: C 65.74, H 5.76, N 8.89; Found: C 65.76, H 5.93, N 9.11.

(Compound of Example 27)
colorless powder
¹H NMR (400 MHz, DMSO-d₆) δ 1.06-1.20 (2H, m), 1.54-1.68 (1H, m), 1.72-1.84 (2H, m), 1.92-2.02 (1H, m), 2.38-2.46 1H, m), 2.52-2.58 (1H, m), 2.61 (3H, s), 2.82-2.89 (1H, m), 2.94-3.00 (1H, m), 3.10-3.20 (1H, m), 3.57 (1H, d, J = 15.9 Hz), 3.62 (1H, d, J= 15.9 Hz), 7.05 (1H, t, J = 7.3 Hz), 7.15 (1H, d, J = 6. 7 Hz), 7.20-7.25 (2H, m), 7.87 (2H, d, J = 7.9 Hz), 8.15 (2H, d, J = 7.9 Hz), 8.40 (1H, t, J = 5.5 Hz), 12.25 (1H, s).
HR-FAB⁺: 518.1702(-2.4 mmu).

(Compound of Example 28)
Colorless powder
¹H NMR (400 MHz, DMSO-d₆) δ 1.06-1.18 (1H, m), 1.54-1.68 (1H, m), 1.70-1.82 (2H, m), 1.90-2.00 (1H, m), 2.40 (3H, s), 2.82-2.88 (1H, m), 2.92-2.98 (1H, m), 3.10-3.20 (1H, m), 3.55 (1H, d, J = 15.9 Hz), 3.62 (1H, d, J = 15.9 Hz), 7.04 (1H, t, J = 7.3 Hz), 7.14 (1H, d, J = 7.9 Hz), 7.20-7.26 (2H, m), 7.39 (1H, s), 7.48 (2H, d, J = 8.6 Hz), 7.67 (2H, d, J = 8.6 Hz), 8.02-8.08 (1H, m), 12.26 (1H, brs).
HR-FAB⁺: 483.1534(+2.5 mmu).

(Compound of Example 29)
Colorless amorphous
¹H NMR (400 MHz, DMSO-d₆) δ 1.08-1.18 (1H, m), 1.54-1.66 (1H, m), 1.73-1.83 (2H, m), 1.90-2.00 (1H, m), 2.40 (1H, t, J = 10.4 Hz), 2.52-2.63 (6H, m), 2.83-2.93 (3H, m), 2.96-3.03 (1H, m), 3.12-3.21 (1H, m), 3.23-3.28 (1H, m), 7.00 (1H, td, J = 7.3, 1.2 Hz), 7.10 (1H, d, J = 7.3 Hz), 7.14-7.20 (2H, m), 7.57 (2H, d, J = 8.6 Hz), 7.94 (2H, d, J = 8.6 Hz), 8.36 (1H, t, J = 5.5 Hz), 12.06 (1H, brs). HR-FAB⁺: 498.1638(+1.9 mmu).
Elemental analysis (%) : Calcd. for C₂₆H₂₈ClN₃0₃S·3/10 H₂O: C 62.03, H 5.67, N 8.35; Found: C 61.99, H 5.60, N 8.21.

(Compound of Example 30)
Colorless solid
¹H NMR (400 MHz, DMSO-d₆) δ 0.79 (3H × 1/2, t, J = 7.3 Hz), 0.80 (3H × 1/2, t, J = 7.3 Hz), 1.05-1.28 (4H, m), 1.40-1.54 (1H, m), 1.54-1.68 (1H, m), 1.73-1.84 (2H, m), 1.90-2.02 (1H. m), 2.30-2.45 (1H, m), 2.58 (3H, s), 2.61-2.80 (4H, m), 2.82-2.90 (1H,m) 2.94-3.03 (1H, m), 3.05-3.20 (1H, m), 6.96-7.02 (1H, m), 7.09-7.20 (3H, m), 7.57 (2H x 1/2, d, J = 8.6 Hz), 7.58 (2H x 1/2, d, J = 8.6 Hz), 7.93 (2H x 1/2, d, J = 8.6 Hz), 7.94 (2H x 1/2, d, J = 8.6 Hz), 8.36 (1H, t, J = 6.1 Hz), 11.96 (1H, brs).
HR-FAB⁺: 540.2084 (-0.4 mmu)
Elemental analysis (%) : Calcd. for C₂₉H₃₄ClN₃O₃S·4/10 H₂O: C 63.63, H 6.34, N 7.68; Found: C 63.48, H 6.26, N 7.43.

<Test Example 1>
Activation of human peroxisome proliferator-activated receptor α (PPARα)
CHO-K1 cells cultured in Ham's F12 medium supplemented with 10% fetal calf serum (FCS) were co-transfected with a receptor-expression plasmid engineered to express a fusion protein in which the DNA-binding domain of yeast transcription factor GAL4 is fused to the ligand-binding domain of human PPARα (Biochemistry, 1993, 32, 5598), a reporter plasmid (firefly luciferase reporter plasmid, STRATAGENE) and a Renilla luciferase reporter plasmid as an internal control. The plasmids were co-transfected for 2 hours in serum-free medium using Lipofectamine (Invitrogen). Subsequently, the cells were cultured at 37 °C for 20 hours in Ham's F12 medium supplemented with 10% defatted bovine serum and a test compound. The activity of the two luciferases was then measured and firefly luciferase activity was normalized to the internal control Renilla luciferase activity. The results are shown in Table 4 below.

**[Table 4]**

| Example No. | EC₅₀(µmol/L) |
|---|---|
| 8 | 0.0001 |
| 9 | 0.045 |
| 11 | 0.037 |
| 14 | 0.067 |

These results demonstrate that the cyclic aminophenylalkanoic acid derivatives of the present invention are a novel group of compounds that effectively activate human PPARα.

### INDUSTRIAL APPLICABILITY

The cyclic aminophenylalkanoic acid derivatives of the present invention are a novel group of compounds that effectively activate human PPARα.

Acting as agonists for human PPARα, the compounds of the present invention serve as a hypolipidemic agent that is particularly effective in lowering the lipid levels in liver and preventing the development of arteriosclerosis.

## Claims

1. A cyclic aminophenylalkanoic acid derivative represented by the following general formula (1): 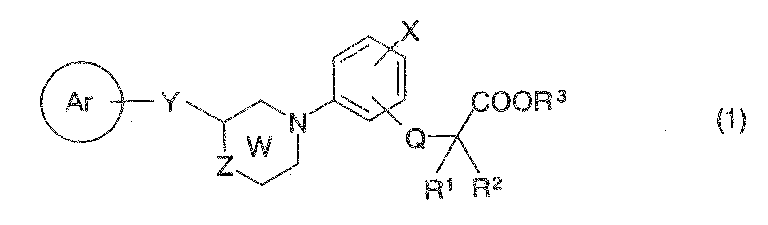 [wherein the ring Ar represents a substituted or unsubstituted aryl group or a substituted or unsubstituted 5- or 6-membered aromatic heterocyclic ring group and a fused ring group thereof;
Y represents C₁-C₄ alkylene, C₂-C₄ alkenylene, C₂-C₄ alkynylene, the following general formula (2):
-T-A-U- (2)
(wherein T represents a single bond, C₁-C₄ alkylene, C₂-C₄ alkenylene or C₂-C₄ alkynylene;
U represents a single bond, C₁-C₄ alkylene or C₂-C₄ alkenylene; and
A represents a carbonyl group, an oxygen atom, a sulfur atom, -NR⁴- (wherein R⁴ represents a hydrogen atom, a lower alkyl group that may be substituted with a halogen atom(s), a substituted or unsubstituted aralkyl group, a substituted or unsubstituted aryl group or a substituted or unsubstituted 5- or 6-membered aromatic heterocyclic ring group and a fused ring thereof), the following general formula (3): 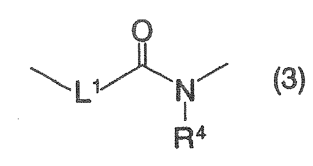
(wherein L¹ represents a single bond, an oxygen atom or -NR⁴- with R⁴ being the same as defined above), or the following general formula (4): 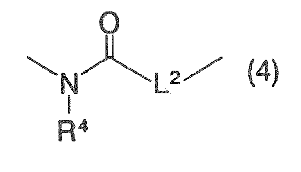 (wherein L² represents a single bond or an oxygen atom; R⁴ is as defined above));
the ring W represents a saturated heterocyclic ring containing a nitrogen atom N;
Z represents an oxygen atom, a sulfur atom or - (CH₂)ₙ- (wherein n represents an integer of 0,1 or 2);
X represents a hydrogen atom, a halogen atom, a lower alkyl group that may be substituted with a halogen atom (s), a lower alkoxy group that may be substituted with a halogen atom(s), a hydroxyl group, a nitro group, a cyano group, a substituted or unsubstituted amino group, a substituted or unsubstituted aryl group, a substituted or unsubstituted 5- or 6-membered aromatic heterocyclic group and a fused ring group thereof, a substituted or unsubstituted aralkyl group, a substituted or unsubstituted aryloxy group, or a substituted or unsubstituted aralkyloxy group;
Q represents a single bond, methylene, an oxygen atom or a sulfur atom;
R¹ and R² each independently represent a hydrogen atom or a lower alkyl group that may be substituted with a halogen atom(s); and
R³ represents a hydrogen atom or a lower alkyl group, wherein -QCR¹R²COOR³ positioned ortho or meta to the ring W], and a pharmaceutically acceptable salt thereof.

2. The cyclic aminophenylalkanoic acid derivative according to claim 1, and a pharmaceutically acceptable salt thereof, wherein in the general formula (1), Y represents the following general formula (2a) :
-T¹-A¹-U¹- (2a)
(wherein T¹ represents a single bond, C₁-C₄ alkylene or C₂-C₄ alkenylene; U1 represents a single bond or C₁-C₄ alkylene; and
A¹ represents an oxygen atom, a sulfur atom, the following general formula (3): 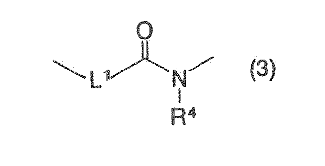 (wherein L¹ represents a single bond, an oxygen atom or -NR⁴- with R⁴ being the same as defined above), or the following general formula (4): 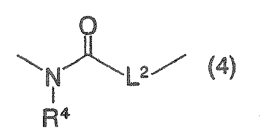 (wherein L² represents a single bond or an oxygen atom and R⁴ is as defined above)).

3. The cyclic aminophenylalkanoic acid derivative according to claim 1, and a pharmaceutically acceptable salt thereof, wherein in the general formula (1), Y represents the following general formula (2b):
-T¹-A²-U¹- (2b)
(wherein T¹ represents a single bond, C₁-C₄ alkylene or C₂-C₄ alkenylene; U¹ represents a single bond or C₁-C₄ alkylene; and
A² represents an oxygen atom, a sulfur atom, the following general formula (3a): 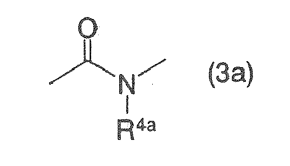
(wherein R^{4a} represents a hydrogen atom, an alkyl group that may be substituted with a halogen atom(s), or a substituted or unsubstituted aralkylgroup), or the following general formula (4a): 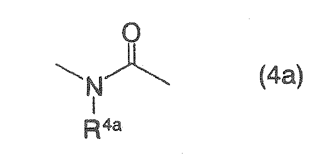 (wherein R⁴ is as defined above)).

4. The cyclic aminophenylalkanoic acid derivative according to claim 1, and a pharmaceutically acceptable salt thereof, wherein in the general formula (1), Y represents the following general formula (2c) :
-T¹-A³-U²- (2c)
(wherein T¹ represents a single bond, C₁-C₄ alkylene or C₂-C₄ alkenylene; U² represents a single bond or methylene; and
A³ represents the following general formula (3a): 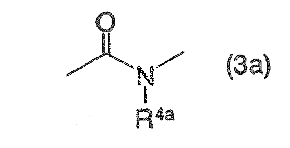
(wherein R^{4a} represents a hydrogen atom, an alkyl group that may be substituted with a halogen atom(s), or a substituted or unsubstituted aralkyl group), or the following general formula (4a): 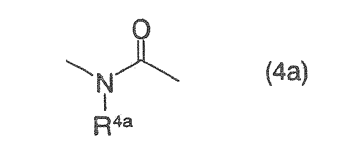 (wherein R⁴ is as defined above)).

5. The cyclic aminophenylalkanoic acid derivative according to any one of claims 1 through 4, and a pharmaceutically acceptable salt thereof, wherein in the general formula (1), Z represents an oxygen atom, a sulfur atom or methylene.

6. The cyclic aminophenylalkanoic acid derivative according to any one of claims 1 through 5, and a pharmaceutically acceptable salt thereof, wherein in the general formula (1), Z represents methylene.

7. The cyclic aminophenylalkanoic acid derivative according to any one of claims 1 through 6, and a pharmaceutically acceptable salt thereof, wherein in the general formula (1), X represents a hydrogen atom, a halogen atom, a lower alkyl group that may be substituted with a halogen atom(s), a lower alkoxy group that may be substituted with a halogen atom(s), a hydroxyl group, or a substituted or unsubstituted amino group.

8. The cyclic aminophenylalkanoic acid derivative according to any one of claims 1 through 7, and a pharmaceutically acceptable salt thereof, wherein in the general formula (1), the ring Ar represents a substituted or unsubstituted 5-or 6-membered aromatic heterocyclic ring group.

9. The cyclic aminophenylalkanoic acid derivative according to any one of claims 1 through 8, and a pharmaceutically acceptable salt thereof, wherein in the general formula (1), the ring Ar represents the following general formula (5): 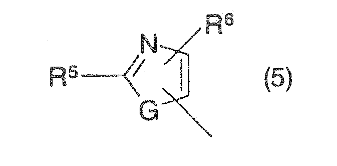 (wherein R⁵ represents a lower alkyl group that may be substituted with a halogen atom(s), a cyclic alkyl group, a lower alkoxy group that may be substituted with a halogen atom(s), a substituted or unsubstituted amino group, a 5- or 6- membered cyclic amino group, a substituted or unsubstituted aryl group, or a substituted or unsubstituted 5- or 6-membered aromatic heterocyclic ring group; R⁶ represents a hydrogen atom or a lower alkyl group that may be substituted with a halogen atom(s) or a cycloalkyl group; and G represents an oxygen atom or a sulfur atom).

10. The cyclic aminophenylalkanoic acid derivative according to claim 1, and a pharmaceutically acceptable salt thereof, wherein the compound of the general formula (1) is
2-[3-[[2-(4-chlorophenyl)-4-methylthiazole-5-yl]carbonylaminom ethyllpiperidine-1-yllphenylacetic acid,
3-[3-[[2-(4-chlorophenyl)-4-methylthiazole-5-yl]carbonylaminom ethyl]piperidine-1-yl]phenylacetic acid,
3-[3-[[2-(4-chlorophenyl)-4-methylthiazole-5-yl]carbonylamino] piperidine-1-yl]phenylacetic acid,
2-[3-[3-[[2-(4-chlorophenyl)-4-methylthiazole-5-yl]carbonylami nolpiperidine-1-yl]phenoxyl-2-methylpropionic acid,
(S)-2-[3-[[2-(4-chlorophenyl)-4-methylthiazole-5-yl]carbonylam inomethyllpiperidine-1-yllphenylacetic acid,
(R)-2-[3-[[2-(4-chlorophenyl)-4-methylthiazole-5-yl]carbonylam inomethyl]piperidine-1-yl]phenylacetic acid,
(S)-3-[3-[[2-(4-chlorophenyl)-4-methylthiazole-5-yl]carbonylam inomethyl]piperidine-1-yl]phenylacetic acid,
(R)-3-[3-[[2-(4-chlorophenyl)-4-methylthiazole-5-yl]carbonylam inomethyl]piperidine-1-yl]phenylacetic acid,
(S)-3-[3-[[2-(4-chlorophenyl)-4-methylthiazole-5-yl]carbonylam ino]piperidine-1-yl]phenylacetic acid,
(R)-3-[3-[[2-(4-chlorophenyl)-4-methylthiazole-5-yl]carbonylam ino]piperidine-1-yl]phenylacetic acid,
(S)-2-[3-[3-[[2-(4-chlorophenyl)-4-methylthiazole-5-yl]carbony lamino]piperidine-1-yl]phenoxyl-2-methylpropionic acid, or
(R)-2-[3-[3-[[2-(4-chlorophenyl)-4-methylthiazole-5-yl]carbony lamino]piperidine-1-yl]phenoxy]-2-methylpropionic acid.

11. A pharmaceutical product containing as an active ingredient at least one of the cyclic aminophenylalkanoic acid derivatives according to any one of claims 1 to 10, and a pharmaceutically acceptable salt thereof.

12. A PPARα agonist containing as an active ingredient at least one of the cyclic aminophenylalkanoic acid derivatives according to any one of claims 1 to 10, and a pharmaceutically acceptable salt thereof.

13. A PPARα/γ-dual agonist containing as an active ingredient at least one of the cyclic aminophenylalkanoic acid derivatives according to any one of claims 1 to 10, and a pharmaceutically acceptable salt thereof.

14. A PPARα/δ-dual agonist containing as an active ingredient at least one of the cyclic aminophenylalkanoic acid derivatives according to any one of claims 1 to 10, and a pharmaceutically acceptable salt thereof.

15. A PPARα/γ/δ-triple agonist containing as an active ingredient at least one of the cyclic aminophenylalkanoic acid derivatives according to any one of claims 1 to 10, and a pharmaceutically acceptable salt thereof.

16. A PPARα modulator containing as an active ingredient at least one of the cyclic aminophenylalkanoic acid derivatives according to any one of claims 1 to 10, and a pharmaceutically acceptable salt thereof.

17. A hypolipidemic agent containing as an active ingredient at least one of the cyclic aminophenylalkanoic acid derivatives according to any one of claims 1 to 10, and a pharmaceutically acceptable salt thereof.

18. A pharmaceutical composition for the prevention or treatment of arteriosclerosis containing as an active ingredient at least one of the cyclic aminophenylalkanoic acid derivatives according to any one of claims 1 to 10, and a pharmaceutically acceptable salt thereof.

19. A pharmaceutical composition for the prevention or treatment of diabetes containing as an active ingredient at least one of the cyclic aminophenylalkanoic acid derivatives according to any one of claims 1 to 10, and a pharmaceutically acceptable salt thereof.

20. A pharmaceutical composition for the prevention or treatment of obesity containing as an active ingredient at least one of the cyclic aminophenylalkanoic acid derivatives according to any one of claims 1 to 10, and a pharmaceutically acceptable salt thereof.
